(19) Europäisches Patentamt
European Patent Office
Office européen des brevets

(11) Veröffentlichungsnummer: **0 099 017**
**B1**

(12) **EUROPÄISCHE PATENTSCHRIFT**

(45) Veröffentlichungstag der Patentschrift: **02.03.88**

(21) Anmeldenummer: **83106233.6**

(22) Anmeldetag: **27.06.83**

(51) Int. Cl.[4]: **C 07 D 295/12**, C 07 C 103/30, C 07 D 295/14, C 07 D 213/80, C 07 D 211/14, A 61 K 31/445, A 61 K 31/16

# (54) Neue Phenylessigsäure-Derivate, ihre Herstellung und diese Verbindungen enthaltende Arzneimittel.

(30) Priorität: **06.07.82 DE 3225155**
**06.07.82 DE 3225188**

(43) Veröffentlichungstag der Anmeldung:
**25.01.84 Patentblatt 84/04**

(45) Bekanntmachung des Hinweises auf die Patenterteilung:
**02.03.88 Patentblatt 88/09**

(84) Benannte Vertragsstaaten:
**AT BE CH DE FR IT LI LU NL SE**

(56) Entgegenhaltungen:
**EP-A-0 058 779**
**BE-A- 764 238**
**FR-A-2 225 163**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH**
**Postfach 1755**
**D-7950 Biberach (Riss) (DE)**

(72) Erfinder: **Grell, Wolfgang, Dr., Dipl.-Chem.**
**Amriswilstrasse 7**
**D-7950 Biberach 1 (DE)**
Erfinder: **Hurnaus, Rudolf, Dr., Dipl.-Chem.**
**Silcherstrasse 19**
**D-7950 Biberach 1 (DE)**
Erfinder: **Griss, Gerhart, Dr., Dipl.-Chem.**
**Schopperweg 1**
**D-7950 Biberach 1 (DE)**
Erfinder: **Sauter, Robert, Dr., Dipl.-Chem.**
**Albert-Schweitzer-Weg 9**
**D-7958 Laupheim (DE)**
Erfinder: **Rupprecht, Eckhard, Dr., Dipl.-Biol.**
**Riedbachstrasse 15**
**D-7960 Aulendorf-Tannhausen (DE)**
Erfinder: **Kähling, Joachim, Dr.**
**Haydnweg 8**
**D-7950 Biberach 1 (DE)**
Erfinder: **Eisele, Bernhard, Dr., Dipl.-Chem.**
**Beethovenstrasse 12**
**D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 099 017 B1

**Beschreibung**

In der EP—A—0.058.779, welche nur teilweise eine Vorveröffentlichung darstellt, werden u. a. bereits Phenylessigsäurederivate der allgemeinen Formel

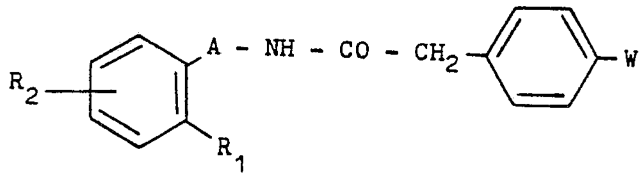 , (I)

in der

A eine durch eine Methyl-, Äthyl-, Isopropyl-, Cyclohexyl- oder Phenylgruppe substituierte Methylengruppe oder eine

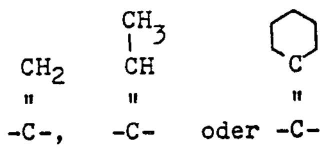 oder Gruppe bedeutet, beschrieben.

Es wurde nun überraschenderweise gefunden, daβ die neuen Phenylessigsäurederivate der obigen allgemeinen Formel I, in der

A eine Gruppe der Formeln

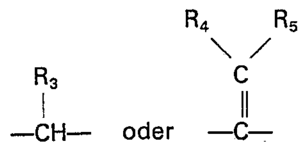 oder

wobei

$R_3$ eine durch eine Alkoxygruppe mit 1 bis 3 Kohlenstoffatomen oder durch eine Phenylgreuppe substituierte Alkylgruppe mit 1 bis 3 Kohlenstoffatom, eine n-Propylgruppe, eine Alkylgruppe mit 4 bis 6 Kohlenstoffatomen, eine Alkenylgruppe mit 3 bis 5 Kohlenstoffatomen, eine Cyano- oder Alkyleniminocarbonylgruppe mit 4 bis 6 Kohlenstoffatomen im Alkylenteil, eine gegebenenfalls durch Alkyl- oder Phenylalkylgruppen mit jeweils 1 bis 3 Kohlenstoffatomen im Alkylteil mono- oder disubstituierte Aminocarbonylgruppe, eine durch Halogenatome, durch Alkyl-, Hydroxy-, Alkoxy-, Phenylalkoxy-, Alkylsulfenyl-, Alkylsulfinyl- und/oder Alkylsulfonylgruppen mono- oder disubstituierte Arylgruppe mit 6 oder 10 Kohlenstoffatomen, wobei die Substituierten gleich oder verschieden sein können und der Alkylteil jeweils 1 bis 3 Kohlenstoffatome enthalten kann, eine Naphthylgruppe oder eine 1 oder 2 Stickstoffatome enthaltende Heteroarylgruppe mit 4, 5, 8 oder 9 Kohlenstoffatomen,

oder auch eine Methylgruppe,

wenn $R_1$ eine Piperidinogruppe sowie $R_2$ in 4-Stellung ein Fluoratom und W eine Carboxy- oder Alkoxycarbonylgruppe, in der der Alkylteil 1 bis 3 Kohlenstoffatome enthalten kann, darstellen,

oder auch eine Phenylgruppe,

wenn $R_1$ eine in 2-oder 3-Stellung durch eine Methylgruppe substituierte Piperidinogruppe, $R_2$ ein Wasserstoffatom und W eine Carboxy- oder Alkoxycarbonylgruppe, im der der Alkylteil 1 bis 3 Kohlenstoffatome enthalten kann, oder

wenn $R_1$ eine Piperidinogruppe, $R_2$ in 3-, 4- oder 6-Stellung ein Chloratom oder in 4- oder 6-Stellung ein Methylgruppe und W eine Carboxy- oder Alkoxycarbonylgruppe, in der der Alkylteil 1 bis 3 Kohlenstoffatome enthalten kann, oder

wenn $R_1$ eine Piperidinogruppe, $R_2$ ein Wasserstoffatom und W eine Formylgruppe, eine 2-Carboxyäthenyl- oder 2-Alkoxycarbonyläthenylgruppe, in der der Alkylteil 1 bis 3 Kohlenstoffatome enthalten kann, darstellen,

$R_4$ und $R_5$ zusammen mit dem dazwischenliegenden Kohlenstoffatom eine Alkylidengruppe mit 3 bis 9 Kohlenstoffatomen oder eine Phenylalkylidengruppe mit 1 bis 3 Kohlenstoffatomen im Alkenylidenteil,

$R_1$ eine gegebenenfalls durch Alkylgruppen mit 1 bis 3 Kohlenstoffatomen mono- oder disubstituierte unverzweigte Alkyleniminogruppe mit 4 bis 8 Kohlenstoffatomen,

$R_2$ ein Wassserstoff-, Fluor-, Chlor- oder Bromatom, eine Hydroxy-, Trifluormethyl-, Nitro-, Amino-, Piperidino-, Alkyl-, Alkoxy-, Alkylsulfenyl-, Alkylsulfinyl-, Alkylsulfonyl-, Phenylalkoxy-, Alkanoyloxy-, Alkanoylamino-, Alkylamino- oder Dialkylaminogruppe, wobei der Alkylteil jeweils 1 bis 3 Kohlenstoffatome enthalten kann, und

W eine Carboxygruppe oder eine Alkoxycarbonylgruppe mit insgesamt 2 bis 5 Kohlenstoffatomen, in welcher der Alkylteil durch eine Phenylgruppe und ab dem β-Kohlenstoffatom durch eine oder zwei

Hydroxygruppen, durch eine Alkoxy-, Alkanoyloxy-, Dialkylamino-, Alkylenimino- oder Pyridincarbonyloxygruppe substituiert sein kann, wobei jeder Alkylteil jeweils 1 bis 3 Kohlenstoffatome und die Alkyleniminogruppe 4 bis 6 Kohlenstoffatome enthalten kann, eine Alkenyloxycarbonylgruppe mit insgesamt 4 bis 6 Kohlenstoffatomen, eine Alkylgruppe mit 1 bis 3 Kohlenstoffatomen, eine Hydroxymethyl-, Formyl-, Cyano-, Aminocarbonyl-, Carboxy-methyl-, 2-Carboxy-äthyl-, 2-Carboxy-äthenyl-, 2,2-Bis-(carboxy)-äthyl-, Alkoxycarbonyl-methyl-, 2-Alkoxycarbonyl-äthyl-, 2-Alkoxycarbonyl-äthenyl- oder 2,2-Bis-(alkoxycarbonyl)-äthylgruppe, wobei die Alkoxygruppe jeweils 1 bis 3 Kohlenstoffatome enthalten kann, bedeuten, gegenüber den strukturähnlichsten vorbekannten Verbindungen wertvollere pharmakologische Eigenschaften aufweisen, insbesondere eine Wirkung auf den Intermediärstoffwechsel, vorzugsweise jedoch eine blutzuckersenkende Wirkung.

Gegenstand der vorliegenden Erfindung sind die neuen Phenylessigsäurederivate der obligen allgemeionen Formel I, deren Enantiomere und deren Salze, insbesondere deren physiologisch verträgliche Salze mit anorganischen oder organischen Säuren oder Basen, diese Verbindungen enthaltende Arzneimittel und Verfahren zu ihrer Herstellung.

Für die bei der Definition der Reste $R_1$ bis $R_5$ und W eingangs erwähnten Bedeutungem kommen beispielsweise

für $R_1$ die der Pyrrolidino-, Piperidino-, Hexamethylenimino-, Heptamethylenimino-, Octamethylenimino-, Methylpyrrolidino-, Dimethyl-pyrrolidino-, Äthyl-pyrrolidino-, Methyl-piperidino-, Äthyl-piperidino-, Dimethyl-piperidino-, Diäthyl-piperidino-, Methyl-äthylpiperidino-, n-Propylpiperidino-, Methyl-n-propylpiperidino-, Isopropyl-piperidino- oder Di-n-propyl-piperidinogruppe,

für $R_2$ die des Wasserstoff-, Fluor-, Chlor- oder Bromatoms, der Methyl-, Äthyl-, n-Propyl-, Isopropyl-, Hydroxy-, Methoxy-, Äthoxy-, n-Propoxy-, Isopropoxy-, Trifluormethyl-, Nitro-, Amino-, Piperidino-, Methylmercapto-, Äthylmercapto-, n-Propylmercapto-, Isopropylmercapto-, Methylsulfinyl-, Äthylsulfinyl-, Methylsulfonyl-, n-Propylsulfonyl-, Benzyloxy-, 1-Phenyl-äthoxy-, 2-Phenyl-äthoxy-, 3-Phenyl-propoxy-, Acetoxy-, Propionyloxy-, Formylamino-, Acetylamino-, Propionylamino-, Methylamino-, Äthylamino-, n-Propylamino-, Dimethylamino-, Diäthylamino-, Di-n-propylamino- oder Methyläthylamino-gruppe,

für $R_3$ die der n-Propyl-, n-Butyl-, n-Pentyl-, n-Hexyl-, Methoxymethyl-, Äthoxymethyl-, n-Propoxymethyl-, Isopropoxymethyl-, 2-Methoxy-äthyl-, 2-Äthoxy-äthyl-, 3-Methoxy-propyl-, Benzyl-, 1-Phenyl-äthyl-, 2-Phenyl-äthyl-, 1-Phenyl-n-propyl-, 2-Phenyl-n-propyl-, 3-Phenyl-propyl-, Allyl-, 3-Buten-1-yl-, 2-Buten-1-yl-, 4-Penten-1-yl-, Cyano-, Amino-, carbonyl-, Methylaminocarbonyl-, Äthylaminocarbonyl-, n-Propylaminocarbonyl-, Dimethylaminocarbonyl-,. Diäthylaminocarbonyl-, Di-n-propylaminocarbonyl-, Benzylaminocarbonyl-, 2-Phenyl-äthylaminocarbonyl-, Pyrrolidinocarbonyl-, Piperidinocarbonyl-, Hexamethyleniminocarbonyl-, Phenyl-, Naphthyl-, Fluorphenyl-, Chlorphenyl-, Bromphenyl-, Methylphenyl-, Äthylphenyl-, Isopropylphenyl-, Hydroxyphenyl-, Methoxyphenyl-, Äthoxyphenyl-, n-Propoxyphenyl-, Benzyloxyphenyl-, 2-Phenyläthoxy-phenyl-, 3-Phenylpropoxy-phenyl-, Methylsulfenyl-phenyl-, Äthylsulfenyl-phenyl-, Methylsulfenyl-phenyl-, n-Propylsulfinyl-phenyl-, Methylsulfonyl-phenyl-, Äthylsulfonyl-phenyl-, Isopropylsulfonyl-phenyl-, Methyl-napthyl-, Hydroxy-naphthyl-, Methoxy-naphthyl-, Dichlorphenyl-, Chlor-brom-phenyl-, Dimethyl-phenyl-, Di-isopropyl-phenyl-, Chlor-methyl-phenyl-, Dimethoxy-phenyl-, Methyl-methoxy-phenyl-, Chlor-methoxy-phenyl-, Brom-methoxy-phenyl-, Pyridyl-, Pyrimidyl-, Chinolyl-, Isochinolyl- oder Chinazolylgruppe,

für $R_4$ und $R_5$ die des Wasserstoffatoms, der Methyl-, Äthyl-, n-Propyl-, Isopropyl-, n-Butyl-, Isobutyl-, sec. Butyl- oder n-Pentylgruppe, wobei $R_4$ und $R_5$ zusammen mit dem Dazwischenliegenden Kohlenstoffatom eine Alkylidengruppe mit 3 bis 9 Kohlenstoffatomen darstellen,

für $R_4$ und $R_5$ zusammen mit dem dazwischenleigenden Kohlenstoffatom die der Benzyliden-, 1-Phenyl-äthyliden-, 2-Phenyl-äthyliden-, 1-Phenyl-n-propyliden-, 1-Phenyl-2,2-propyliden- oder 3-Phenyl-n-propylidengruppe und

für W die der Hydroxymethyl-, Formyl-, Carboxy-, carboxymethyl-, 2-Carboxy-äthyl-, 2-Carboxy-äthenyl-, 2,2-Bis-(carboxy)-äthyl-, Methoxycarbonyl-, Äthoxycarbonyl-, n-Propoxycarbonyl-, Isopropoxycarbonyl-, n-Butoxycarbonyl-, Isobutoxycarbonyl-, Allyloxycarbonyl-, Crotyloxycarbonyl-, (2-Hydroxy-äthoxy)carbonyl-, (2-Hydroxy-n-propoxy)carbonyl-, (1-Hydroxy-2-propoxy)carbonyl-, (2-Methoxyäthoxy)-carbonyl-, (2-Äthoxyäthoxy)carbonyl-, (2-n-Propoxyäthoxy)carbonyl-, (2-Nicotinoyloxy-äthoxy)-carbonyl-, (2-Isonicotinoyloxyäthoxy)-carbonyl-, (2,3-Dihydroxy-n-propoxy)carbonyl-, (2-Dimethylamino-äthoxy)-carbonyl-, (2-Diäthylaminoäthoxy)-carbonyl-, Methyl-, Äthyl-, n-Propyl-, Isopropyl-, Cyano-, Aminocarbonyl-, Methoxycarbonyl-methyl-, Äthoxycarbonyl-methyl-, n-Propoxycarbonyl-methyl-, 2-Methoxycarbonyl-äthyl-, 2-Äthoxycarbonyl-äthyl-, 2-Isopropoxycarbonyl-äthyl-, 2-Methoxycarbonyl-äthenyl-, 2-Äthoxycarbonyl-äthenyl-, 2-n-Propoxycarbonyl-äthenyl-, 2,2-Bis-(methoxycarbonyl)-äthyl-, 2,2-Bis (äthoxycarbonyl)-äthyl- oder 2,2-Bis-(isopropoxycarbonyl)-äthyl-gruppe

in Betracht.

Bevorzugte Verbindungen der obigen allgemeinen Formel I sind diejenigen, in denen

A eine Gruppe der Formeln

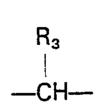 oder 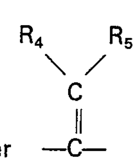

wobei

$R_3$ eine durch eine Alkoxygruppe mit 1 bis 3 Kohlenstoffatomen oder durch eine Phenylgruppe substituierte Alkylgruppe mit 1 bis 3 Kohlenstoffatomen, eine n-Propylgruppe, eine Alkylgruppe mit 4 bis 6 Kohlenstoffatomen, eine Alkenylgruppe mit 3 bis 5 Kohlenstoffatomen, eine Cyano- oder Alkleniminocarbonylgruppe mit 4 bis 6 Kohlenstoffatomen im Alkylenteil oder eine gegebenenfalls durch Alkyl- oder Phenylalkylgruppen mit jeweils 1 bis 3 Kohlenstoffatomen im Alkylteil mono-oder disubstituierte Aminocarbonylgruppe, eine durch Halogenatome, durch Alkyl-, Hydroxy-, Alkoxy-, Phenylalkoxy-, Alkylsulfenyl-, Alkylsulfinyl- und/oder Alkylsulfonylgruppen mono- oder disubstituierte Arylgruppe mit 6 oder 10 Kohlenstoffatomen, wobei die Substituenten gleich oder verschieden sein können und der Alkylteil jeweils 1 bis 3 Kohlenstoffatome enthalten kann, eine Naphthylgruppe oder eine 1 oder 2 Stickstoffatome enthaltende Heteroarylgruppe mit 4, 5, 8 oder 9 Kohlenstoffatomen,

$R_4$ und $R_5$ zusammen mit dem dazwischen liegenden Kohlenstoffatom eine Alkylidengruppe mit 3 bis 9 Kohlenstoffatomen oder eine Phenylalkylidengruppe mit 1 bis 3 Kohlenstoffatomen im Alkylidenteil,

$R_1$ eine unverzweigte Alkyleniminogruppe mit 4 bis 8 Kohlenstoffatomen oder eine durch Alkylgruppen mit 1 bis 3 Kohlenstoffatomen mono- oder disubstituierte Piperidinogruppe,

$R_2$ ein Wasserstoff-, Fluor-, Chlor- oder Bromatom, eine Nitrogruppe, eine Alkyl- oder Alkoxygruppe mit jeweils 1 bis 3 Kohlenstoffatomen,

W eine Carboxy-, Hydroxymethyl-, Carboxymethyl-, 2-Carboxyäthyl-, 2-Hydroxy-äthoxycarbonyl-, 2-Methoxy-äthoxycarbonyl-, 2-Nicotinoyloxy-äthoxycarbonyl-, 2,3-Dihydroxy-n-propoxycarbonyl- oder Alkoxycarbonylgruppe mit insgesamt 2 bis 5 Kohlenstoffatomen, eine Formyl-, Cyano-, Aminocarbonyl-, 2-Carboxy-äthenyl-, 2,2-Bis(carboxy)-äthyl-, 2-Alkoxycarbonyl-äthenyl-, 2-Alkoxycarbonyl-äthyl- oder 2,2-Bis(alkoxycarbonyl)-äthylgruppe, wobei die Alkoxygruppe jeweils 1 bis 3 Kohlenstoffatome enthalten kann, bedeuten,

insbesondere jedoch diejenigen Verbindungen der all gemeinen Formel I, in der

A eine Gruppe der Formeln

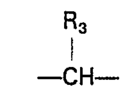 oder 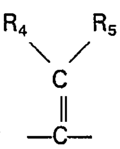

wobei

$R_3$ eine durch eine Alkoxygruppe mit 1 bis 3 Kohlenstoffatomen oder durch eine Phenylgruppe substituierte Alkylgruppe mit 1 bis 3 Kohlenstoffatomen, eine n-Propylgruppe, eine Alkylgruppe mit 4 bis 6 Kohlenstoffatomen, eine Alkenylgruppe mit 3 bis 5 Kohlenstoffatomen, eine Cyano- oder Aminocarbonylgruppe, eine durch ein Halogenatom, durch eine Alkyl-, Hydroxy-, Alkoxy-, Phenylalkoxy-, Alkylsulfenyl-, Alkylsulfinyl- oder Alkylsulfonylgruppe substituierte Phenylgruppe, wobei der Alkylteil jeweils 1 bis 3 Kohlenstoffatome enthalten kann, eine Naphthyl-, Pyridyl-, Chinolyl- oder Isochinolylgruppe,

$R_4$ und $R_5$, zusammen mit dem dazwischen liegenden Kohlenstoffatom eine Alkylidengruppe mit 3 bis 5 Kohlenstoffatomen oder eine Phenylalkylidengruppe mit 1 bis 3 Kohlenstoffatomen im Alkylidenteil,

$R_1$ eine unverzweigte Alkyleniminogruppe mit 4 bis 8 Kohlenstoffatomen oder eine durch Alkylgruppen mit 1 bis 3 Kohlenstoffatomen mono- der disubstituierte Piperidinogruppe,

$R_2$ ein Wasserstoff-, Fluor-, Chlor- oder Bromatom, eine Nitro-, Alkyl- oder Alkoxygruppe mit jeweils 1 bis 3 Kohlenstoffatomen,

W eine Carboxy-, Hydroxymerthyl-, Carboxymethyl-, Carboxymethyl-, 2-Carboxyäthyl-, 2-Hydroxy-äthoxycarbonyl-, 2-Methoxy-äthoxycarbonyl-, 2-Nicotinoyloxy-äthoxycarbonyl-, 2,3-Dihydroxy-n-propoxycarbonyl- oder Alkoxycarbonylgruppe mit insgesamt 2 bis 5 Kohlenstoffatomen, eine Formyl-, 2-Carboxy-äthenyl-, 2,2-Bis-(carboxy)-äthyl-, 2-Alkoxycarbonyl-äthenyl-, 2-Alkoxycarbonyl-äthyl-, Alkoxycarbonylmethyl oder 2,2-Bis(alkoxycarbonyl)-äthylgruppe, wobei die Alkoxygruppe jeweils 1 bis 3 Kohlenstoffatome enthalten kann, bedeuten,

deren optisch aktive Antipoden und deren Salze, insbesondere deren physiologisch verträgliche Salze mit anorganischen oder organischen Säuren oder Basen.

Eine weitere Gruppe von bevorzugten Verbindungen stellen jedoch diejenigen dar, in denen , $R_1$ und $R_2$ wie vorstehend erwähnt definiert sind, und

W eine Carboxygruppe oder eine Alkoxycarbonylgruppe mit insgesamt 2 bis 5 Kohlenstoffatomen darstellt, wobei der Alkylteil ab dem β-Kohlenstoffatom durch eine oder zwei Hydroxygruppen substituiert sein kann, sowie deren optisch aktive Antipoden und deren Salze, insbesondere deren physiologisch verträgliche Salze mit anorganischen oder organischen Säuren oder Basen.

Besondere bevorzugte Verbindungen der obigen allgemeinen Formel I sind diejenigen, in denen

A eine Gruppe der Formeln

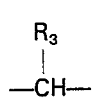 oder 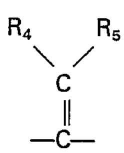

$R_3$ eine n-Propylgruppe, eine Alkylgruppe mit 4 oder 5 Kohlenstoffatomen, eine durch eine Methylgruppe, durch ein Fluor- oder Chloratom substituierte Phenylgruppe oder eine Pyridylgruppe,

$R_4$ und $R_5$ zusammen mit dem dazwischenliegenden Kohlenstoffatom eine Alkylidengruppe mit 3 bis 5 Kohlenstoffatomen oder eine Phenylalkylidengruppe mit 1 bis 3 Kohlenstoffatomen im Alkylidenteil darstellen,

$R_1$ eine gegebenenfalls durch eine oder zwei Methylgruppen substituierte Piperidinogruppe,

$R_2$ ein Wasserstoff-, Fluor- oder Chloratom, eine Methyl- oder Methoxygruppe und

W ein Carboxygruppe oder eine Alkoxycarbonylgruppe mit insgesamt 2 bis 4 Kohlenstoffatomen bedeuten,

insbesondere diejenigen, in denen

A eine Gruppe der Formeln

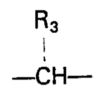 oder 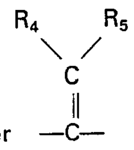

wobei

$R_3$ eine n-Propylgruppe oder eine Alkylgruppe mit 4 oder 5 Kohlenstoffatomen und

$R_4$ und $R_5$ zusammen mit dem dazwischenliegenden Kohlenstoffatom eine Alkylidengruppe mit 3 bis 5 Kohlenstoffatomen oder eine Phenylalkylidengruppe mit 1 bis 3 Kohlenstoffatomen im Alkylidenteil bedeuten,

$R_2$ ein Wasserstoff-, Fluor- oder Chloratom, eine Methyl- oder Methoxygruppe und

W eine Carboxygruppe oder eine Alkoxycarbonylgruppe mit insgesamt 2 bis 4 Kohlenstoffatomen bedeuten,

deren optisch aktive Antipoden und deren Salze, insbesondere deren physiologisch verträgliche Salze mit anorganischen oder organischen Säuren oder Basen.

Erfindungsgemäß erhält man die neuen Verbindungen nach folgenden Verfahren:

a) Umsetzung eines Amine der allgemeinen Formel

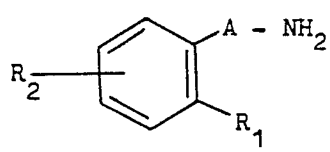 , (II)

in der

A, $R_1$ und $R_2$ wie eingangs definiert sind, bzw., wenn A eine der eingangs erwähnten Vinylidengruppen darstellt, dessen Tautomeren oder dessen Lithium- oder Magnesiumhalogenid-Komplex mit einer Carbonsäure der allgemeinen Formel

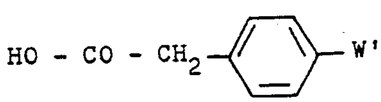 , (III)

in der

W' die für W eingangs erwähnten Bedeutungen besitzt oder eine durch einen Schutzrest geschützte Carboxygruppe darstellt, oder mit deren gegebenenfalls im Reaktionsgemisch hergestellten reaktionsfähigen Derivaten und erforderlichenfalls anschließende Abspaltung eines verwendeten Schutzrestes.

Als reaktions fähige Derivate einer Verbindung der allgemeinen Formel III kommen beispielsweise deren Ester wie der Methyl-, Äthyl- oder Benzylester, deren Thioester wie der Methylthio- oder Äthylthioester, deren Halogenide wie das Säurechlorid, deren Anhydrdide oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxy-succinimid, N,N'-Carbonyldiimidazol oder N,N'-Thionyldiimidazol oder Triphenylphosphorin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen −25°C und 250°C, vorzugsweise jedoch bei Temperaturen zwischen −10°C und der Sietemperatur des verwendeten Lösungsmittels, durchgeführt. Die Umsetzung kann auch ohne Lösungsmittel durchgefürt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Erforderlichenfalls wird die anschließende Abspaltung eines Schutzrestes vorzugsweise hydrolytisch durchgeführt, zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Äthanol, Äthanol/Wasser, Wasser/Isopropanol oder Wasser/Dioxan bei Temperaturen zwischen −10°C und 120°C, z.B. bei Temperaturen zwischen Raumtemperatur und der Siedetemperatur des Reaktionsgemisches.

Ein als Schutzrest verwendeter tert. Butylrest kann auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure abgespalten werden.

Desweiteren kann ein als Schutzrest verwendeter Benzylrest auch hydrogenolytisch in Gegenwart eines Hydrierungskatalystors wie Palladium/Kohle in einem geeigneten Lösungsmittel wie Methanol, Äthanol/Wasser, Eisessig, Essigsäureäthylester, Dioxan oder Dimethylformamid abgespalten werden.

b) Zur Herstellung einer Verbindungen der allgemeinen Formel I, in der W eine Carboxy-, Carboxymethyl-, 2-Carboxyäthyl- oder 2-Carboxyäthenylgruppe darstellt:

Spaltung einer Verbindung der allgemeinen Formel

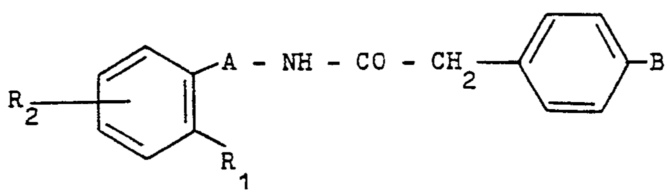 , (IV)

in der

$R_1$, $R_2$, A und X wie eingangs definiert sind und

B eine durch Hydrolyse, Thermolyse oder Hydrogenolyse in eine Carboxy-, Carboxymethyl-, 2-Carboxyäthyl- oder 2-Carboxyäthenylgruppe überführbare Gruppe darstellt.

Als hydrolysierbare Gruppen kommen beispielsweise funktionelle Derivate der Carboxy-, Carboxymethyl-, 2-Carboxyäthyl- oder 2-Carboxyäthenylgruppe wie deren unsubstituierte oder substituierte Amide, deren Nitrile, Ester, Thiolester, Orthoester, Iminoäther, Amidine oder Anhydride, eine Malonester-(1)-yl-gruppe, die Tetrazolylgruppe, eine gegebenenfalls substituierte 1,3-Oxazol-2-yl- oder 1,3-Oxazolin-2-yl-gruppe, und

als thermolytisch spaltbare Gruppen beispielsweise Ester mit tertiären Alkoholen, z.B. der tert. Butylester, und

als hydrogenolytisch spaltbare Gruppen beispielsweise Ester mit Aralkanolen, z.B. der Benzylester, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthonol, Wasser/Isopropanol oder Wasser/Dioxan bei Temperaturen zwischen −10°C und 120°C, z.B. bei Temperaturen zwischen Raumtemperatur und der Siedetemperatur des Reaktionsgemisches, durchgeführt.

Bedeutet B in einer Verbindung der allgemeinen Formel IV eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Bedeutet B in einer Verbindung der allgemeinen Formel IV beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet B in einer Verbindung der allgemeinen Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. eine Halogenatom durch ein Wasserstoffatom, ersetzt werden.

c) Zur Herstellung von Verbindungen der allgemeinen Formel I, in der A eine Gruppe der Formel

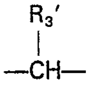

darstellt, wobei

$R_3'$ mit Ausnahme der Alkenylgruppe und der Cyanogruppe die für $R_3$ eingangs erwähnten Bedeutungen besitzt:

Reduktion eine Verbindung der allgemeinen Formel

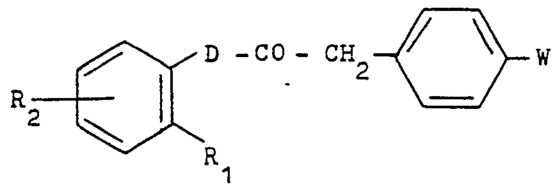

, (V)

in der

$R_1$, $R_2$ und W wie eingangs definiert sind und

D eine Gruppe der Formeln

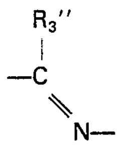

oder

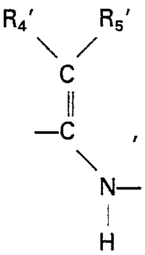

,

bedeutet, wobei $R_3''$ mit Ausnahme der Cyanogruppe die für $R_3$ eingangs erwähnten Bedeutungen besitzt und $R_4'$ und $R_5'$ zusammen mit dem dazwischen liegenden Kohlenstoffatom eine Propylidengruppe, eine Alkylidengruppe mit 4 bis 6 Kohlenstoffatomen oder eine Phenylalkylidengruppe mit 1 bis 3 Kohlenstoffatomen im Alkylidenteil bedeuten.

Die Reduktion wird vorzugsweise mit Wasserstoff in Gegenwart eines Hydrierungskatalyators wie Palladium/Kohle oder Raney-Nickel in einem geeigneten Lösungsmittel wie Methanol, Äthanol, Isopropanol, Äthanol/Wasser, Eisessig, Essigsäureäthylester, Dioxan, Tetrahydrofuran, Dimethylformamid, Benzol oder Benzol/Äthol bei Temperaturen zwischen 0 und 100°C, vorzugsweise jedoch bei Temperaturen zwischen 20°C und 50°C, und einem Wasserstoffdruck von 1 bis 5 bar durchgeführt. Bei Verwendung eines geeigneten chiralen Hydrierungskatalysators wie einem Metalligandenkomplex, z.B. einem Komplex aus μ,μ'-Dichlor-bis[1,5-cyclooctadienrhodium] und (+)- oder (−) O,O-Isopropyliden -2,3-dihydroxy-1,4-bis(diphenylphosphino)-butan (=DIOP), erfolgt die Wasserstoffanlagerung enantioselektiv. Desweiteren können bei der katalytischen Hydrierung andere Gruppen, z.B. eine Nitrogruppe zur Aminogruppe, eine Benzyloxygruppe zur Hydroxygruppe oder eine Zimtsäuregruppe zur Phenylpropionsäuregruppe, mitreduziert oder durch Wasserstoffatome, z.B. ein Halogenatom durch ein Wasserstoffatom, ersetzt werden.

d) Zur Herstellung von Verbindungen der allgemeinen Formel I, in der $R_3$ mit Ausnahme der Cyanogruppe die für $R_3$ eingangs erwähnten Bedeutungen besitzt:

Umsetzung einer Verbindung der allgemeinen Formel

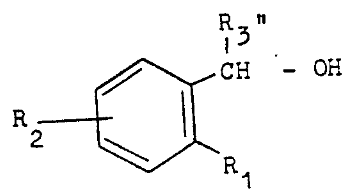

, (VI)

in der

$R_3''$ wie oben definiert ist sowie $R_1$ und $R_2$ wie eingangs definiert sind, mit einer Verbindung der allgemeinen Formel

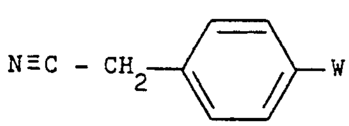

, (VII)

in der

W wie eingangs definiert ist.

Die Umsetzung wird in Gegenwart einer starken Säure, welche gleichzeitig als Lösungsmittel dienen

kann, vorzugsweise in konzentrierter Schwefelsäure, bei Temperaturen zwischen 0°C und 150°C, vorzugsweise jedoch bei Temperaturen zwischen 20°C und 100°C, durchgeführt.

e) Zur Herstellung von Verbindungen der allgemeinen Formel I, in der $R_2$ ein Wasserstoffatom und A eine Gruppe der Formel

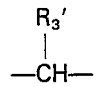

darstellen, wobei

$R_3$ wie eingangs definiert ist:

Enthalogenierung einer Verbindung der allgemeinen Formel

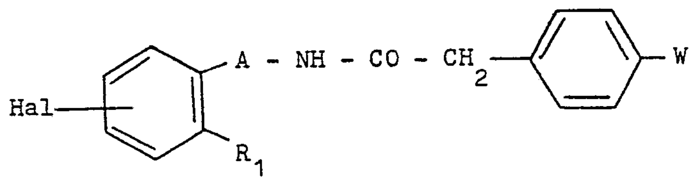

, (VIII)

in der

$R_1$, A und W wie eingangs definiert sind und

Hal eine Fluor-, Chlor-, Brom- oder Jodatom darstellt.

Die Enthalogenierung wird zweckmäßigerweise in einem Lösungsmittel wie Methanol, Äthanol, Essigester, Eisessig oder Dimethylformamid mittels katalytisch angeregtem Wasserstoff, z.B. mit Wasserstoff in Gegenwart von Platin oder Palladium/Kohle, bei Temperaturen zwischen 0 und 100°C, vorzugsweise jedoch bei Raumtemperatur, und bei einem Wasserstoffdruck von 1—5 bar durchgeführt. Bei der Enthalogenierung können gleichzeitig andere Gruppen, z.B. eine Benzyloxygruppe zur Hydroxygruppe, eine Vinylidengruppe zur entsprechenden Alkalidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

f) Zur Herstellung von Verbindungen der allgemeinen Formel I, in der $R_3$ eine Alkyleniminocarbonylgruppe mit 4 bis 6 Kohlenstoffatomen in Alkylenring oder eine gegebenenfalls durch Alkyl- oder Phenylalkylgruppen mit jeweils 1 bis 3 Kohlenstoffatomen im Alkylteil mono- oder disubstituierte Aminocarbonylgruppe darstellt:

Umsetzung einer Verbindung der allgemeinen Formel

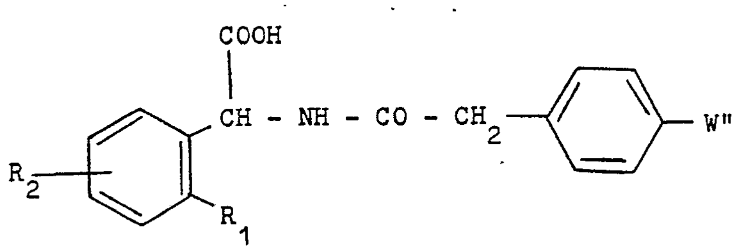

, (IX)

in der

$R_1$ und $R_2$ wie eingangs definiert sind und

W'' mit Ausnahme der Carboxygruppe die für W eingangs erwähnten Bedeutungen besitzt, mit einem Amin der allgemeinen Formel

$$H—R_6, \qquad (X)$$

in der

$R_6$ eine Alkyleniminogruppe mit 4 bis 6 Kohlenstoffatomen oder eine gegebenenfalls durch Alkyl- oder Phenylalkylgruppen mit jeweils 1 bis 3 Kohlenstoffatomen im Alkylteil mono- oder disubstituierte Aminogruppe darstellt.

Die Amidierung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, vorzugsweise in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol, N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen −25°C und 250°C, vorzugsweise jedoch bei Temperaturen zwischen −10°C und der Siedetemperatur des verwendeten Lösungsmittels, durchgeführt.

g) Zur Herstellung von Verbindungen der allgemeinen Formel I, in der W die Carboxygruppe darstellt: Oxidation einer Verbindung der allgemeinen Formel

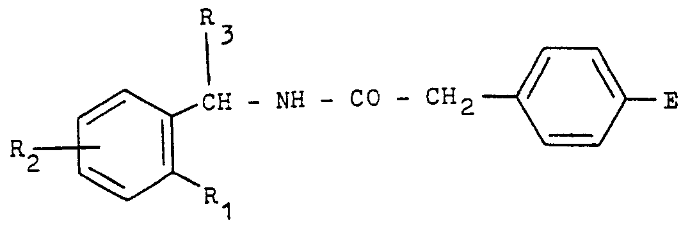

, (XI)

in der

$R_1$ bis $R_3$ wie eingangs definiert sind und

E eine durch Oxidation in eine Carboxygruppe überführbare Gruppe darstellt.

Als eine derartige oxidierbare Gruppe kommt beispielsweise die Formylgruppe und deren Acetale, die Hydroxymethylgruppe und deren Äther, eine unsubstituierte oder substituierte Acylgruppe wie die Acetyl-, Chloracetyl-, Propionyl-, die Malonsäure-(1)-yl-gruppe oder eine Malonester-(1)-yl-gruppe in Betracht.

Die Umsetzung wird mit einem Oxidationsmittel in einem geeigneten Lösungsmittel wie Wasser, Eisessig, Methylenchlorid, Dioxan oder Glykoldimethyläther bei Temperaturen zwischen 0 und 100°C, zweckmäßigerweise jedoch bei Temperaturen zwischen 20°C und 50°C, durchgeführt. Die Umsetzung wird jedoch vorzugsweise mit Silberoxid/Natronlauge, Mangandioxid/Aceton oder Methylenchlorid, Wasserstoffperoxid/Natronlauge, Brom oder Chlor/Natron- oder Kalilauge, Chromtrioxid/Pyridin oder Pyridiniumchlorchromat durchgeführt.

h) Zur Herstellung von Verbindungen der allgemeinen Formel I, in der W eine Alkoxycarbonylgruppe mit insgesamt 2 bis 6 Kohlenstoffatomen darstellt, in welcher der Alkylteil ab dem β-Kohlenstoffatom durch eine oder zwei Hydroxygruppen oder durch eine Alkoxygruppe mit 1 bis 3 Kohlenstoffatomen substituiert sein kann:

Veresterung einer Carbonsäure der allgemeinen Formel

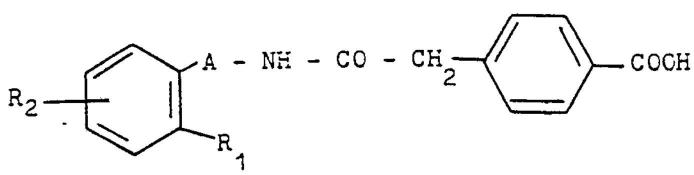

, (XII)

in der

$R_1$, $R_2$ und A wie eingangs definiert sind, oder deren gegebenenfalls im Reaktionsgemisch hergestellte reaktionsfähige Derivate, mit einem Alkohol der allgemeinen Formel

$$HO—R_7, \qquad (XIII)$$

in der

$R_7$ eine Alkylgruppe mit 1 bis 4 Kohlenstoffatomen darstellt, welche ab dem β-Kohlenstoffatom durch eine oder zwei Hydroxygruppen oder eine Alkoxygruppe mit 1 bis 3 Kohlenstoffatomen substituiert sein kann.

Als reaktionsfähige Derivate einer Verbindung der allgemeinen Formel XII kommen beispielsweise deren Halogenide wie das Säurechlorid, deren Anhydride oder Imidazolide in Betracht.

Die Umsetzung wird zweckmäßigerweise in dem entsprechenden Alkohol als Lösungsmittel oder in einem geeigneten Lösungsmittel wie Methylenchlorid, chloroform, Äther, Tetrahydrofuran, Dioxan, Benzol oder Toluol, gegebenenfalls in Gegenwart eines die Säure aktivierenden Mittels oder eines wasserentziehenden Mittels, z.B. in Gegenwart von Chlorwasserstoff, Schwefelsäure, Chlorameisensäureäthylester, Thionylchlorid, Tetrachlorkohlenstoff/Triphenylphosphin, Carbonyldiimidazol oder N,N'-Dicyclohexylcarbodiimid oder dessen Isoharnstoffäthern, gegebenenfalls in Gegenwart eines Reaktionsbeschleunigers wie Kupfer-chlorid, und gegebenenfalls in Gegenwart einer anorganischen Base wie Natriumkarbonat oder einer tertiären organischen Base wie Triäthylamin oder Pyridin, oder durch Umesterung, z.B. mit einem entsprechenden Kohlensäurediester, bei Temperaturen zwischen −20°C und 100°C, vorzugsweise jedoch bei Temperaturen zwischen −10°C und der Siedetemperatur des verwendeten Lösungsmittels, durchgeführt.

i) Zur Herstellung von Verbindungen der allgemeinen Formel I, in der W eine Alkoxycarbonyl-, Alkoxycarbonyl-methyl-, 2-Alkoxycarbonyl-äthyl- oder 2-Alkoxycarbonyl-äthenylgruppe und A eine Gruppe der Formel

$$—\overset{\displaystyle R_3''}{\overset{|}{CH}}—$$

darstellen, wobei

$R_3''$ mit Ausnahme der Cyanogruppe die für $R_3$ eingangs erwähnten Bedeutungen besitzt:

Alkoholyse einer Verbindung der allgemeinen Formel

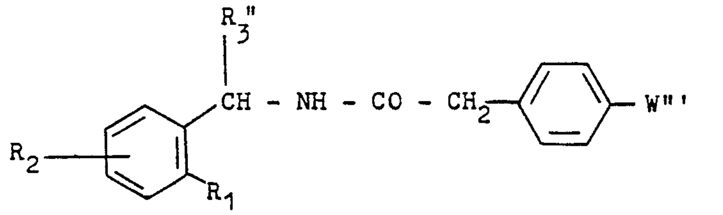

, (XIV)

in der

$R_3''$ wie vorstehend erwähnt definiert ist sowie $R_1$ und

$R_2$ wie eingangs definiert sind und

W''' eine Cyano-, Cyanomethyl-, 2-Cyanoäthyl- oder 2-Cyanoäthenylgruppe darstellt.

Die Alkoholyse wird zweckmäßigerweise in einem entsprechenden Alkohol als Lösungsmittel wie Methanol, Äthanol oder Propanol vorzugsweise in Gegenwart einer Säure wie Chlorwasserstoff oder Schwefelsäure bei Temperaturen zwischen 20°C und der Siedetemperatur des verwendeten Lösungsmittels, vorzugsweise bei Temperaturen zwischen 50 und 100°C, durchgeführt.

Erhält man erfindungsgemäß

eine Verbindung der allgemeinen Formel I, in der W eine Carboxy- oder Alkoxycarbonylgruppe darstellt, so kann diese mittels Reduktion in eine entsprechende Verbindung der allgemeinen Formel I, in der W eine Formyl- oder Hydroxymethyl-gruppe darstellt, übergeführt werden, und/oder eine Verbindung der allgemeinen Formel I, in der W die Carboxy-gruppe darstellt, so kann diese mittels Überführung in eine Sulfonsäurehydrazid und anschließende Disproportionierung in eine entsprechende Verbindung der allgemeinen Formel I, in der W die Formyl-gruppe darstellt, übergeführt werden, und/oder

eine Verbindung der allgemeinen Formel I, in der W eine Formyl-gruppe darstellt, so kann diese mittels Kondensation und gegebenenfalls anschließender Hydrolyse und/oder Decarboxylierung in eine entsprechende Verbindung der allgemeinen Formel I, in der W eine 2-Alkoxycarbonyl-äthenyl- oder eine 2-Carboxy-äthenyl-gruppe darstellt, übergeführt werden, und/oder

eine Verbindung der allgemeinen Formel I, in der W eine 2-Carboxy-äthenyl- oder 2-Alkoxycarbonyl-äthenyl-gruppe darstellt, so kann diese mittels katalytischer Hydrierung in eine entsprechende Verbindung der allgemeinen Formel I, in der W eine 2-Carboxy-äthyl- oder 2-Alkoxycarbonyl-äthyl-gruppe darstellt, übergeführt werden, und/oder

eine Verbindung der allgemeinen Formel I, in der W eine Alkoxycarbonyl-gruppe darstellt, welche ab dem β-Kohlenstoffatom durch eine Hydroxygruppe substituierte ist, so kann diese mittels Acylierung durch eine Pyridincarbonsäure in eine entsprechende (Pyridincarbonyloxyalkoxl)-carbonyl-verbindung der allgemeinen Formel I übergeführt werden, und/oder

eine Verbindung der allgemeinen Formel I, in der W eine Hydroxymethylgruppe darstellt, so kann diese, nach Überführung in eine entsprechende Halogenmethylverbindung, durch Umsetzung mit einem Malonsäurediester in eine entsprechende Verbindung der allgemeinen Formel I, in der W eine durch zwei Alkoxycarbonylgruppen substituierte Äthylgruppe darstellt, übergeführt werden, und/oder

eine Verbindung der allgemeinen Formel I, in der W eine durch zwei Alkoxycarbonylgruppen substituierte Äthylgruppe darstellt, so kann diese mittels Hydrolyse in eine entsprechende Verbindung der allgemeinen Formel I, in der W eine durch zwei Carboxygruppen substituierte Äthylgruppe darstellt, übergeführt werden, und/oder

eine Verbindung der allgemeinen Formel I, in der W eine durch zwei Alkoxycarbonylgruppen substituierte Äthylgruppe darstellt, so kann diese mittels Hydrolyse und Decarboxylierung in eine entsprechende Verbindung der allgemeinen Formel I, in der W eine 2-Carboxy-äthyl-gruppe darstellt, übergeführt werden, und/oder

eine Verbindung der allgemeinen Formel I, in der $R_2$ eine Nitrogruppe darstellt, so kann diese mittels Reduktion in eine entsprechende Verbindung der allgemeinen Formel I, in der $R_2$ eine Aminogruppe darstellt, übergeführt werden, und/oder

eine Verbindung der allgemeinen Formel I, in der $R_2$ eine Aminogruppe darstellt, so kann diese über ein entsprechendes Diazoniumsalze in eine entsprechende Verbindung der allgemeinen Formel I, in der $R_2$ ein Wasserstoff- oder Halogenatom, eine Hydroxy-, Alkoxy- oder Alkylsulfenyl-gruppe darstellt, übergeführt werden, und/oder

eine Verbindung der allgemeinen Formel I, in der $R_2$ einen Benzyloxy-rest und/oder $R_3$ einen durch einen Benzyloxyrest substituierten Arylrest darstellt, so kann diese mittels Entbenzylierung in eine entsprechende Verbindung der allgemeinen Formel I, in der $R_2$ die Hydroxygruppe und/oder $R_3$ einen durch eine Hydroxygruppe substituierten Arylrest darstellt, übergeführt werden, und/oder

eine Verbindung der allgemeinen Formel I, in der $R_3$ eine Aminocarbonylgruppe darstellt, so kann diese mittels Dehydratisierung in eine entsprechende Verbindung der allgemeinen Formel I, in der $R_3$ eine Cyanogruppe darstellt, übergeführt werden.

Die nachträgliche Reduktion wird vorzugsweise mit einem Metallhydrid, z.B. mit einem komplexen Metallhydrid wie Lithiumaluminiumhydrid, in einem geeigneten Lösungsmittel wie Diäthyläther, Tetrahydrofuran oder Dioxan bei Temperaturen zwischen 0 und 100°C, vorzugsweise jedoch bei Temperaturen zwischen 20°C und 60°C, durchgeführt.

Die nachträgliche Disproportionierung eines Sulfonsäurehydrazides, welche man durch Umsetzung eines entsprechenden Hydrazins mit einem entsprechenden reaktionsfähigen Carbonsäurederivat erhält, wird in Gegenwart einer Base wie Natriumkarbonat in einem Lösungsmittel wie Äthylenglykol bei Temperaturen zwischen 100°C und 200°C, vorzugsweise jedoch bei 160—170°C, durchgeführt.

Die nachträgliche Kondensation einer Formyl-Verbindung wird zweckmäßigerweise in einem Lösungsmittel wie Pyridin oder Tetrahydrofuran mit Malonsäure, mit einem Malonsäureester, mit einem Dialkylphosphono-essigsäureester oder einem Alkoxycarbonylmethylen-triphenyl-phosphoran gegebenenfalls in Gegenwart einer Base als Kondensationsmittel, z.B. in Gegenwart von Piperidin, Kalium-tert.butylat oder Natriumhydrid, bei Temperaturen zwischen 0 und 100°C durchgeführt; durch anschließendes Ansäuern, z.B. mit Salzsäure oder Schwefelsäure, bzw. durch anschließende alkalische Hydrolyse erhält man die gewünschte Verbindung.

Die nachträgliche katalytische Hydrierung wird zweckmäßigerweise in einem Lösungsmittel wie Methanol, Äthanol, Essigsäureäthylester, Eisessig oder Dimethylformamid mit Wasserstoff in Gegenwart eines Hydrierungskatalysators wie Platin oder Palladium/Kohle bei Temperaturen zwischen 0 und 75°C, vorzugsweise jedoch bei Raumtemperatur, und bei eine Wasserstoffdruck von 1—5 bar durchgeführt.

Die anschließende O-Acylierung wird Zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid vorzugsweise mit einem reaktiven Derivat der Säure, beispielsweise einem Halogenid wie dem Säurechlorid, einem Anhydrid oder Imidazolid und gegebenenfalls in Gegenwart einer anorganischen Base wie Natriumkarbonat oder einer tertiären organischen Base wie Triäthylamin oder Pyridin, welche gleichzeitig als Lösungsmittel dienen können, bei Temperaturen zwischen −25°C und 250°C, vorzugsweise jedoch bei Temperaturen zwischen −10°C und der Siedetemperatur des verwendeten Lösungsmittels, durchgeführt.

Die nachträgliche Überführung einer Hydroxymethyl-gruppe in eine Halogenmethylgruppe wird mit einem Halogenierungsmittel wie Thionylchlorid, Phosphortrichlorid, Phosphortribromid oder Phosphorpentachlorid in einem Lösungsmittel wie Methylenchlorid, Tetrachlorkohlenstoff, Benzol oder Nitrobenzol und deren anschließende Umsetzung mit einem Malonsäureester, z.B. mit einem Alkalisalz des Malonsäurediäthylesters, bei Temperaturen zwischen 0 und 100°C, vorzugsweise jedoch bei Temperaturen zwischen 50°C und 80°C, durchgeführt.

Die nachträgliche Hydrolyse oder Hydrolyse und Decarboxylierung wird zweckmäßigerweise in Gegenwart einer Säure wie Salzsäure, Schwefelsäure, Phosphorsäure, Polyphosphorsäure oder Trifluoressigsäure in einem geeigneten Lösungsmittel wie Wasser, Äthanol, Wasser/Äthanol, Wasser/Isopropanol oder Wasser/Dioxan bei erhöhten Temperaturen, z.B. bei der Siedetemperatur des Reaktionsgemisches, durchgefurt.

Die nachträgliche Reduktion der Nitroverbindung wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)-chlorid oder Natriumdithionit oder mit Hydrazin in Gegenwart von Raney-Nickel bei Temperaturen zwischen 0 und 50°c, vorzugsweise jedoch bei Raumtemperatur, durchgeführt.

Die nachträgliche Umsetzung eines Diazoniumsalzes, z.B. des Fluoroborats, des Fluorides in 40%iger Flußsäure, des Hydrosulfats in Schwefelsäure oder des Hydrochlorids, erforderlichenfalls in Gegenwart von Kupfer oder eines entsprechenden Kupfer-(I)-Salzes wie Kupfer-(I)-chlorid/Salzsäure oder Kupfer-(I)-bromid/Bromwasserstoffsäure, wird bei leicht erhöhten Temperaturen, z.B. bei Temperaturen zwischen 15°C und 100°C, durchgeführt; die nachträgliche Umsetzung mit unterphosphoriger Säure wird vorzugsweise bei −50°C bis 0°C durchgeführt. Das erforderliche Diazoniumsalz wird zweckmäßigerweise in einem geeigneten Lösungsmittel, z.B. in Wasser/Salzsäure, Methanol/Salzsäure, Äthanol/Salzsäure oder Dioxan/Salzsäure, durch Diazotierung einer entsprechenden Aminoverbindung mit einem Nitrit, z.B. Natriumnitrit oder einem Ester der salpetrigen Säure, bei niedrigen Temperaturen, z.B. bei Temperaturen zwischen −10°C und 5°C, hergestellt.

Die nachträgliche Entbenzylierung wird zweckmäßigerweise in einem Lösungsmittel wie Methanol, Äthanol, Essigester, Eisessig oder Dimethylformamid mittels katalytisch angeregtem Wasserstoff, z.B. mit Wasserstoff in Gegenwart von Platin oder Palladium/Kohle, bei Temperaturen zwischen 0 und 75°C, vorzugsweise jedoch bei Raumtemperatur, und bei einem Wasserstoffdruck von 1—5 bar durchgeführt.

Die nachträgliche Dehydratisierung wird mit einem wasserentziehenden Mittel wie Phosphorpentoxid, Schwefelsäure oder p-Toluolsulfonsäurechlorid gegebenenfalls in einem Lösungsmittel wie Methylenchlorid oder Pyridin bei Temperaturen zwischen 0 und 100°C, vorzugsweise bei Temperaturen zwischen 20° und 80°C, durchgeführt.

Die erhaltenen Verbindungen der allgemeinen Formel I, sofern sie ein chirales Zentrum besitzen, lessen sich desweiteren in ihre Enantiomeren nach üblichen Methoden auftrennen. Diese Trennung erfolgt durch Säulenchromatographie an einer chiralen Phase.

Die erhaltenen Verbindungen der allgemeinen Formel I lassen sich ferner in ihre Additionssalze, insbesondere in ihre physiologisch verträglichen Salze mit anorganischen oder organischen Säuren oder auch Basen überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid, Kaliumhydoxid, Cyclohexylamin, Äthanolamin, Diäthanolamin, Triäthanolamin oder Äthylendiamin in Betracht.

Die als Ausgangsstoffe verwendeten Verbindungen der allgemeinen Formeln II bis XIV erhält man nach literaturbekannten Verfahren bzw. sind literaturbekannt.

So erhält man beispielsweise eine Verbindung der allgemeinen Formel II, in der A eine Gruppe der Formel

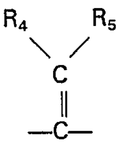

bedeutet, bzw. deren tautomeres Ketimin durch Umsetzung eines entsprechenden Nitrils mit einer entsprechenden Grignard- oder Lithium-Verbindung und anschließende Hydrolyse oder durch Umsetzung eines entsprechenden Ketons mit Ammoniak in Gegenwart von Titantetrachlorid. Zur weiteren Umsetzung mit einer Verbindung der allgemeinen Formel III bzw. deren reaktionsfähigen Derivaten, insbesondere deren Säurechloride, kann auch der metallorganische Ketimin-Komplex verwendet werden.

Eine Verbindung der allgemeinen Formel II, in der A eine Gruppe der Formel

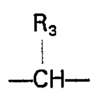

darstellt, wobei

$R_3$ mit Ausnahme der Cyano- und Aminocarbonylgruppe wie eingangs definiert ist, erhält man beispielsweise durch Umsetzung eines entsprechenden Nitrils mit einer entsprechenden Grignard- oder Lithium-Verbindung und gegebenenfalls anschließende Lithiumaluminiumhydrid-Reduktion oder anschließende Hydrolyse zum Ketimin, welches anschließend mit katalytisch angeregtem Wasserstoff, mit einem komplexen Metallhydid oder mit nascierendem Wasserstoff reduziert wird, durch Hydrolyse bzw. durch Hydrazinolyse einer entsprechenden Phthalimidoverbindung, durch Umsetzung eines entsprechenden Ketons mit Ammoniumformiat und anschließende Hydrolyse bzw. mit einem Ammoniumsalz in Gegenwart von Natriumcyanoborhydrid, durch Reduktion eines entsprechenden Oxims mit Lithiumaluminiumhydrid oder mit katalytisch angeregtem oder nascierendem Wasserstoff, durch Reduktion eines entsprechenden N-Benzyl- oder N-(1-Phenyläthyl)-ketimins, z.B. mit katalytisch angeregtem Wasserstoff oder mit einem komplexen Metallhydrid in Äther oder Tetrahydrofuran bei Temperaturen zwischen −78°C und der Siedetemperatur des verwendeten Lösungsmittels und anschließender Anspaltung der Benzyl- oder 1-Phenyläthylgruppe mittels katalytischer Hydrierung, durch Ritter-Reaktion eines entsprechenden Alkohols mit Kaliumcyanid in Schwefelsäure, oder durch Hofmann-, Curtius-, Lossen- oder Schmidt-Abbau einer entsprechenden Verbindung.

Eine Verbindung der allgemeinen Formel II, in der A die Gruppe

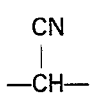

darstellt, erhält man durch Umsetzung eines entsprechenden Aldehyds mit Ammoniumcyanid oder durch Umsetzung eines entsprechenden Cyan-hydrines mit Ammoniak.

Ein so erhaltenes Amin der allgemeinen Formel II mit einem chiralen Zentrum, in der A eine Gruppe der Formel

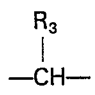

darstellt, wobei

$R_3$ mit Ausnahme der Cyano-gruppe wie eingangs definiert ist, kann durch Racematspaltung, z.B. mittels fraktionierter Kristallisation der diastereomeren Salze mit optisch aktiven Säuren und anschließende Zerlegung der Salze oder durch Säulenchromatographie an einer chiralen Phase, oder durch Bildung von diastereomeren Verbindungen, deren Trennung und anschließende Spaltung in die Enantiomeren aufgrennt werden.

Ferner kann ein optisch aktives Amin der allgemeinen Formel II auch durch enantio-selektive Reduktion eines entsprechenden Ketimins mittels komplexer Bor- oder Aluminiumhydride, in denen ein Teil der Hydridwasserstoffatome durch optisch aktive Alkoholatreste ersetzt ist, oder mittels Wasserstoff in Gegenwart eines geeigneten chiralen Hydrierungskatalysators bzw. analog ausgehend von einem entsprechenden N-Benzyl- oder N-(1-Phenäthyl)-ketimin oder von einem entsprechenden N-Acyl-ketimin bzw. Eanmid und gegebenenfalls anschließende Abspaltung des Benzyl-, 1-Phenäthyl- oder Acyl-Restes hergestellt werden.

Ferner kann ein optisch aktives Amin der allgemeinen Formel II auch durch diastereo-selektive Reduktion eines entsprechenden am Stickstoffatom chiral substituierten Ketimins oder Hydrazons mittels komplexer oder auch nichtkomplexer Bor- oder Aluminiumhydride, in denen gegebenenfalls ein Teil der Hydridwasserstoffe durch entsprechende Alkoholat-, Phenolat- oder Alkyl-Reste ersetzt ist, oder mittels Wasserstoff in Gegenwart eines geeigneten Hydrierungskatalysators und gegebenenfalls anchließende Abspaltung des chiralen Hilfsrestes durch katalytische Hydrogenolyse oder Hydrolyse hergestellt werden.

Ferner kann ein optisch aktives Amin der allgemeinen Formel II auch durch diastereo-selektive Addition einer entsprechenden metallorganischen Verbindung, vorzugsweise einer Grignard- oder einer Lithiumverbindung, an ein entsprechendes am Stickstoffatom chiral substituiertes Aldimin, durch anschließende Hydrolyse und gegebenenfalls anschließende Abspaltung des chiralen Hilfsrestes durch katalytische Hydrogenolyse oder Hydrolyse hergestellt werden.

Die als Ausgangsstoffe verwendeten Verbindungen der allgemeinen Formeln IV, VIII, IX, XII und XIV erhält man durch Umsetzung eines entsprechenden Amins mit einer entsprechenden Verbindung der allgemeinen Formel III bzw. deren reaktiven Derivaten und gegebenenfalls anschließende Hydrolyse.

Eine als Ausgansstoff verwendete Verbindung der allgemeinen Formel V erhält man vorzugsweise durch Acylierung eines entsprechenden Ketimins oder dessen metallorganischem Komplex mit einer entsprechenden Carbonsäure bzw. deren reaktiven Derivaten.

Wie bereits eingangs erwähnt, weisen die neuen Verbindungen der allgemeinen Formel I wertvolle pharmakologische Eigenschaften auf nämlich eine Wirkung auf den Intermediärstoffwechsel, insbesondere jedoch eine blutzuckersenkende Wirkung, teilweise auch eine Wirkung auf das Herz-Kreislauf-System.

Beispielsweise wurden die Verbindungen

A=(Z)-4-[(1-(2-Piperidino-phenyl)-1-buten-1-yl)-aminocarbonylmethyl]-benzoesäure,
B=(Z)-4-[(1-(2-Piperidino-phenyl)-1-buten-1-yl)-aminocarbonylmethyl]-benzoesäure-äthylester,
C=(E)-4-[(1-(2-Piperidino-phenyl)-1-buten-1-yl)-aminocarbonylmethyl]-benzoesäure,
D=4-[(2-Methyl-1-(2-piperidino-phenyl)-1-propen1-yl)-aminocarbonylmethyl]-benzoesäure,
E=(Z)-4-[(1-(2-Piperidino-phenyl)-1-hexen-1-yl)-aminocarbonylmethyl]-benzoesäure-äthylester,
F=(Z)-4-[(3-Phenyl-1-(2-piperidino-phenyl)-1-propen-1-yl)-aminocarbonylmethyl]-benzoesäure,
G=(Z)-4-[(1-(2-(3,3-Dimethyl-piperidino)-phenyl)-1-buten-1-yl)-aminocarbonylmethyl]-benzoesäure,
H=4-[(1-(2-Pyrrolidino-phenyl)-1-butyl)-aminocarbonylmethyl]-benzoesäure,
J=(±)-4-[(1-(2-Piperidino-phenyl)-1-butyl)-aminocarbonylmethyl]-benzoesäure,
K=(+)-4-[(1-(2-Piperidino-phenyl)-1-butyl)-aminocarbonylmethyl]-benzoesäure,
L=(+)-4-[(1-(2-Piperidino-phenyl)-1-butyl)-aminocarbonylmethyl]-benzoesäure-äthylester,
M=4-[(1-(2-Hexahydroazepino-phenyl)-1-butyl)-aminocarbonylmethyl]-benzoesäure,
N=4-[(1-(2-Piperidino-phenyl)-1-hexyl)-aminocarbonylmethyl]-benzoesäure,
O=4-[(3-Phenyl-1-(2-piperidino-phenyl)-1-propyl)-aminocarbonylmethyl]-benzoesäure,
P=4-[(2-Methoxy-1-(2-piperidino-phenyl)-1-äthyl)-aminocarbonylmethyl]-benzoesäure,
Q=4-[(α-Cyano-2-piperidino-benzyl)-1-äthyl)-aminocarbonylmethyl]-benzoesäure,
R=4-[(1-(2-Piperidino-phenyl)-1-butyl)-aminocarbonylmethyl]-benzylalkohol,
S=4-[(1-(2-Piperidino-phenyl)-1-butyl)-aminocarbonylmethyl]-phenylessigsäure,
T=4-[(1-(2-Piperidino-phenyl)-1-butyl)-aminocarbonylmethyl]-zimtsäure,
U=4-[(1-(2-Piperidino-phenyl)-1-butyl)-aminocarbonylmethyl]-benzoesäure-(2,3-dihydroxy-propyl)ester,
V=4-[(1-(4-Fluor-2-piperidino-phenyl)-1-butyl)-aminocarbonylmethyl]-benzoesäure,
W=4-[(1-(4-Methoxy-2-piperidino-phenyl)-1-butyl)-aminocarbonylmethyl]-benzoesäure,
X=4-[(α-(4-Methyl-phenyl)-2-piperidino-benzyl)-aminocarbonylmethyl]-benzoesäure,
Y=4-[(α-(3-Methyl-phenyl)-2-piperidino-benzyl)-aminocarbonylmethyl]-benzoesäure,
Z=4-[(α-(4-Fluor-phenyl)-2-piperidino-benzyl)-aminocarbonylmethyl]-benzoesäure,
AA=4-[(α-(4-Fluor-phenyl)-2-piperidino-benzyl)-aminocarbonylmethyl]-benzoesäure,
AB=4-[(α-(4-Chlor-phenyl)-2-piperidino-benzyl)-aminocarbonylmethyl]-benzoesäure,
AC=4-[(α-(3-Chlor-phenyl)-2-piperidino-benzyl)-aminocarbonylmethyl]-benzoesäure,
AD=4-[(2-Piperidino-α-(2-pyridyl)-benzyl)-aminocarbonylmethyl]-benzoesäure,
AE=4-[(2-Piperidino-α-(4-pyridyl)-benzyl)-aminocarbonylmethyl]-benzoesäure,
AF=4-[(6-Chlor-α-phenyl-2-piperidino-benzyl)-aminocarbonylmethyl]-benzoesäure,
AG=4-[(α-Phenyl-2-piperidino-benzyl)-aminocarbonylmethyl]-zimtsäure,
AH=4-[(4-Chlor-α-phenyl-2-piperidino-benzyl)-aminocarbonylmethyl]-benzoesäure,
AJ=4-[(6-Methyl-α-phenyl-2-piperidino-benzyl)-aminocarbonylmethyl]-benzoesäure,

AK=4-[(4-Methyl-α-phenyl-2-piperidino-benzyl)-aminocarbonylmethyl]-benzoesäure,
AL=4-[(α-Phenyl-2-piperidino-benzyl)-aminocarbonylmethyl]-benzaldehyd,
AM=4-[(2-Methyl-piperidino)-α-phneyl-benzyl)-aminocarbonylmethyl]-benzoesäure,
AN=4-[(2-(3-Methyl-piperidino)-α-phneyl-benzyl)-aminocarbonylmethyl]-benzowsäure und
AO=4-[(3-Chlor-α-phenyl-2-piperidino-benzyl)-aminocarbonylmethyl]-benzoesäure
auf ihre Eigenschaften wie folgt untersucht:

1. Blutzuckersenkende Wirkung

Die blutzuckersenkende Wirkung der zu untersuchenden Substanzen wurde an weiblichen Ratten eigener Zucht mit dem Gewicht von 180—220 g geprüft, welche 24 Stunden vor Versuchsbeginn nüchtern gesetzt wurden. Die zu untersuchenden Substanzen wurden unmittelbar vor Versuchsbeginn in 1,5%iger Methylcellulose suspendiert und per Schlundsonde appliziert.

Die Bluntentnahme erfolgte unmittelbar vor Substanzapplikation sowie 1, 2, 3 und 4 Stunden danach, jeweils aus dem retroorbitalen Venenplexus. Hiervon wurden jeweils 50 µl mit 0,5 ml 0,33 N Perchlorsäure enteiweißt und zentifugiert. Im Überstand wurde Glukose nach der Hexokinase-Methode mit Hilfe eines Aanltsenphotometers bestimmt. Die Statistische Auswertung erfolgte nach dem t-Test nach Student mit p=0,05 als Signifikanzgrenze.

Die nachfolgende Tabella enthält die gefundenen Werte in Prozent gegeüber Kontrolle:

| Substanz | 5 mg/kg | | | | 1 mg/kg | | | |
|---|---|---|---|---|---|---|---|---|
| | 1 | 2 | 3 | 4 | 1 | 2 | 3 | 4 |
| A | | | | | −43 | −40 | −33 | −35 |
| B | −44 | −39 | −26 | −35 | −39 | −19 | −26 | −30 |
| C | | | | | −43 | −43 | −37 | −38 |
| D | | | | | −36 | −32 | −27 | −25 |
| E | −46 | −40 | −38 | −26 | −23 | −23 | −12 | −18 |
| F | −43 | −42 | −39 | −32 | | | | |
| G | | | | | −44 | −42 | −37 | −31 |
| H | −50 | −46 | −44 | −45 | | | | |
| J | −44 | −37 | −42 | −42 | −38 | −32 | −34 | −29 |
| K | | | | | −41 | −43 | −38 | −31 |
| L | −42 | −45 | −31 | −22 | −14 | −18 | −14 | n.s. |
| M | −46 | −43 | −40 | −36 | −33 | −30 | −21 | n.s. |
| N | −42 | −42 | −37 | −33 | | | | |
| O | −38* | −31* | n.s.* | n.s.* | | | | |
| P | −49 | −43 | −34 | −22 | −37 | −19 | n.s. | n.s. |
| Q | −28 | −13 | n.s. | n.s. | | | | |
| R | −38 | −40 | −35 | −29 | −39 | −34 | −29 | −24 |
| S | −49 | −42 | −30 | −17 | −29 | −20 | −10 | n.s. |
| T | −48 | −46 | −42 | −40 | −42 | −42 | −40 | −32 |
| U | −43 | −43 | −49 | −45 | −39 | −35 | −29 | −24 |

| Substanz | 5 mg/kg | | | | 1 mg/kg | | | |
|---|---|---|---|---|---|---|---|---|
| | 1 | 2 | 3 | 4 | 1 | 2 | 3 | 4 |
| V | −45 | −41 | −46 | −40 | −37 | −23 | −30 | −18 |
| W | −46 | −45 | −39 | −37 | −36 | −25 | −16 | n.s. |
| X | −30 | −33 | −14 | n.s. | −15 | −15 | −13 | n.s. |
| Y | −43 | −38 | −36 | −27 | −26 | −15 | n.s. | n.s. |
| Z | −36 | −37 | −36 | −33 | | | | |
| AA | −28 | −32 | −27 | −28 | −16 | −20 | −17 | −14 |
| AB | −30 | −28 | −39 | −36 | −21 | −20 | −22 | −n.s. |
| AC | −43 | −39 | −30 | −26 | −17 | −19 | n.s. | n.s. |
| AD | −49* | −50* | −36* | −31* | −18 | n.s. | n.s. | n.s. |
| AE | −41 | −37 | −20 | n.s. | −26 | −14 | n.s. | n.s. |
| AF | −44 | −40 | −39 | −40 | −35 | −34 | −28 | −20 |
| AG | −48* | −47* | −40* | −45* | −32 | −19 | −10 | −17 |
| AH | −34 | −35 | −32 | −29 | −11 | −13 | n.s. | n.s. |
| AJ | −39 | −35 | −27 | −26 | −27 | −24 | n.s. | n.s. |
| AK | −37 | −34 | −32 | −31 | −21 | −17 | −15 | −11 |
| AL | | | | | −26 | −28 | −22 | −17 |
| AM | −32 | −31 | −24 | −19 | −16 | −11 | n.s. | n.s. |
| AN | −35 | −30 | −29 | −31 | −13 | −9 | n.s. | n.s. |
| AO | −45 | −44 | −42 | −32 | −21 | −13 | n.s. | n.s. |

* = bei 10 mg/kg
n.s. = statistisch nicht signifikant

2. Akute Toxizität:

Bei weiblichen und männlichen Mäusen eigener Zucht mit dem Gewicht von 20—26 g wurde die toxische Wirkung nach oraler Gabe (Suspension in 1%iger Methylcellulose) einer einmaligen Dosis bei einer Nachbeobachtungszeit von 14 Tagen geprüft:

| Substanz | orientierende akute Toxizität |
|---|---|
| A | > 1 000 mg/kg p.o. (0 von 6 Tieren gestorben) |
| C | > 2 000 mg/kg p.o. (0 von 6 Tieren gestorben) |
| D | > 500 mg/kg p.o. (0 von 6 Tieren gestorben) |
| J | > 2 000 mg/kg p.o. (0 von 10 Tieren gestorben) |
| X | > 1 000 mg/kg p.o. (0 von 10 Tieren gestorben) |
| Y | > 1 000 mg/kg p.o. (0 von 10 Tieren gestorben) |
| Z | > 1 000 mg/kg p.o. (0 von 10 Tieren gestorben) |
| AA | > 1 000 mg/kg p.o. (0 von 10 Tieren gestorben) |
| AB | > 1 000 mg/kg p.o. (0 von 10 Tieren gestorben) |
| AD | > 1 000 mg/kg p.o. (0 von 10 Tieren gestorben) |

Aufgrund ihrer pharmakologischen Eigenschaften eignen sich die erfindungsgemäß hergestellten Verbindungen der allgemeinen Formel I und deren physiologisch verträglichen Salze zur Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, Pulver oder Suspensionen einarbeiten. Die Einzeldosis am Erwachsenen beträgt hierbei 1—50 mg, vorzugsweise jedoch 2,5—20 mg, 1 oder 2 mal täglich.

Die nachfolgenden Beispiele sollen die Erfindung näher erläutern:

Beispiel 1

4-[N-[α-(4-Methyl-phenyl)-2-piperidino-benzyl]-aminocarbonylmethyl]-benzoesäure-äthylester

Zu, 4,2 g (15 mMol) α-(4-Methyl-phenyl)-2-piperidino-benzylamin und 3,4 g (16,5 mMol) 4-Äthoxycarbonyl-phenylessigsäure, gelöst in 40 ml Acetonitril, gibt man nacheinander 4,7 g (18 mMol) Triphenylphosphin, 3 g (30 mMol) Triäthylamin und 1,5 ml (15 mMol) Tetrachlorkohlenstoff. Das Reaktionsgemisch wird 2 Stunden bei 50°C gerührt, dann eingeengt und nach Ansäuern mit 6N Salzsäure mit Essigsäure-äthyl-ester extrahiert. Die saure wässrige Phase wird anschließend mit Methylenchlorid mehrmals extrahiert. Die Methylenchloridextrakte werden mit Natriumbikarbonatlösung gewaschen, über Magnesiumsulfat getrocknet und eingeengt. Der Einengungsrückstand wird mit Äthanol verrieben und abgesaugt.

Ausbeute: 4,55 g (65% der Theorie),
Schmelzpunkt: 177—178°C

| | | | |
|---|---|---|---|
| Ber.: | C 76,57 | H 7,28 | N 5,95 |
| Gef.: | 76,19 | 7,16 | 5,82 |

Analog Beispiel 1 wurden hergestellt:

(a) 4-[N-[α-(3-Methyl-phenyl)-2-piperidino-benzyl]-aminocarbonyl-methyl]-benzoesäure-äthylester
Ausbeute: 48% der Theorie,
Schmelzpunkt: 159—160°C

| | | | |
|---|---|---|---|
| Ber.: | C 76,57 | H 7,28 | N 5,95 |
| Gef.: | 76,80 | 7,35 | 5,76 |

(b) 4-[N-[α-(2-Methyl-phenyl)-2-piperidino-benzyl]-aminocarbonylmethyl]-benzoesäure-äthylester
Ausbeute: 35,4% der Theorie,
Schmelzpunkt: 196—198°C

| | | | |
|---|---|---|---|
| Ber.: | C 76,57 | H 7,28 | N 5,95 |
| Gef.: | 76,65 | 7,35 | 5,90 |

(c) 4-[N-[α-(4-Methoxy-phenyl)-2-piperidino-benzyl]-aminocarbonylmethyl]-benzoesäure-äthylester
Ausbeute: 45% der Theorie,
Schmelzpunkt: 167—168°C

| | | | |
|---|---|---|---|
| Ber.: | C 74,05 | H 7,04 | N 5,76 |
| Gef.: | 73,72 | 6,99 | 5,62 |

(d) 4-[N-[α-(4-Benzyloxy-phenyl)-2-piperidino-benzyl]-aminocarbonylmethyl]-benzoesäure-äthylester
Ausbeute: 96% der Theorie,
Schmelzpunkt: 154—155°C

| | | | |
|---|---|---|---|
| Ber.: | C 76,84 | H 6,81 | N 4,98 |
| Gef.: | 76,82 | 6,68 | 5,03 |

(e) 4-[N-[α-(4-Fluor-phenyl)-2-piperidino-benzyl]-aminocarbonylmethyl]-benzoesäure-äthylester
Ausbeute: 58% der Theorie,
Schmelzpunkt: 174—176°C

| | | | |
|---|---|---|---|
| Ber.: | C 73,40 | H 6,58 | N 5,90 |
| Gef.: | 73,55 | 6,72 | 5,91 |

(f) 4-[N-[α-(2-Fluor-phenyl)-2-piperidino-benzyl]-aminocarbonylmethyl]-benzoesäure-äthylester
Ausbeute: 83% der Theorie,
Schmelzpunkt: 173—175°C

| | | | |
|---|---|---|---|
| Ber.: | C 73,40 | H 6,58 | N 5,90 |
| Gef.: | 73,61 | 6,62 | 5,85 |

(g) 4-[N-[α-(4-Chlor-phenyl)-2-piperidino-benzyl]-aminocarbonylmethyl]-benzoesäure-äthylester
Ausbeute: 57% der Theorie,
Schmelzpunkt: 178—181°C

| | | | | |
|---|---|---|---|---|
| Ber.: | C 70,94 | H 6,36 | N 5,71 | Cl 7,22 |
| Gef.: | 71,10 | 6,56 | 5,26 | 7,11 |

(h) 4-[N-[α-(3-Chlor-phenyl)-2-piperidino-benzyl]-aminocarbonylmethyl]-benzoesäure-äthylester
Ausbeute: 71% der Theorie,
Schmelzpunkt: 153—156°C

| | | | | |
|---|---|---|---|---|
| Ber.: | C 70,94 | H 6,36 | N 5,71 | Cl 7,22 |
| Gef.: | 70,86 | 6,26 | 5,65 | 7,25 |

(i) 4-[N-[α-(2-Chlor-phenyl)-2-piperidino-benzyl]-aminocarbonylmethyl]-benzoesäure-äthylester
Ausbeute: 66% der Theorie,
Schmelzpunkt: 196—198°C

| | | | | |
|---|---|---|---|---|
| Ber.: | C 70,94 | H 6,36 | N 5,71 | Cl 7,22 |
| Gef.: | 70,90 | 6,30 | 5,61 | 7,10 |

(k) 4-[N-[α-(4-Methylmercapto-phenyl)-2-piperidino-benzyl]-aminocarbonylmethyl]-benzoesäure-äthylester
Ausbeute: 84% der Theorie,
Schmelzpunkt: 173—175°C

| | | | | |
|---|---|---|---|---|
| Ber.: | C 71,68 | H 6,82 | N 5,57 | Cl 6,38 |
| Gef.: | 71,92 | 6,97 | 5,45 | 6,21 |

(l) 4-[N-[5-Chlor-α-(2-chlor-phenyl)-2-piperidino-benzyl]-aminocarbonylmethyl]-benzoesäure-äthylester
Ausbeute: 92% der Theorie,
Schmelzpunkt: 213—215°C

| | | | | |
|---|---|---|---|---|
| Ber.: | C 66,28 | H 5,75 | N 5,33 | Cl 13,49 |
| Gef.: | 66,45 | 5,86 | 5,25 | 13,51 |

(m) 4-[N-[2-Piperidino-α-(2-pyridyl)-benzyl]-aminocarbonylmethyl]-benzoesäure-äthylester
Ausbeute: 51% der Theorie,
Schmelzpunkt: 158—159°C

| | | | |
|---|---|---|---|
| Ber.: | C 73,50 | H 6,83 | N 9,18 |
| Gef.: | 73,40 | 6,95 | 9,10 |

(n) 4-[N-[2-Piperidino-α-(3-pyridyl)-benzyl]-aminocarbonylmethyl]-benzoesäure-äthylester
Ausbeute: 85% der Theorie,
Schmelzpunkt: 172°C

| | | | |
|---|---|---|---|
| Ber.: | C 73,50 | H 6,83 | N 9,18 |
| Gef.: | 73,42 | 6,76 | 9,25 |

(o) 4-[N-[2-Piperidino-α-(4-pyridyl)-benzyl]-aminocarbonylmethyl]-benzoesäure-äthylester
Ausbeute: 20% der Theorie,
Schmelzpunkt: 150—152°C

| | | | |
|---|---|---|---|
| Ber.: | C 73,50 | H 6,83 | N 9,18 |
| Gef.: | 73,61 | 6,91 | 9,15 |

(p) 4-[N-(6-Chloro-α-phenyl-2-piperidino-benzyl)-aminocarbonylmethyl]-benzoesäure-äthylester
Ausbeute: 12% der Theorie,
Schmelzpunkt: Öl
Ber.: Molpeak m/e = 490/492
Gef.: Molpeak m/e = 490/492

(q) 4-[N-(4-Chlor-α-phenyl-2-piperidino-benzyl)-aminocarbonylmethyl]-benzoesäure-äthylester
Ausbeute: 37% der Theorie,
Schmelzpunkt: 148—150°C

| | | | | |
|---|---|---|---|---|
| Ber.: | C 70,94 | H 6,36 | N 5,71 | Cl 7,22 |
| Gef.: | 70,81 | 6,25 | 5,61 | 7,12 |

(r) 4-[N-(3-Chlor-α-phenyl-2-piperidino-benzyl)-aminocarbonylmethyl]-benzoesäure-äthylester
Ausbeute: 74% der Theorie,
Schmelzpunkt: 176—178°C

| | | | | |
|---|---|---|---|---|
| Ber.: | C 70,94 | H 6,36 | N 5,71 | Cl 7,22 |
| Gef.: | 70,59 | 6,25 | 5,68 | 7,16 |

(s) 4-[N-(6-Methyl-α-phenyl-2-piperidino-benzyl)-aminocarbonylmethyl]-benzoesäure-äthylester
Ausbeute: 65% der Theorie,
Schmelzpunkt: Öl
Ber.: Molpeak m/e = 470
Gef.: Molpeak m/e = 470

(t) 4-[N-[4-Methyl-α-phenyl-2-piperidino-benzyl)-aminocarbonylmethyl]-benzoesäure-äthylester
Ausbeute: 76% der Theorie,
Schmelzpunkt: 133—135°C

| | | | |
|---|---|---|---|
| Ber.: | C 76,57 | H 7,28 | N 5,95 |
| Gef.: | 76,51 | 7,16 | 5,83 |

(u) 4-[N-[5-Chlor-2-(2-methyl-piperidino)-α-phenyl-benzyl]-aminocarbonylmethyl]-benzoesäure-äthylester
Ausbeute: 36,5% der Theorie,
Schmelzpunkt: 171—173°C

| | | | | |
|---|---|---|---|---|
| Ber.: | C 71,24 | H 6,58 | N 5,54 | Cl 7.01 |
| Gef.: | 71,45 | 6,68 | 5,59 | 7,20 |

## Beispiel 2

### 4-[N-[α-(4-Chlor-phenyl)-2-piperidino-benzyl]-aminocarbonylmethyl]-benzoesäure-äthylester

Zu einer Lösung von 6,02 g (20 mMol) α-(4-Chlor-phenyl)-2-piperidino-benzylamin und 3,5 ml (25 mMol) Triäthylamin in 50 ml Chloroform tropft man unter Eiskühlung eine Lösung von 5 g (22,1 mMol) 4-Äthoxycarbonyl-phenylacetylchlorid in 20 ml Chloroform. Man rührt zwei Stunden bei Raumtemperatur, gibt dann auf Wasser und extrahiert mit Chloroform. Die Extrakte werden getrocknet und eingeengt. Der Einengungsrückstand wird an Kieselgel mit Toluol/Essigsäure-äthylester 5:1 als Fließmittel chromatographiert.

Ausbeute: 5,6 g (57% der Theorie),
Schmelzpunkt: 178—181°C

| | | | | |
|---|---|---|---|---|
| Ber.: | C 70,94 | H 6,36 | N 5,71 | Cl 7,22 |
| Gef.: | 71,09 | 6,47 | 5,61 | 7,10 |

Analog Beispiel 2 wurde hergestellt:

(a) 4-[N-[5-Chlor-2-(3-methyl-piperidino)-α-phenyl-benzyl]-aminocarbonylmethyl]-benzoesäure-äthylester
Ausbeute: 54% der Theorie,
Schmelzpunkt: 178—180°C

| | | | | |
|---|---|---|---|---|
| Ber.: | C 71,24 | H 6,58 | N 5,54 | Cl 7,01 |
| Gef.: | 70,91 | 6,64 | 5,75 | 7,01 |

Beispiel 3

4-[N-[α-(4-Methyl-phenyl)-2-piperidino-benzyl]-aminocarbonylmethyl]-benzoesäure

4,4 g (9,35 mMol) 4-[N-[α-(4-Methyl-phenyl)-2-piperidino-benzyl]-aminocarbonylmethyl]-benzoesäure-äthylester werden in 150 ml Äthanol unter Erwärmen gelöst. Anschließend gibt man 20 ml 1 N Natronlauge zu und rührt 3 Stunden bei 50°C. Zur Reaktionsmischung gibt man dann 20 ml 1 N Salzsäure und entfernt überschüssiges Äthanol durch Einengen am Rotationsverdampfer. Die verbliebende wässrige Suspension wird filtriert und der Niederschlag gut mit Wasser gewaschen. Anschließend wird aus Acetonitril umkristallisiert.

Ausbeute: 2,45 g (59,3% der Theorie),
Schmelzpunkt: 226—228°C

| | | | |
|---|---|---|---|
| Ber.: | C 75,99 | H 6,83 | N 6,33 |
| Gef.: | 75,60 | 6,75 | 6,29 |

Analog Beispiel 3 wurden hergestellt:

(a) 4-[N-[α-(3-Methyl-phenyl)-2-piperidino-benzyl]-aminocarbonylmethyl]-benzoesäure
Ausbeute: 72% der Theorie,
Schmelzpunkt: 202—203°C

| | | | |
|---|---|---|---|
| Ber.: | C 75,99 | H 6,83 | N 6,33 |
| Gef.: | 75,64 | 6,91 | 6,37 |

(b) 4-[N-[α-(2-Methyl-phenyl)-2-piperidino-benzyl]-aminocarbonylmethyl]-benzoesäure
Ausbeute: 42,6% der Theorie,
Schmelzpunkt: 285—290°C

| | | | |
|---|---|---|---|
| Ber.: | C 75,99 | H 6,83 | N 6,33 |
| Gef.: | 76,05 | 6,98 | 6,25 |

(c) 4-[N-[α-(4-Methoxy-phenyl)-2-piperidino-benzyl]-aminocarbonylmethyl]-benzoesäure
Ausbeute: 72,4% der Theorie,
Schmelzpunkt: 228—230°C

| | | | |
|---|---|---|---|
| Ber.: | C 73,34 | H 6,59 | N 6,11 |
| Gef.: | 73,22 | 6,61 | 6,13 |

(d) 4-[N-[α-(4-Benzyloxy-phenyl)-2-piperidino-benzyl]-aminocarbonylmethyl]-benzoesäure
Ausbeute: 57% der Theorie,
Schmelzpunkt: 219—221°C

| | | | |
|---|---|---|---|
| Ber.: | C 76,38 | H 6,41 | N 5,24 |
| Gef.: | 76,05 | 6,44 | 5,24 |

(e) 4-[N-[α-(4-Fluor-phenyl)-2-piperidino-benzyl]-aminocarbonylmethyl]-benzoesäure
Ausbeute: 75% der Theorie,
Schmelzpunkt: 238—240°C

| | | | |
|---|---|---|---|
| Ber.: | C 72,63 | H 6,09 | N 6,27 |
| Gef.: | 72,98 | 6,29 | 6,32 |

(f) 4-[N-[α-(2-Fluor-phenyl)-2-piperidino-benzyl]-aminocarbonylmethyl]-benzoesäure
Ausbeute: 87% der Theorie,
Schmelzpunkt: 280—283°C

| | | | |
|---|---|---|---|
| Ber.: | C 72,63 | H 6,09 | N 6,27 |
| Gef.: | 72,70 | 6,10 | 6,37 |

(g) 4-[N-[α-(4-Chlor-phenyl)-2-piperidino-benzyl]-aminocarbonylmethyl]-benzoesäure
Ausbeute: 89% der Theorie,
Schmelzpunkt: 241—242°C

| | | | | |
|---|---|---|---|---|
| Ber.: | C 70,05 | H 5,88 | N 6,05 | Cl 7,66 |
| Gef.: | 69,74 | 6,05 | 6,01 | 7,64 |

(h) 4-[N-[α-(3-Chlor-phenyl)-2-piperidino-benzyl]-aminocarbonylmethyl]-benzoesäure
Ausbeute: 53% der Theorie,
Schmelzpunkt: 223—225°C

| | | | | |
|---|---|---|---|---|
| Ber.: | C 70,05 | H 5,88 | N 6,05 | Cl 7,66 |
| Gef.: | 70,28 | 5,98 | 5,78 | 7,84 |

(i) 4-[N-[α-(2-Chlor-phenyl)-2-piperidino-benzyl]-aminocarbonylmethyl]-benzoesäure
Ausbeute: 98% der Theorie,
Schmelzpunkt: 303—305°C

| | | | | |
|---|---|---|---|---|
| Ber.: | C 70,05 | H 5,88 | N 6,05 | Cl 7,66 |
| Gef.: | 69,88 | 6,05 | 5,87 | 7,74 |

(k) 4-[N-[α-(4-Methylmercapto-phenyl)-2-piperidino-benzyl]-aminocarbonylmethyl]-benzoesäure
Ausbeute: 84,6% der Theorie,
Schmelzpunkt: 225—227°C

| | | | | |
|---|---|---|---|---|
| Ber.: | C 70,86 | H 6,37 | N 5,90 | Cl 6,75 |
| Gef.: | 70,34 | 6,37 | 5,68 | 6,82 |

(l) 4-[N-[5-Chlor-α-(2-chlor-phenyl)-2-piperidino-benzyl]-aminocarbonylmethyl]-benzoesäure
Ausbeute: 90% der Theorie,
Schmelzpunkt: 317—320°C

| | | | | |
|---|---|---|---|---|
| Ber.: | C 65,19 | H 5,27 | N 5,63 | Cl 14,25 |
| Gef.: | 64,87 | 5,34 | 5,69 | 14,22 |

(m) 4-[N-[2-Piperidino-α-(2-pyridyl)-benzyl]-aminocarbonylmethyl]-benzoesäure
Ausbeute: 81% der Theorie,
Schmelzpunkt: 160—161°C

| | | | |
|---|---|---|---|
| Ber.: | C 72,71 | H 6,34 | N 9,78 |
| Gef.: | 72,43 | 6,39 | 10,00 |

(n) 4-[N-[2-Piperidino-α-(3-pyridy)l-benzyl]-aminocarbonylmethyl]-benzoesäure
Ausbeute: 72% der Theorie,
Schmelzpunkt: 252—253°C

| | | | |
|---|---|---|---|
| Ber.: | C 72,71 | H 6,34 | N 9,78 |
| Gef.: | 72,56 | 6,53 | 9,60 |

(o) 4-[N-[2-Piperidino-α-(4-pyridyl)-benzyl]-aminocarbonylmethyl]-benzoesäure
Ausbeute: 68,5% der Theorie,
Schmelzpunkt: ab 260°C (Zersetzung)

| | | | |
|---|---|---|---|
| Ber.: | C 72,71 | H 6,34 | N 9,78 |
| Gef.: | 72,31 | 6,29 | 9,63 |

(p) 4-[N-(6-Chlor-α-phenyl-2-piperidino-benzyl)-aminocarbonylmethyl]-benzoesäure
Ausbeute: 82% der Theorie,
Schmelzpunkt: 91—94°C

| | | | | |
|---|---|---|---|---|
| Ber.: | C 70,04 | H 5,88 | N 6,05 | Cl 7,66 |
| Gef.: | 69,61 | 5,77 | 5,96 | 7,78 |

(q) 4-[N-(4-Chlor-α-phenyl-2-piperidino-benzyl)-aminocarbonylmethyl]-benzoesäure
Ausbeute: 61% der Theorie,
Schmelzpunkt: 221—223°C

| | | | | |
|---|---|---|---|---|
| Ber.: | C 70,05 | H 5,88 | N 6,05 | Cl 7,66 |
| Gef.: | 69,73 | 5,89 | 5,87 | 7,52 |

(r) 4-[N-(3-Chlor-α-phenyl-2-piperidino-benzyl)-aminocarbonylmethyl]-benzoesäure
Ausbeute: 83% der Theorie,
Schmelzpunkt: 210—213°C

| | | | | |
|---|---|---|---|---|
| Ber.: | C 70,05 | H 5,88 | N 6,05 | Cl 7,66 |
| Gef.: | 70,31 | 6,03 | 5,90 | 7,79 |

(s) 4-[N-(6-Methyl-α-phenyl-2-piperidino-benzyl)-aminocarbonylmethyl]-benzoesäure
Ausbeute: 64% der Theorie,
Schmelzpunkt: 165—170°C (Sinterung ab 150°C)

| | | | |
|---|---|---|---|
| Ber.: | C 75,99 | H 6,83 | N 6,33 |
| Gef.: | 75,73 | 6,96 | 6,14 |

(t) 4-[N-(4-Methyl-α-phenyl-2-piperidino-benzyl)-aminocarbonylmethyl]-benzoesäure
Ausbeute: 96% der Theorie,
Schmelzpunkt: 202—203°C

| | | | |
|---|---|---|---|
| Ber.: | C 75,99 | H 6,83 | N 6,33 |
| Gef.: | 76.04 | 6,78 | 6,23 |

(u) 4-[N-[5-Chlor-2-(2-methyl-piperidino)-α-phenyl-benzyl]-aminocarbonylmethyl]-benzoesäure
Ausbeute: 52% der Theorie,
Schmelzpunkt: 280—282°C

| | | | | |
|---|---|---|---|---|
| Ber.: | C 70,50 | H 6,13 | N 5,87 | Cl 7,43 |
| Gef.: | 70.14 | 6,10 | 5,75 | 7,45 |

(v) 4-[N-[5-Chlor-2-(3-methyl-piperidino)-α-phenyl-benzyl]-aminocarbonylmethyl]-benzoesäure
Ausbeute: 66% der Theorie,
Schmelzpunkt: 246—248°C

| | | | | |
|---|---|---|---|---|
| Ber.: | C 70,50 | H 6,13 | N 5,87 | Cl 7,43 |
| Gef.: | 70.16 | 6,07 | 5,87 | 7,30 |

Beispiel 4

4-[N-[α-(4-Hydroxy-phenyl)-2-piperidino-benzyl]-aminocarbonylmethyl]-benzoesäure

1,1 g (2 mMol) 4-[N-[α-(4-Benzyloxy-phenyl)-2-piperidino-benzyl]-aminocarbonylmethyl]-benzoesäure werden in 200 ml Äthanol suspendiert und bei 50°C und einem Wasserstoffdruck von 5 bar in Gegenwart von 0,4 g 10%iger Palladium/Kohle katalytisch entbenzyliert. Anschließend wird vom Katalysator abfiltriert, eingeengt und aus Acetonitril umkristallisiert.

Ausbeute: 720 mg (66,7% der Theorie),
Schmelzpunkt: 202—204°C

| | | | |
|---|---|---|---|
| Ber.: | C 72,95 | H 6,35 | N 6,30 |
| Gef.: | 72.65 | 6,17 | 6,20 |

Beispiel 5

4-[N-[α-(4-Methyl-phenyl)-2-piperidino-benzyl]-aminocarbonylmethyl]-benzylalkohol

2,5 g (5,3 mMol) 4-[N-[α-(4-Methyl-phenyl)-2-piperidino-benzyl]-aminocarbonylmethyl]-benzoesäure-äthylester werden portionsweise zu einer Suspension von 0,5 g (13,2 mMol) Lithiumaluminiumhydrid in 50 ml absolutem Tetrahydrofuran gegeben. Man rührt noch 30 Minuten bei Raumtemperatur, zersetzt durch Zutropfen von 4 N Natronlauge und filtriert vom gebildeten Natriumaluminat ab. Das Filtrat wird eingeengt und der Rückstand aus wenig Toluol umkristallisiert.

Ausbeute: 0.98 g (43% der Theorie),
Schmelzpunkt: 144—146°C

| | | | |
|---|---|---|---|
| Ber.: | C 78,47 | H 7,53 | N 6,54 |
| Gef.: | 78.20 | 7,39 | 6,58 |

Beispiel 6

4-[N-[α-(4-Methyl-phenyl)-2-piperidino-benzyl]-aminocarbonylmethyl]-benzaldehyd

8,85 g (20 mMol) 4-[N-[α-(4-Methyl-phenyl)-2-piperidino-benzyl]-aminocarbonylmethyl]-benzoesäure und 3,25 g (20 mMol) N,N'-Carbonyldiimidazol werden in 100 ml absolutem Tetrahydrofuran 2 Stunden am Rückfluß erhitzt. Dann wird eingeengt und nach Zugabe von 50 ml Pyridin und 3,7 g (20 mMol) 4-Toluolsulfonsäure-hydrazid nochmals 2 Stunden am Rückfluß gekocht. Anschließend gibt man auf Eiswasser, saugt ab und trocknet den Niederschlag. Das so erhaltene rohe Toluolsulfonsäurehydrazid der eingesetzten Carbonsäure wird mit 20 g wasserfreiem Natriumcarbonat versetzt und in 50 ml Äthylenglykol zwei Stunden auf 170°C erhitzt. Danach gibt man auf Wasser und extrahiert mit Chloroform. Die eingeengten Extrakte werden an Kieselgel mit Toluol/Essigsäure-äthylester 5:1 als Fließmittel säulenchromatographisch gereinigt.

Ausbeute: 1,73 g (21% der Theorie),
Schmelzpunkt: 144—146°C

| | | | |
|---|---|---|---|
| Ber.: | C 78,84 | H 7,09 | N 6,57 |
| Gef.: | 78.95 | 7,19 | 6,50 |

Analog Beispiel 6 wurde hergestellt:

(a) 4-[N-(α-Phenyl-2-piperidino-benzyl)-aminocarbonylmethyl]-benzaldehyd
Ausbeute: 29% der Theorie,
Schmelzpunkt: 168—170°C

| | | | |
|---|---|---|---|
| Ber.: | C 78,61 | H 6,84 | N 6,79 |
| Gef.: | 78.60 | 7,00 | 6,72 |

Beispiel 7

4-[N-[α-(4-Methyl-phenyl)-2-piperidino-benzyl]-aminocarbonylmethyl]-benzaldehyd

0,5 g (1,2 mMol) 4-[N-[α-(4-Methyl-phenyl)-2-piperidino-benzyl]-aminocarbonylmethyl]-benzylalkohol gibt man zu einer Suspension von 0,4 g (1,5 mMol) Pyridiniumchlorchromat in 2 ml Chloroform. Nach 12

Stunden bei Raumtemperatur wird mit Ather versetzt, filtriert und das eingeengte Filtrat an Kieselgel säulenchromatographisch gereinigt (Fließmittel: Toluol/Essigsäure-äthylester = 5:1).

Ausbeute: 0,3 g (60% der Theorie),
Schmelzpunkt: 145—146°C

| | | | |
|---|---|---|---|
| Ber.: | C 78,84 | H 7,09 | N 6,57 |
| Gef.: | 78.97 | 7,12 | 6,57 |

Analog Beispiel 7 wurde hergestellt:
(a) 4-[N-(α-Phenyl-2-piperidino-benzyl)-aminocarbonylmethyl]-benzaldehyd

Ausbeute: 40% der Theorie,
Schmelzpunkt: 170°C

| | | | |
|---|---|---|---|
| Ber.: | C 78,61 | H 6,84 | N 6,79 |
| Gef.: | 78.59 | 6,87 | 6,61 |

## Beispiel 8

4-[N-[α-(4-Methyl-phenyl)-2-piperidino-benzyl]-aminocarbonylmethyl]-zimtsäure-äthylester

427 mg (1 mMol) 4-[N-[α-(4-Methyl-phenyl)-2-piperidino-benzyl]-aminocarbonylmethyl]-benzaldehyd werden zu einer ätherischen Lösung von 450 mg (2 mMol) Diäthylphosphonoessigsäure-äthylester und 100 mg (2 mMol) 50 %igem Natriumhydrid gegeben. Nach Rühren über Nacht wird mit Wasser zersetzt und mit Chloroform extrahiert und an Kieselgel mit Toluol/Essigsäure-äthylester 5:1 als Fließmittel säulenchromotagraphisch gereinigt.

Ausbeute: 0,18 g (36% der Theorie),
Schmelzpunkt: 175—180°C

| | | | |
|---|---|---|---|
| Ber.: | C 77,39 | H 7,31 | N 5,64 |
| Gef.: | 77.64 | 7,25 | 5,71 |

Analog Beispiel 8 wurde hergestellt:
(a) 4-[N-(α-Phenyl-2-piperidino-benzyl)-aminocarbonylmethyl]-zimtsäure-äthylester

Ausbeute: 28,6% der Theorie,
Schmelzpunkt: 159—161°C

| | | | |
|---|---|---|---|
| Ber.: | C 77,14 | H 7,10 | N 5,80 |
| Gef.: | 77.28 | 7,21 | 5,65 |

## Beispiel 9

4-[N-[α-(4-Methyl-phenyl)-2-piperidino-benzyl]-aminocarbonylmethyl]-zimtsäure

Hergestellt durch alkalische Verseifung von 4-[N-[α-(4-Methyl-phenyl)-2-piperidino-benzyl]-aminocarbonylmethyl]-zimtsäure-äthylester analog Beispiel 3.

Ausbeute: 84% der Theorie,
Schmelzpunkt: 173—176°C

| | | | |
|---|---|---|---|
| Ber.: | C 76,90 | H 6,88 | N 5,98 |
| Gef.: | 77.24 | 7,01 | 5,64 |

Analog Beispiel 9 wurde hergestellt:
(a) 4-[N-(α-Phenyl-2-piperidino-benzyl)-aminocarbonylmethyl]-zimtsäure

Ausbeute: 75% der Theorie,
Schmelzpunkt: 177—180°C

| | | | |
|---|---|---|---|
| Ber.: | C 76,62 | H 6,65 | N 6,16 |
| Gef.: | 76.75 | 6,57 | 6,07 |

## Beispiel 10

4-[N-[α-(3-Methyl-phenyl)-2-piperidino-benzyl]-aminocarbonylmethyl]-benzoesäure-äthylester

Zu 1,5 ml o-Dichlorbenzol und 1,5 ml konzentrierter Schwefelsäure tropft man bei Raumtemperatur ein Gemisch aus 0,22 g (0,8 mMol) α-(3-Methyl-phenyl)-2-piperidino-benzylalkohol und 0,15 g (0,8 mMol) 4-Cyanomethyl-benzoesäureäthylester in 2 ml o-Dichlorbenzol. Nach 2 Stunden Rühren gibt man auf Eiswasser, extrahiert einmal mit Äther, stellt mit verdünnter Natronlauge alkalisch und extrahiert mit Chloroform. Der Chloroform-Extrakt wird eingeengt und der Rückstand aus Äthanol umkristallisiert.

Ausbeute: 0,22 g (60% der Theorie),
Schmelzpunkt: 158—159°C

| | | | |
|---|---|---|---|
| Ber.: | C 76,57 | H 7,28 | N 5,95 |
| Gef.: | 76.41 | 7,39 | 5,76 |

## Beispiel 11

4-[N-[α-(4-Methyl-phenyl)-2-piperidino-benzyl]-aminocarbonylmethyl]-benzoesäure

240 mg (5 mMol) 4-[N-(5-Chlor-α-(4-methyl-phenyl)-2-piperdino-benzyl]-aminocarbonylmethyl]-benzoesäure werden in 80 ml Äthanol/Dioxan 1/1 in Gegenwart von 0,1 g Palladium auf Kohle (10%ig) bei

50°C und einem Wasserstoffdruck von 5 bar katalytisch enthalogeniert. Nach dem Abkühlen wird vom Katalysator abfiltriert. Das Filtrat wird eingeengt und der Rückstand aus Äthanol umkristallisiert.

Ausbeute: 0,16 g (72% der Theorie),
Schmelzpunkt: 226—228°C

| | | | |
|---|---|---|---|
| Ber.: | C 75,99 | H 6,83 | N 6,33 |
| Gef.: | 75,81 | 6,73 | 6,10 |

Analog Beispiel 11 wurden hergestellt:

(a) 4-[N-[2-(2-Methyl-piperidino)-α-phenyl-benzyl]aminocarbonylmethyl)benzoesäure aus 4-[N-[5-Chlor-2-(2-methyl-piperidino)-α-phenylbenzyl]-aminocarbonylmethyl]-benzoesäure

Ausbeute: 68% der Theorie,
Schmelzpunkt: 246—248°C

| | | | |
|---|---|---|---|
| Ber.: | C 75,99 | H 6,83 | N 6,33 |
| Gef.: | 75.57 | 7,10 | 6,44 |

(b) 4-[N-[2-(3-Methyl-piperidino)-α-phenyl-benzyl]aminocarbonylmethyl)benzoesäure aus 4-[N-[5-Chlor-2-(3-methyl-piperidino)-α-phenyl-benzyl]-aminocarbonylmethyl]-benzoesäure

Ausbeute: 43% der Theorie,
Schmelzpunkt: 228—230°C

| | | | |
|---|---|---|---|
| Ber.: | C 75,99 | H 6,83 | N 6,33 |
| Gef.: | 75,91 | 6,82 | 6,33 |

## Beispiel 12

4-[N-[α-(4-Methyl-phenyl)-2-piperidino-benzyl]-aminocarbonylmethyl]-benzoesäure-äthylester

Eine Lösung von 2,78 g (10 mMol) frisch hergestelltem (4-Methyl-phenyl)-(2-piperdino-phenyl)-ketimin in 50 ml Methylenchlorid versetzt man mit 1,5 ml (11 mMol) Triäthylamin und anschließend unter Eiskühlung tropfenweise mit einer Lösung von 2,5 g (11 mMol) 4-Äthoxycarbonyl-phenylessigsäurechlorid in 20 ml Methylenchlorid. Nach 1 Stunde bei Raumtempertur gibt man auf Eiswasser und extrahiert mit Methylenchlorid. Die Extrakte werden getrocknet und eingeengt und der Einengungsrückstand an Kieselgel säulenchloròmatographisch geeinigt (Fließmittel: Toluol/Essigsäure-äthlester 10:1). Das rohe Acylimin wird in Dimethylformamid gelöst und nach Zugabe von 0,5 g Palladium (10%ig auf Kohle) bei 5 bar Wasserstoffdruck bei Raumtemperatur hydriert. Nach Aufnahme der berechneten Wasserstoffdruck bei Raumtemperatur hydriert. Nach Aufnahme der berechneten Wasserstoffmenge wird vom Katalysator abfiltriert, eingeengt und der Rückstand aus wenig Alkohol umkristallisiert.

Ausbeute: 2,8 (60% der Theorie),
Schmelzpunkt: 175—177°C

| | | | |
|---|---|---|---|
| Ber.: | C 76,57 | H 7,28 | N 5,95 |
| Gef.: | 76,41 | 7,19 | 5,76 |

## Beispiel 13

4-[N-[α-(4-Methyl-phenyl)-2-piperidino-benzyl]-aminocarbonylmethyl]-benzonitril

Hergestellt aus α-(4-Methyl-phenyl)-2-piperidino-benzylamin und 4-Cyano-phenylessigsäure analog Beispiel 1.

Ausbeute: 64% der Theorie,
Schmelzpunkt: 144—146°C

| | | | |
|---|---|---|---|
| Ber.: | C 79,40 | H 6,90 | N 9,92 |
| Gef.: | 79,10 | 6,90 | 9,78 |

## Beispiel 14

4-[N-[α-(4-Methyl-phenyl)-2-piperidino-benzyl]-aminocarbonylmethyl]-benzoesäure-äthylester

4,2 g (10 mMol) 4-[N-[α-(4-Methyl-phenyl)-2-piperidino-benzyl]-aminocarbonylmethyl]-benzonitril werden mit 50 ml äthanolischer Salzsäure 24 Stunden am Rückfluß gekocht. Anschließend engt man ein, versetzt den Einengungsrückstand mit wässriger Natriumbicarbonatlösung und extrahiert mit Chloroform. Der Chloroformextrakt wird eingeengt und der Rückstand mit Äthanol verrieben und abgesaugt.

Ausbeute: 2,9 g (61.6% der Theorie),
Schmelzpunkt: 177—179°C

| | | | |
|---|---|---|---|
| Ber.: | C 76,57 | H 7,28 | N 5,95 |
| Gef.: | 76,41 | 7,35 | 5,76 |

## Beispiel 15

4-[N-[5-Chlor-α-(2-chlor-phenyl)-2-piperidino-benzyl]-aminocarbonylmethyl]-benzoesäure-äthylester

10 mMol 4-[N-[α-(2-Chlor-phenyl)-5-nitro-2-piperidino-benzyl]-aminocarbonylmethyl]-benzoesäure-äthylester werden in 50 ml Dimethylformamid gelöst und nach Zugabe von 1 g Raney-Nickel bei 60°C und einem Wasserstoffdruck von 6 bar hydriert. Anschließend wird vom Katalysator abfiltriert, das Filtrat

eingeengt und der Rückstand, bestehend aus 4-[N-[5-Amino-α-(2-chlor-phenyl)-2-piperidino-benzyl]-amino-carbonylmethyl]-benzoesäure-äthylester in 100 ml konzentrierter Salzsäure gelöst. Unter Eiskühlung wird nun eine Lösung von 1,0 g (14 mMol) Natriumnitrit in 10 mlw14 mMol) Natriumnitrit in 10 ml Wasser zugetropft und 1 Stunde bei 0—5°C nachgerührt. Die Reaktionsmischung tropft man anschließend auf eine Lösung von 3 g Kupfer-I-chlorid in 25 ml konzentrierter Salzsäure. Nach einer Stunde Nachrühren wird mit Natronlauge alkalisch gestellt und mit Chloroform extrahiert. Die eingeengten Chloroformextrakte werden an Kieselgel im Fließmittel Toluol/Essigsäure-äthylester 5:1 säulenchromatographisch gereinigt.

Ausbeute: 1,5 g (28.6% der Theorie),
Schmelzpunkt: 213—215°C

| | | | | |
|---|---|---|---|---|
| Ber.: | C 66,28 | H 5,75 | N 5,33 | Cl 13,49 |
| Gef.: | 66,40 | 5,91 | 5,41 | 13,40 |

Beispiel 16

3-[4-[N-(α-(4-methyl-phenyl)-2-piperidino-benzyl]-aminocarbonylmethyl]-phenyl]-propionsäure

0,91 g (2 mMol) 4-[N-(α-(4-Methyl-phenyl)-2-piperidino-benzyl]-aminocarbonylmethyl]-zimtsäure werden in 50 ml Methanol gelöst und nach Zugabe von 0,5 g Palladium (10%ig auf Kohle) bei Raumtemperatur und einem Wasserstoffdruck von 3 bar katalytisch hydriert. Nach Beendigung der Wasserstoffaufnahme wird vom Katalysator abfiltriert und aus wenig Acetonitril umkristallisiert.

Ausbeute: 0,68 g (74% der Theorie),
Schmelzpunkt: 146—148°C

| | | | |
|---|---|---|---|
| Ber.: | C 76,57 | H 7,28 | N 5,85 |
| Gef.: | 76,41 | 7,19 | 5,61 |

Beispiel 17

4-[N-(α-(4-Methyl-phenyl)-2-piperidino-benzyl]-aminocarbonylmethyl]-benzoesäure-natriumsalz

442 mg (1 mMol) (4[N-(α(4-Methyl-phenyl)-2-piperidino-benzyl)-aminocarbonylmethyl]-benzoesäure werden in 25 ml Äthanol gelöst und mit 1 ml 1 N-Natronlauge versetzt. Anschließend engt man im Vakuum ein, versetzt mit 20 ml Aceton, saugt vom ausgefallenen Niederschlag ab und wäscht mit Essigsäure-äthylester nach.

Ausbeute: 410 mg (85% der Theorie),
Schmelzpunkt: 295—300°C

| | | | |
|---|---|---|---|
| Ber.: | C 72,40 | H 6,29 | N 6,03 |
| Gef.: | 72,15 | 6,46 | 5,93 |

Analog Beispiel 17 wurden hergestellt:

(a) 4-[N-(α-(4-Methyl-phenyl)-2-piperidino-benzyl]-aminocarbonylmethyl]-benzoesäure-äthanolaminsalz

Ausbeute: 75% der Theorie,
Schmelzpunkt: 188—191°C

| | | | |
|---|---|---|---|
| Ber.: | C 71,55 | H 7,41 | N 8,34 |
| Gef.: | 71,16 | 7,48 | 8,52 |

(b) 4-[N-(α-(4-Methyl-phenyl)-2-piperidino-benzyl]-aminocarbonylmethyl]-benzoesäure-diäthanolaminsalz

Ausbeute: 81% der Theorie,
Schmelzpunkt: 178—180°C

| | | | |
|---|---|---|---|
| Ber.: | C 70,70 | H 6,86 | N 7,73 |
| Gef.: | 70,25 | 6,75 | 7,58 |

(c) 4-[N-(α-(4-Methyl-phenyl)-2-piperidino-benzyl]-aminocarbonylmethyl]-benzoesäure-triäthanolaminsalz

Ausbeute: 76% der Theorie,
Schmelzpunkt: 160—165°C

| | | | |
|---|---|---|---|
| Ber.: | C 69,01 | H 7,67 | N 7,10 |
| Gef.: | 68,91 | 7,64 | 7,45 |

(d) 4-[N-(α-(4-Methyl-phenyl)-2-piperidino-benzyl]-aminocarbonylmethyl]-benzoesäure-äthylendiaminsalz

Ausbeute: 65% der Theorie,
Schmelzpunkt: 160—163°C

| | | | |
|---|---|---|---|
| Ber.: | C 71,69 | H 7,62 | N 11,15 |
| Gef.: | 72,04 | 7,80 | 10,96 |

Beispiel 18

4-[(2-Methoxy-1-(2-piperidino-phenyl)-äthyl)-aminocarbonylmethyl]-benzoesäure-äthylester

Zu einer Lösung von 0,55 g (2,34 mMol) 2-Methoxy-1-(2-piperidino-phenyl)-äthylamin in 5 ml Acetonitril gibt man nacheinander 0,49 g (2,34 mMol) 4-Äthoxycarbonyl-phenyl-essigsäure, 0,73 g (2,78 mMol) Triphenylphosphin, 0,50 ml (3,66 mMol) Triäthylamin und 0,23 ml (2,34 mMol) Tetrachlor kohlenstoff und

rührt 20 Stunden Raumtemperatur Anschließend dampf man im Vakuum ein und verteilt zwischen Äthylacetat und Wasser. Den organischen Extrakt trocknet und filtriert man und dampft ihn im Vakuum ein. Den Eindampfrückstand reinigt man durch Säulenchromatographie an Kieselgel (Toluol/Aceton = 10/2).

Ausbeute: 0,45 g (45% der Theorie),
Schmelzpunkt: 122—123°C

| | | | |
|---|---|---|---|
| Ber.: | C 70,73 | H 7,60 | N 6,60 |
| Gef.: | 71,04 | 7,48 | 6,39 |

Analog Beispiel 18 wurden folgende Verbindungen erhalten:

(a) 4-[(1-(3-Chlor-2-piperidino-phenyl)-1-butyl)-aminocarbonylmethyl]-benzoesäure-äthylester
Ausbeute: 55% der Theorie,
Schmelzpunkt: 141—143°C

| | | | | |
|---|---|---|---|---|
| Ber.: | C 68,33 | H 7,28 | Cl 7,76 | N 6,13 |
| Gef.: | 68,30 | 7,16 | 8,03 | 6,20 |

(b) 4-[(1-(6-Chlor-2-piperidino-phenyl)-1-butyl)-aminocarbonylmethyl]-benzoesäure-äthylester
Ausbeute: 73,9% der Theorie,
Schmelzpunkt: 79—82°C

| | | | | |
|---|---|---|---|---|
| Ber.: | C 68,33 | H 7,28 | Cl 7,76 | N 6,13 |
| Gef.: | 68,45 | 7,24 | 7,80 | 6,09 |

(c) 4-[(1-(4-Brom-2-piperidino-phenyl)-1-butyl)-aminocarbonylmethyl]-benzoesäure-äthylester
Ausbeute: 62,1% der Theorie,
Schmelzpunkt: 116—118°C

| | | | | |
|---|---|---|---|---|
| Ber.: | C 62,27 | H 6,63 | Br 15,93 | N 5,58 |
| Gef.: | 62,53 | 6,48 | 15,98 | 5,66 |

(d) 4-[(1-(4-Nitro-2-piperidino-phenyl)-1-butyl)-aminocarbonylmethyl]-benzoesäure-äthylester
Ausbeute: 74,6% der Theorie,
Schmelzpunkt: 127—130°C

| | | | |
|---|---|---|---|
| Ber.: | C 66,79 | H 7,11 | N 8,99 |
| Gef.: | 66,88 | 7,08 | 9,15 |

(e) 4-[(1-(3-Methyl-2-piperidino-phenyl)-1-butyl)-aminocarbonylmethyl]-benzoesäure-äthylester
Ausbeute: 68% der Theorie,
Schmelzpunkt: 145—147°C

| | | | |
|---|---|---|---|
| Ber.: | C 74,28 | H 8,31 | N 6,42 |
| Gef.: | 74,40 | 8,30 | 6,41 |

(f) 4-[(1-(4-Methyl-2-piperidino-phenyl)-1-butyl)-aminocarbonylmethyl]-benzoesäure-äthylester
Ausbeute: 54,7% der Theorie,
Schmelzpunkt: 113—114°C

| | | | |
|---|---|---|---|
| Ber.: | C 74,28 | H 8,31 | N 6,42 |
| Gef.: | 74,23 | 8,30 | 6,55 |

(i) 4-[(1-(2-Pyrrolidino-phenyl)-1-butyl)-aminocarbonylmethyl]-benzoesäure-äthylester
Ausbeute: 57,3% der Theorie,
Schmelzpunkt: 122—125°C

| | | | |
|---|---|---|---|
| Ber.: | C 73,50 | H 7,90 | N 6,86 |
| Gef.: | 73,63 | 8,07 | 7,01 |

(g) 4-[(1-(5-Methyl-2-piperidino-phenyl)-1-butyl)-aminocarbonylmethyl]-benzoesäure-äthylester
Ausbeute: 67,9% der Theorie,
Schmelzpunkt: 149—150°C

| | | | |
|---|---|---|---|
| Ber.: | C 74,28 | H 8,31 | N 6,42 |
| Gef.: | 74,38 | 8,21 | 6,49 |

(h) 4-[(1-(6-Methyl-2-piperidino-phenyl)-1-butyl)-aminocarbonylmethyl]-benzoesäure-äthylester
Ausbeute: 47% der Theorie,
Schmelzpunkt: 92—93°C

| | | | |
|---|---|---|---|
| Ber: | C 74,28 | H 8,31 | N 6,42 |
| Gef.: | 74,50 | 8,46 | 6,48 |

(k) 4-[(1-(2-Piperidino-phenyl)-1-butyl)-aminocarbonylmethyl]-benzoesäure-äthylester
Ausbeute: 71,5% der Theorie,
Schmelzpunkt: 127—128°C

| | | | |
|---|---|---|---|
| Ber.: | C 73,90 | H 8,11 | N 6,63 |
| Gef.: | 73,90 | 8,06 | 6,72 |

(l) 4-[(1-(2-(4-Methyl-piperidino)-phenyl)-1-butyl)-aminocarbonylmethyl]-benzoesäure-äthylester
Ausbeute: 51,1% der Theorie,
Schmelzpunkt: 153—155°C

| | | | |
|---|---|---|---|
| Ber.: | C 74,28 | H 8,31 | N 6,42 |
| Gef.: | 74,55 | 8,33 | 6,45 |

(m) 4-[(1-(2-Hexahydroazepino-phenyl(-1-butyl)-aminocarbonylmethyl]-benzoesäure-äthylester
Ausbeute: 42,7% der Theorie,
Schmelzpunkt: 145—147°C

| | | | |
|---|---|---|---|
| Ber.: | C 74,28 | H 8,31 | N 6,42 |
| Gef.: | 73,98 | 8,26 | 6,58 |

(n) 4-[(1-(5-Fluor-2-piperidino-phenyl)-1-butyl)-aminocarbonylmethyl]-benzoesäure-äthylester
Ausbeute: 55% der Theorie,
Schmelzpunkt: 128—130°C

| | | | |
|---|---|---|---|
| Ber.: | C 70,88 | H 7,55 | N 6,36 |
| Gef.: | 71,14 | 7,57 | 6,49 |

(o) 4-[(1-(2-Piperidino-phenyl)-1-butyl)-aminocarbonylmethyl]-benzoesäure-methylester
Ausbeute: 63,2% der Theorie,
Schmelzpunkt: 147—148°C

| | | | |
|---|---|---|---|
| Ber.: | C 73,50 | H 7,90 | N 6,86 |
| Gef.: | 73,66 | 7,88 | 6,80 |

(p) 4-[(1-(2-Piperidino-phenyl)-1-butyl)-aminocarbonylmethyl]-benzoesäure-n-butylester
Ausbeute: 50,9% der Theorie,
Schmelzpunkt: 117—119°C (Äther)

| | | | |
|---|---|---|---|
| Ber.: | C 74,63 | H 8,50 | N 6,22 |
| Gef.: | 74,49 | 8,46 | 6,14 |

(q) 4-[(1-(2-Piperidino-phenyl)-4-penten-1-yl)-aminocarbonylmethyl]-benzoesäure-äthylester
Ausbeute: 18,9% der Theorie,
Schmelzpunkt: 103—105°C

| | | | |
|---|---|---|---|
| Ber.: | C 74,62 | H 7,89 | N 6,45 |
| Gef.: | 75,01 | 8,10 | 6,26 |

## Beispiel 19

4-[(1-(5-Nitro-2-piperidino-phenyl)-1-butyl)-aminocarbonylmethyl]-benzoesäure-äthylester

Zu einer gerührten Lösung von 15,1 g (54,4 mMol) 1-(5-Nitro-2-piperdino-phenyl)-1-butylamin und 8,46 ml (61,4 mMol) Triäthylamin in 55 ml trockenem Methylenchlorid tropft man die Lösung von 14,6 g (64,6 mMol) 4-Äthoxycarbonyl-phenyl-essigsäurechlorid in 20 ml Methylenchlorid innerhalb 30 Minuten so zu, daß die Temperatur von 30°C nicht überschritten wird. Man rührt noch 2 Stunden bei Raumtemperatur, gibt 300 ml Methylenchlorid zu und schüttelt zweimal mit je 50 ml Wasser aus. Die organische Phase trocknet man über Natriumsulfat, filtriert und dampft sie im Vakuum ein. Den rotbraunen, öligen Eindampfrückstand reinigt man durch Säulenenchromatographie an Kieselgel (Toluol/Aceton = 10/1).

Ausbeute: 17,7% (69,7% der Theorie),
Schmelzpunkt: 135—137°C (Äther)

| | | | |
|---|---|---|---|
| Ber.: | C 66,79 | H 7,11 | N 8,99 |
| Gef.: | 66,73 | 6,99 | 9,09 |

Analog Beispiel 19 wurden folgende Verbindungen erhalten:

(a) 4-[(1-(2-Piperidino-phenyl)-1-butyl)-aminocarbonylmethyl]-benzoesäure-äthylester
Ausbeute: 80,2% der Theorie,
Schmelzpunkt: 127—129°C

| | | | |
|---|---|---|---|
| Ber.: | C 73,90 | H 8,11 | N 6,63 |
| Gef.: | 73,98 | 8,26 | 6,89 |

(b) 4-[(1-(4-Hydroxy-piperidino-phenyl)-1-butyl)-aminocarbonylmethyl]-benzoesäure-äthylester
Ausbeute: 13,5% der Theorie,
Schmelzpunkt: 178—180°C

| | | | |
|---|---|---|---|
| Ber.: | C 71,21 | H 7,81 | N 6,39 |
| Gef.: | 71,27 | 7,82 | 6,40 |

(c) 4-[(1-(5-Hydroxy-piperidino-phenyl)-1-butyl)-aminocarbonylmethyl]-benzoesäure-äthylester
Ausbeute: 37,4% der Theorie,
Schmelzpunkt: 188—190

| | | | |
|---|---|---|---|
| Ber.: | C 71,21 | H 7,81 | N 6,39 |
| Gef.: | 71,34 | 7,89 | 6,38 |

## Beispiel 20

4-[(1-(2-Piperidino-phenyl)-1-butyl)-aminocarbonylmethyl]-phenylessigsäure

Man erhitzt 3,0 g (15,45 mMol) p-Phenylen-diessigsäure und 10 ml Thionylchlorid 90 Minuten unter Rückfluß und dampft dann im Vakuum ein. Das rohe Disäurechlorid löst man in 100 ml Methylenchlorid. Zu dieser Lösung tropft man unter Rühren die Lösung von 3,6 g (15,45 mMol) 1-(2-Piperidinophenyl)-1-butylamin bei einer Innentemperatur von 10—15°C langsam zu. Nach 2 Stunden bei Raumtemperatur dampft man im Vakuum ein und verteilt den Eindampfrückstand zwischen 100 ml eiskalter 5 %iger Natronlauge und Äthylacetat. Man filtriert durch Kieselgur und trennt die organische Phase ab. Die alkalisch-wäßridge Phase stellt man mit halbkonzentrierter Salzsäure auf pH 5,5 ein und extrahiert mit Äthylacetat. Man trocknet über Natriumsulfat, filtriert und dampft das Filtrat im Vakuum ein. Den Eindampfrückstand reinigt man durch Säulenchromatographie an Kieselgel (Chloroform/Methanol = 20/1).

Ausbeute: 0,10 g (1,6% der Theorie),
Schmelzpunkt: 136—140 (Acetonitril/Äther)

| | | | |
|---|---|---|---|
| Ber.: | C 73,50 | H 7,90 | N 6,86 |
| Gef.: | 73,17 | 8,10 | 6,85 |

## Beispiel 21

4-[(2-Methyl-(2-piperidino-phenyl)-1-propen-1-yl)-aminocarbonylmethyl]-benzoesäure-äthylester

Zu einer Lösung von 6,17 g (26,8 mMol) frisch hergestelltem Isopropyl-(2-piperdino)-ketimin in 62 ml Acetonitril gibt man nacheinander 5,58 g (26,8 mMol) 4-Äthoxycarbonylphenylessigsäre, 8,43 g (32,2 mMol) Triphenylphosphin, 11,2 ml (80,4 mMol) Triäthylamin und 2,6 ml (0,0268 Mol) Tetrachlorkohlenstoff und rührt 20 Stunden bei Raumtemperatur. Anschließend dampf man im Vakuum ein und verteilt zwischen Äthylacetat und Wasser. Den getrockneten und filtrierten Äthylacetat-Extrakt dampf man im Vakuum ein. Den Eindampfrückstand reinigt man durch Säulenchromatographie an Kieselgel (Toluol/Äthylacetat = 5/1).

Ausbeute: 3,0 g (26,6% der Theorie),
Schmelzpunkt: 82—84 (Äther)

| | | | |
|---|---|---|---|
| Ber.: | C 74,26 | H 7,67 | N 6,66 |
| Gef.: | 74,20 | 7,49 | 6,56 |

Analog Beispiel 21 wurden folgende Verbindungen erhalten:

(a) 4-[(1-(2-Piperidino-phenyl)-1-penten-1-yl)-aminocarbonylmethyl]-benzoesäure-äthylester
Ausbeute: 16% der Theorie,
Schmelzpunkt: 94—97° (Äthanol)

| | | | |
|---|---|---|---|
| Ber.: | C 74,62 | H 7,89 | N 6,45 |
| Gef.: | 74,75 | 7,71 | 6,24 |

(b) 4-[(1-(2-Piperidino-phenyl)-1-hexen-1-yl)-aminocarbonylmethyl]-benzoesäure-äthylester
Ausbeute: 27,4% der Theorie,
Schmelzpunkt: 83—85° (Äthanol)

| | | | |
|---|---|---|---|
| Ber.: | C 74,97 | H 8,09 | N 6,24 |
| Gef.: | 74,42 | 7,95 | 6,00 |

(c) 4-[(1-(2-Piperidino-phenyl)-1-buten-1-yl)-aminocarbonylmethyl]-benzoesäure-äthylester
Ausbeute: (lipophileres Isomere; wahrscheinlich E-Form): 4,1% der Theorie,
Schmelzpunkt: < 20°C
Ber.: m/e = 420
Gef.: m/e = 420
Ausbeute: (weniger lipophiles Isomere; wahrescheinlich Z-Form): 51,9% der Theorie,
Schmelzpunkt: 115—117°C (Äthanol)

| | | | |
|---|---|---|---|
| Ber.: | C 74,26 | H 7,67 | N 6,66 |
| Gef.: | 73,85 | 7,59 | 6,44 |

(d) 4-[2-Phenyl-1-(2-piperidino-phenyl)-äthen-1-yl)-aminocarbonylmethyl]-benzoesäure-äthylester
Ausbeute (lipophileres Isomere; wahrscheinlich E-Form): 4% der Theorie,
Schmelzpunkt: 75—77°C (Äther/Petroläther)

| | | | |
|---|---|---|---|
| Ber.: | C 76,90 | H 6,88 | N 5,98 |
| Gef.: | 77,31 | 7,20 | 5,93 |

Ausbeute (weniger lipophiles Isomere; wahrscheinlich Z-Form): 42,7% der Theorie,
Schmelzpunkt: 157—160°C (Äthanol)

| | | | |
|---|---|---|---|
| Gef.: | 77,19 | 6,95 | 6,02 |

(e) 4-[(3-Phenyl-1-(2-piperidino-phenyl)-1-propen-1-yl)-aminocarbonylmethyl]-benzoesäure-äthylester
Ausbeute: 62,6% der Theorie,
Schmelzpunkt: < 20°
Ber.: m/e = 482
Gef.: m/e = 482

(f) 4-[(1-(2-(3,3-Dimethyl-piperidino)-phenyl)-1-buten-1-yl)-aminocarbonylmethyl]-benzoesäure-äthylester
Ausbeute: 33% der Theorie,
Schmelzpunkt: 113—116°C (Äthanol)

| | | | |
|---|---|---|---|
| Ber.: | C 74,97 | H 8,09 | N 6,24 |
| Gef.: | 75,37 | 7,93 | 6,03 |

(g) 4-[(1-(6-Methyl-piperidino-phenyl)-1-buten-1-yl)-aminocarbonylmethyl]-benzoesäure-äthylester
Ausbeute: 60,4% der Theorie (wahrscheinlich Z-Form),
Schmelzpunkt: 95—96°C

| | | | | |
|---|---|---|---|---|
| Ber.: | C 74,62 | H 7,89 | N 6,45 | m/e = 434 |
| Gef.: | 74,44 | 8,00 | 6,59 | m/e = 434 |

### Beispiel 22

4-[(1-(2-Piperidino-phenyl)-1-buten-1-yl)-aminocarbonylmethyl]-benzoesäure-äthylester

Man erhitzt eine gerührte Lösung von 19,0 g (82,46 mMol) frisch hergestelltem (2-Piperidino-phenyl)-propyl-ketimin und 11,5 ml (82,46 mMol) Triäthylamin in 190 ml wasserfreiem Toluol auf eine Innentemperatur von 85°C, tropft dann innerhalb 10 Minuten die Lösung von 18,7 g (82,46 mMol) 4-Äthoxycarbonyl-phenylessigsäurechlorid in 95 ml wasserfreiem Toluol zu und rührt 30 Minuten bei einer Innentemperatur von 95°C. Man kühlt auf 20°C ab und schüttelt zweimal mit Wasser aus. Die organische Phase trocknet man über Natriumsulfat, filtriert und dampft sie im Vakuum ein. Den Eindampfrückstand reinigt man durch mehrfache Saülenchromatographie (Toluol/Aceton = 20/1 und 50/1).

Ausbeute (lipophileres Isomere; wahrscheinlich E-Form): 11,2 g (23,6% der Theorie),
Schmelzpunkt: < 20°C (honiggelbes, zähes Öl)

| | | | |
|---|---|---|---|
| Ber.: | C 74,26 | H 7,67 | N 6,66 |
| Gef.: | 73,90 | 7,92 | 6,91 |

Ausbeute (weniger lipophiles Isomere; wahrscheinlich Z-Form): 15,9 g (33,5% der Theorie),
Schmelzpunkt: 114—116°C

| | | | |
|---|---|---|---|
| Gef.: | C 74,02 | H 7,69 | N 6,85 |

### Beispiel 23

(E)- und (Z)-4-[(1-(2-Piperidino-phenyl)-1-buten-1-yl)-aminocarbonylmethyl]-benzoesäure-äthylester

Man erhitzt 1,0 g Z-Ester (siehe Beispiel 21c) in einem vorgeheizten Ölbad von 230°C 30 Minuten lang. Nach Abkühlen wird das erhaltene Produkt durch Säulenchromatographie an Kieselgel (Toluol/Aceton = 20/1) gereinigt.

Ausbeute (E-Ester): 0,365 g (36,5% der Theorie),
Schmelzpunkt: < 20°C
Ausbeute (Z-Ester): 0,380 g (38,0% der Theorie),
Schmelzpunkt: 115—117°C

Bei 3,5-stündigem Erhitzen des (E)-Esters mit katalytischen Mengen Jod in Benzol erhält man laut dünnschichtchromatographischer Untersuchung (Toluol/Aceton = 10/1) ein 1/1 Gemisch von (E)- und (Z)-Ester.

Analog Beispiel 23 wurden folgende Verbindungen erhalten:

(a) (E)- und (Z)-4-[(1-(6-Methyl-2-piperdino-phenyl)-1-buten-1-yl)-aminocarbonylmethyl]-benzoesäure-äthylester

Aus (Z)-Ester (siehe Beispiel 21g) erhält man laut Dünnschichtchromatogramm ein 1/1-Gemisch von (E)- und (Z)-Ester.

| | |
|---|---|
| oberer Fleck (E): | Ber.: m/e = 434 |
| | Gef.: m/e = 434 |
| unterer Fleck (Z): | Gef.: m/e = 434 |

Beispiel 24

4-[(1-(2-Piperidino-phenyl)-1-butyl)-aminocarbonylmethyl]-benzoesäure-äthylester

Man hydriert 2,9 g (6,90 mMol) 4-[(1-(2-Piperidino-phenyl)-1-buten-1-yl)-aminocarbonylmethyl]-benzoesäure-äthylester in 100 ml Äthanol an 0,77 g Palladium/Kohle (10%ig) bei 50°C und 1 bar Wasserstoff. Nach 2 Stunden filtriert man vom Katalysator über Kieselgur ab und dampft in Vakuum ein. Den Eindampfrückstand kristallisiert man aus Äthanol.

Ausbeute: 1,5 g (51,5% der Theorie),
Schmelzpunkt: 126—128°C

| | | | |
|---|---|---|---|
| Ber.: | C 73,90 | H 8,11 | N 6,63 |
| Gef.: | 73,97 | 8,22 | 6,57 |

Analog Beispiel 24 wurden folgende Verbindungen ergalten:

(a) 4-[(1-(2-Piperidino-phenyl)-1-pentyl)-aminocarbonylmethyl]-benzoesäure-äthylester
Ausbeute: 45% der Theorie,
Schmelzpunkt: 117—120°C (Äther)

| | | | |
|---|---|---|---|
| Ber.: | C 74,28 | H 8,31 | N 6,42 |
| Gef.: | 74,60 | 8,13 | 6,27 |

(b) 4-[(1-(2-Piperidino-phenyl)-1-hexyl)-aminocarbonylmethyl]-benzoesäure-äthylester
Ausbeute: 50% der Theorie,
Schmelzpunkt: 108—110°C (Äther)

| | | | |
|---|---|---|---|
| Ber.: | C 74,63 | H 8,50 | N 6,22 |
| Gef.: | 74,85 | 8,33 | 6,01 |

(c) 4-[(2-Phenyl-1-(2-piperidino-phenyl)-1-äthyl)-aminocarbonylmethyl]-benzoesäure-äthylester
Ausbeute: 87,6% der Theorie,
Schmelzpunkt: 161—162°C (Äthanol)

| | | | |
|---|---|---|---|
| Ber.: | C 76,57 | H 7,28 | N 5,95 |
| Gef.: | 76,71 | 7,19 | 5,99 |

(d) 4-[(3-Phenyl-1-(2-piperidino-phenyl)-1-propyl)-aminocarbonylmethyl]-benzoesäure-äthylester
Ausbeute: 57,6% der Theorie,
Schmelzpunkt: 118—119°C (Äthanol)

| | | | |
|---|---|---|---|
| Ber.: | C 76,83 | H 7,49 | N 5,78 |
| Gef.: | 76,70 | 7,49 | 5,90 |

(e) 4-[(1-(2-(3,3-Dimethyl-piperidino)-phenyl)-1-butyl)-aminocarbonylmethyl]-benzoesäure-äthylester
Ausbeute: 36,5% der Theorie,
Schmelzpunkt: 140—141°C (Äthanol)

| | | | |
|---|---|---|---|
| Ber.: | C 74,63 | H 8,50 | N 6,22 |
| Gef.: | 74,30 | 8,23 | 6,12 |

Beispiel 25

4-[(1-(2-Piperidino-phenyl)-1-butyl)-aminocarbonylmethyl]-benzoesäure

Man rührt das Gemisch aus 1,2 g (2,84 mMol) 4-[(1-(2-Piperidino-phenyl)-1-butyl)-aminocarbonylmethyl]-benzoesäure-äthylester und 4,26 ml 1 N-Natronlauge in 12 ml Äthanol 1 Stunde bei 60°C, neutralisiert dann mit 4,26 ml 1N-Salzsäure und dampft im Vakuum das Äthanol ab. Man verteilt zwischen Äthylacetat und Wasser; den organischen Extrakt trocknet und filtriert man und dampft ihn im Vakuum ein. Den Eindampfrückstand kristallisiert man aus Äthanol.

Ausbeute: 0,50 g (44,6% der Theorie),
Schmelzpunkt: 213—215°C

| | | | |
|---|---|---|---|
| Ber.: | C 73,07 | H 7,66 | N 7,10 |
| Gef.: | 73,18 | 7,51 | 7,10 |

Analog Beispiel 25 wurden folgende Verbindungen erhalten:

(a) 4-[(1-(2-Piperidino-phenyl)-1-pentyl)-aminocarbonylmethyl]-benzoesäure
Ausbeute: 70,2% der Theorie,
Schmelzpunkt: 213—215°C (Aceton)

| | | | |
|---|---|---|---|
| Ber.: | C 73,50 | H 7,90 | N 6,86 |
| Gef.: | 73,71 | 7,70 | 6,90 |

(b) 4-[(1-(2-Piperidino-phenyl)-1-hexyl)-aminocarbonylmethyl]-benzoesäure
Ausbeute: 72,2% der Theorie,
Schmelzpunkt: 197—200°C (Aceton)

| | | | |
|---|---|---|---|
| Ber.: | C 73,90 | H 8,11 | N 6,63 |
| Gef.: | 73,83 | 7,93 | 6,77 |

(c) 4-[(2-Phenyl-1-(2-piperidino-phenyl)-1-äthyl)-aminocarbonylmethyl]-benzoesäure
Ausbeute: 68,7% der Theorie,
Schmelzpunkt: 214—215°C (Äthanol)

| | | | |
|---|---|---|---|
| Ber.: | C 75,99 | H 6,83 | N 6,33 |
| Gef.: | 75,70 | 6,60 | 6,32 |

(d) 4-[(3-Phenyl-1-(2-piperidino-phenyl)-1-propyl)-aminocarbonylmethyl]-benzoesäure
Ausbeute: 67,7% der Theorie,
Schmelzpunkt: 167—170°C (Äthylacetat)

| | | | |
|---|---|---|---|
| Ber.: | C 76,29 | H 7,06 | N 6,14 |
| Gef.: | 76,56 | 7,06 | 6,23 |

(e) 4-[2-Methoxy-1-(2-piperidino-phenyl)-1-äthyl)-aminocarbonylmethyl]-benzoesäure
Ausbeute: 60,8% der Theorie,
Schmelzpunkt: 196—198°C (Äther)

| | | | |
|---|---|---|---|
| Ber.: | C 69,68 | H 7,12 | N 7,07 |
| Gef.: | 69,72 | 6,52 | 6,71 |

(f) 4-[(1-(2-Piperidino-phenyl)-4-penten-1-yl)-aminocarbonylmethyl]-benzoesäure x 0,67 $H_2O$
Ausbeute: 30,7% der Theorie,
Schmelzpunkt: 193—197°C (Äther/Petroläther)

| | | | |
|---|---|---|---|
| Ber.: | C 71,74 | H 7,38 | N 6,69 |
| Gef.: | 71,63 | 7,21 | 6,34 |

(g) 4-[(1-(2-(3,3-Dimethyl-piperidino)-phenyl)-1-butyl)-aminocarbonylmethyl]-benzoesäure
Ausbeute: 48,2% der Theorie,
Schmelzpunkt: 168—170°C (Petroläther)

| | | | |
|---|---|---|---|
| Ber.: | C 73,91 | H 8,11 | N 6,63 |
| Gef.: | 73,51 | 7,89 | 6,32 |

(h) 4-[(1-(3-Methyl-2-piperidino-phenyl)-1-butyl)-aminocarbonylmethyl]-benzoesäure
Ausbeute: 53% der Theorie,
Schmelzpunkt: 179—182°C

| | | | |
|---|---|---|---|
| Ber.: | C 73,50 | H 7,90 | N 6,86 |
| Gef.: | 73,50 | 7,82 | 7,01 |

(i) 4-[(1-(4-Methyl-2-piperidino-phenyl)-1-butyl)-aminocarbonylmethyl]-benzoesäure
Ausbeute: 85,6% der Theorie,
Schmelzpunkt: 170—172°C

| | | | |
|---|---|---|---|
| Ber.: | C 73,50 | H 7,90 | N 6,86 |
| Gef.: | 73,25 | 7,64 | 6,89 |

(k) 4-[(1-(5-Methyl-2-piperidino-phenyl)-1-butyl)-aminocarbonylmethyl]-benzoesäure
Ausbeute: 62,1% der Theorie,
Schmelzpunkt: 219—221°C

| | | | |
|---|---|---|---|
| Ber.: | C 73,50 | H 7,90 | N 6,86 |
| Gef.: | 73,20 | 7,74 | 6,89 |

(l) 4-[(1-(6-Methyl-2-piperidino-phenyl)-1-butyl)-aminocarbonylmethyl]-benzoesäure x 0,3 $H_2O$
Ausbeute: 89% der Theorie,
Schmelzpunkt: 158—160°C

| | | | |
|---|---|---|---|
| Ber.: | C 72,53 | H 7,93 | N 6,77 |
| Gef.: | 72,40 | 7,91 | 6,92 |

(m) 4-[(1-(3-Chlor-2-piperidino-phenyl)-1-butyl)-aminocarbonylmethyl]-benzoesäure
Ausbeute: 70% der Theorie,
Schmelzpunkt: 189—191°C

| | | | | |
|---|---|---|---|---|
| Ber.: | C 67,20 | H 6,81 | Cl 8,27 | N 6,53 |
| Gef.: | 67,30 | 6,85 | 8,36 | 6,58 |

(n) 4-[(1-(4-Chlor-2-piperidino-phenyl)-1-butyl)-aminocarbonylmethyl]-benzoesäure
Ausbeute: 57,8% der Theorie,
Schmelzpunkt: 188—189°C

| | | | | |
|---|---|---|---|---|
| Ber.: | C 67,20 | H 6,81 | Cl 8,27 | N 6,53 |
| Gef.: | 66,90 | 7,00 | 8,22 | 6,53 |

(o) 4-[(1-(5-Chlor-2-piperidino-phenyl)-1-butyl)-aminocarbonylmethyl]-benzoesäure
Ausbeute: 81,6% der Theorie,
Schmelzpunkt: 226—229°C

| | | | | |
|---|---|---|---|---|
| Ber.: | C 67,20 | H 6,81 | Cl 8,27 | N 6,53 |
| Gef.: | 67,17 | 6,59 | 8,51 | 6,60 |

(p) 4-[(1-(6-Chlor-2-piperidino-phenyl)-1-butyl)-aminocarbonylmethyl]-benzoesäure
Ausbeute: 69,4% der Theorie,
Schmelzpunkt: 150—153°C

| | | | | |
|---|---|---|---|---|
| Ber.: | C 67,20 | H 6,81 | Cl 8,27 | N 6,53 |
| Gef.: | 67,18 | 6,91 | 8,42 | 6,77 |

(q) 4-[(1-(4-Brom-2-piperidino-phenyl)-1-butyl)-aminocarbonylmethyl]-benzoesäure
Ausbeute: 84,4% der Theorie,
Schmelzpunkt: 198—201°C

| | | | | |
|---|---|---|---|---|
| Ber.: | C 60,89 | H 6,17 | Br 16,88 | N 5,92 |
| Gef.: | 60,88 | 5,98 | 17,20 | 5,98 |

(r) 4-[(1-(5-Brom-2-piperidino-phenyl)-1-butyl)-aminocarbonylmethyl]-benzoesäure
Ausbeute: 90,7% der Theorie,
Schmelzpunkt: 232—235°C

| | | | | |
|---|---|---|---|---|
| Ber.: | C 60,89 | H 6,17 | Br 16,88 | N 5,92 |
| Gef.: | 60,96 | 6,13 | 16,85 | 5,90 |

(s) 4-[(1-(4-Nitro-2-piperidino-phenyl)-1-butyl)-aminocarbonylmethyl]-benzoesäure
Ausbeute: 70,9% der Theorie,
Schmelzpunkt: 188—190°C

| | | | |
|---|---|---|---|
| Ber.: | C 65,59 | H 6,65 | N 9,56 |
| Gef.: | 65,30 | 6,44 | 5,53 |

(t) 4-[(1-(5-Nitro-2-piperidino-phenyl)-1-butyl)-aminocarbonylmethyl]-benzoesäure
Ausbeute: 90,7% der Theorie,
Schmelzpunkt: 225—227°C

| | | | |
|---|---|---|---|
| Ber.: | C 65,59 | H 6,65 | N 9,56 |
| Gef.: | 65,80 | 6,61 | 9,72 |

(u) 4-[(1-(4-Hydroxy-2-piperidino-phenyl)-1-butyl)-aminocarbonylmethyl]-benzoesäure x 0,5 $H_2O$
Ausbeute: 85,7% der Theorie,
Schmelzpunkt: Erweichung ab 70°C (Schaum)

| | | | |
|---|---|---|---|
| Ber.: (x 0,5 $H_2O$) | C 68,71 | H 7,45 | N 6,68 |
| Gef.: | 68,63 | 7,55 | 6,26 |

(v) 4-[(1-(5-Hydroxy-2-piperidino-phenyl)-1-butyl)-aminocarbonylmethyl]-benzoesäure
Ausbeute: 89,3% der Theorie,
Schmelzpunkt: 186—190°C

| | | | |
|---|---|---|---|
| Ber.: | C 70,22 | H 7,37 | N 6,82 |
| Gef.: | 70,31 | 7,58 | 6,51 |

(w) 4-[(1-(4-Methoxy-2-piperidino-phenyl)-1-butyl)-aminocarbonylmethyl]-benzoesäure
Ausbeute: 78,6% der Theorie,
Schmelzpunkt: 185—187°C

| | | | |
|---|---|---|---|
| Ber.: | C 70,73 | H 7,60 | N 6,60 |
| Gef.: | 70,46 | 7,77 | 6,56 |

(x) 4-[(1-(5-Methoxy-2-piperidino-phenyl)-1-butyl)-aminocarbonylmethyl]-benzoesäure
Ausbeute: 75% der Theorie,
Schmelzpunkt: 182—185°C (Zers.)

| | | | |
|---|---|---|---|
| Ber.: | C 70,73 | H 7,60 | N 6,60 |
| Gef.: | 70,52 | 7,50 | 6,70 |

(y) 4-[(1-(2-Pyrrolidino-phenyl)-1-butyl)-aminocarbonylmethyl]-benzoesäure
Ausbeute: 64,5% der Theorie,
Schmelzpunkt: 200—203°C

| | | | |
|---|---|---|---|
| Ber.: | C 72,61 | H 7,42 | N 7,36 |
| Gef.: | 72,64 | 7,50 | 7,38 |

(z) 4-[(1-(2-(4-Methyl-piperidino)-phenyl)-1-butyl)-aminocarbonylmethyl]-benzoesäure
Ausbeute: 81,4% der Theorie,
Schmelzpunkt: 197—201°C

| | | | |
|---|---|---|---|
| Ber.: | C 73,50 | H 7,90 | N 6,86 |
| Gef.: | 73,90 | 8,06 | 7,00 |

(aa) 4-[(1-(2-Hexahydroazepino-phenyl)-1-butyl)-aminocarbonylmethyl]-benzoesäure
Ausbeute: 65,6% der Theorie,
Schmelzpunkt: 199—202°C

| | | | |
|---|---|---|---|
| Ber.: | C 73,50 | H 7,90 | N 6,86 |
| Gef.: | 73,50 | 7,90 | 6,76 |

(ab) 4-[(1-(4-Fluor-2-piperidino-phenyl)-1-butyl)-aminocarbonylmethyl]-benzoesäure
Ausbeute: 87,1% der Theorie,
Schmelzpunkt: 204—207°C

| | | | |
|---|---|---|---|
| Ber.: | C 69,88 | H 7,09 | N 6,79 |
| Gef.: | 70,25 | 7,02 | 7,12 |

(ac) 4-[(1-(5-Fluor-piperidino-phenyl)-1-butyl)-aminocarbonylmethyl]-benzoesäure
Ausbeute: 53,9% der Theorie,
Schmelzpunkt: 200—202°C

| | | | |
|---|---|---|---|
| Ber.: | C 69,88 | H 7,09 | N 6,79 |
| Gef.: | 69,67 | 7,24 | 6,90 |

## Beispiel 26

4-[(2-Methyl-1-(2-piperidino-phenyl)-1-propen-1-yl)-aminocarbonylmethyl]-benzoesäure

Man rührt das Gemisch aus 3,5 g (8,3 mMol) 4-[(2-Methyl-1-(2-piperidino-phenyl)-1-propen-1-yl)-aminocarbonylmethyl]benzoesäure-äthylester und 12,5 ml 1 N-Natronlauge in 35 ml Äthanol 2 Stunden bei 60°C. Man neutralisiert mit 12,5 ml 1N Salzsäure, dampft im Vakuum ein und verteilt zwischen Äthylacetat und Wasser. Den getrockneten filtrierten organischen Extrakt dampft man im Vakuum ein. Den Eindampfrückstand kristallisiert man aus Äthanol.

Ausbeute: 2,4 g (73,6% der Theorie),
Schmelzpunkt: 188—191°C

| | | | |
|---|---|---|---|
| Ber.: | C 73,44 | H 7,19 | N 7,14 |
| Gef.: | 73,60 | 7,19 | 7,02 |

5 Analog Beispiel 26 wurden folgende Verbindungen erhalten:

(a) (E)-4-[(1-(2-Piperidino-phenyl)-1-buten-1-yl)-aminocarbonylmethyl]-benzoesäure
Ausbeute: 71,5% der Theorie,
Schmelzpunkt: 188—190°C

| | | | |
|---|---|---|---|
| Ber.: | C 73,44 | H 7,19 | N 7,14 |
| Gef.: | 73,15 | 7,13 | 7,10 |

Olefinisches Proton: $^1H$—NMR ($CDCl_3$): $\delta = 6,42$ ppm

(b) (Z)-4-[(1-(2-Piperidino-phenyl)-1-buten-1-yl)-aminocarbonylmethyl]-benzoesäure
Ausbeute: 57,8% der Theorie,
Schmelzpunkt: 174—175°C (Äthanol)

| | | | |
|---|---|---|---|
| Ber.: | C 73,44 | H 7,19 | N 7,14 |
| Gef.: | 73,54 | 6,97 | 7,17 |

Olefinisches Proton: $^1H$—NMR ($CDCl_3$): $\delta = 5,60$ ppm

(c) (E)-4-[(2-Phenyl-1-(2-piperidino-phenyl)-äthen-1-yl)-aminocarbonylmethyl]-benzoesäure × 0,4 $H_2O$
Ausbeute: 33,2% der Theorie,
Schmelzpunkt: 165—167°C (Äther/Petroläther)

| | | | |
|---|---|---|---|
| Ber. (× 0,4 $H_2O$): | C 75,11 | H 6,48 | N 6,26 |
| Gef.: | 75,22 | 6,39 | 6,26 |

Olefinisches Proton: $^1H$—NMR ($CDCl_3$): $\delta > 6,9$ ppm

(d) (Z)-4-[(2-Phenyl-1-(2-piperidino-phenyl)-äthen-1-yl)aminocarbonylmethyl]-benzoesäure × 1 $H_2O$
Ausbeute: 72% der Theorie,
Schmelzpunkt: 182—185°C (Methanol)

| | | | |
|---|---|---|---|
| Ber. (×1 $H_2O$): | C 73,34 | H 6,60 | N 6,11 |
| Gef.: | 73,55 | 6,45 | 6,00 |

Olefinisches Proton: $^1H$—NMR ($CDCl_3$): $\delta = 6,50$ ppm

(e) 4-[(3-Phenyl-1-(2-piperidino-phenyl)-1-propen-1-yl)-aminocarbonylmethyl]-benzoesäure
Ausbeute: 48,3% der Theorie,
Schmelzpunkt: 162—164°C (Äther); wahrscheinlich (Z)-Form

| | | | |
|---|---|---|---|
| Ber.: | C 76,63 | H 6,65 | N 6,16 |
| Gef.: | 76,30 | 6,47 | 6,31 |

Olefinisches Proton: $^1H$—NMR ($CDCl_3$): δ = 5,80 ppm

(f) 4-[(1-(2-(3,3-Dimethyl-piperidino)-phenyl)-1-buten-1-yl)-aminocarbonylmethyl]-benzoesäure
Ausbeute: 64,1% der Theorie,
Schmelzpunkt: 152—153°C (Äthylacetat); wahrscheinlich (Z)-Form

| | | | |
|---|---|---|---|
| Ber.: | C 74,26 | H 7,67 | N 6,67 |
| Gef.: | 73,93 | 7,57 | 6,50 |

Olefinisches Proton: $^1H$—NMR ($CDCl_3$): δ = 5,55 ppm

(g) (Z)-4-[(1-(6-Methyl-2-piperidino-phenyl)-1-buten-1-yl)-aminocarbonylmethyl]-benzoesäure
Ausbeute: 53,3% der Theorie,
Schmelzpunkt: 142—145°C

| | | | |
|---|---|---|---|
| Ber.: | C 73,66 | H 7,44 | N 6,89 |
| Gef.: | 73,56 | 7,73 | 7,15 |

Olefinisches Proton: $^1H$—NMR ($CDCl_3$): δ = 5,38 ppm

### Beispiel 27

4-[(1-(2-Piperidino-phenyl)-1-butyl)-aminocarbonylmethyl]benzoesäure

Man hydriert 200 mg (0,51 mMol) 4-[(1-(2-Piperidino-phenyl)-1-buten-1-yl)-aminocarbonylmethyl]-benzoesäure in 10 ml absolutem Äthanol an 100 mg Palladium/Kohle (10%ig) bei 50°C und 1 bar Wasserstoff unter Schütteln. Nach 1, 5 Stunden filtriert man und dampft im Vakuum ein.

Ausbeute: 68% der Theorie,
Schmelzpunkt: 213—214°C

| | | | |
|---|---|---|---|
| Ber.: | C 73,07 | H 7,66 | N 7,10 |
| Gef.: | 73,21 | 7,82 | 7,02 |

Die Ausbeute beträgt 56% der Theorie, wenn man bei 50°C und 1 bar Wasserstoff an Raney-Nickel hydriert.

### Beispiel 28

4-[(1-(2-Piperidino-phenyl)-1-butyl)-aminocarbonylmethyl]-benzoesäure-Natriumsalz × 0,5 $H_2O$

Man löst 10,0 g (25,35 mMol) 4-[(1-(2-Piperidino-phenyl)-1-butyl)-aminocarbonylmethyl]-benzosäure bei 50°C in 200 ml Äthanol und fügt 25,35 ml 1 N-Natronlauge hinzu. Man dampft im Vakuum zur Trochne ein und löst den Eindampfrückstand unter Erhitzen auf dem Dampfbad in der minimalen Menge Äthanol. Man kühlt im Eisbad, filtriert von den ausgefallenen Kristallen ab, wäscht mit Äther und trocknet bei 140°C/15 Torr.

Ausbeute: 9g (85,3% der Theorie),
Schmelzpunkt: 280—285°C (Zers.); Erweichung ab 255°C

| | | | |
|---|---|---|---|
| Ber. (× 0,5 $H_2O$): | C 67,74 | H 6,87 | N 66,58 |
| Gef.: | 67,86 | 7,13 | 6,49 |

### Beispiel 29

(+)-4-[(1-(2-Piperidino-phenyl)-1-butyl)-aminocarbonylmethyl]-benzosäure-äthylester

Zu einer gerührten Lösung von 2,58 g (11,1 mMol) (+)-1-(2-Piperidino-phenyl)-1-butylamin [$K_{p\ 0,03}$: 87°C; ee = 86 (HPLC, nach Derivatisierung mit (+)-1-Phenäthyl-isocyanat)] in 26 ml Acetonitril gibt man bei 20°C nacheinander 2,31 g (11,1 mMol) 4-Äthoxycarbonyl-phenylessigsäure, 3,50 g (13,3 mMol) Triphenylphosphin, 4,60 ml (33,9 mMol) Triäthylamin und 1,03 ml (11,1 mMol) Tetrachlorkohlenstoff. Nach 14 Stunden bei 20°C und 1,5 Stunden bei 40°C dampft man im Vakuum ein und verteilt zwischen Wasser und Äther. Die organische Phase trocknet man über Natriumsulfat, filtriert sie und dampft im Vakuum ein. Den Eindampfrückstand reinigt man durch Säulenchromatographie an Kieselgel (Toluol/Aceton = 6:1).

Ausbeute: 2,63 g (56% der Theorie),
Schmelzpunkt: 118—120°C

| | | | |
|---|---|---|---|
| Ber.: | C 73,90 | H 8,11 | N 6,63 |
| Gef.: | 74,02 | 7,97 | 6,51 |

$[\alpha]_D^{20}$ = + 9,2° (c = 1; Methanol)

Analog Beispiel 29 wurde folgende Verbindung erhalten:
(a) (−)-4-[(1-(2-Piperidino-phenyl)-1-butyl)-aminocarbonylmethyl]-benzoesäure-äthylester
Hergestellt aus (−)-1-(2-Piperidino-phenyl)-1-butylamin X 1,4 HCl [$[\alpha]_D^{20}$ = − 20,0° (c = 1, Methanol),

Schmelzbereich: 90—100°C; ee = 80 (HPLC, nach Derivatisierung der base mit (+)-1-phenäthyl-isocyanat)]

Ausbeute: 52,6% der Theorie,
Schmelzpunkt: 115—120°C

| | | | |
|---|---|---|---|
| Ber.: | C 73,90 | H 8,11 | N 6,63 |
| Gef.: | 73,83 | 8,01 | 6,47 |

$[\alpha]_D^{20} = -9,0°$ (c = 1, Methanol)

Beispiel 30

(+)-4-[(1-(2-Piperidino-phenyl)-1-butyl)-aminocarbonylmethyl]-benzosäure-äthylester

Man suspendiert 1,0 g (3,27 mMol) (+)-1-(2-Piperidino-phenyl)-1-butylamin-dihydrochlorid [$[\alpha]_D^{20} = +$ 18,7°
(c = 1, Methanol); Schmelzpunkt: ab 115°C Zersetzung;
ee = 91,6 (HPLC, nach Derivatisierung der Base mit (+)-1-Phenäthyl-isocyanat)] in 6 ml Methylenchlorid, gibt unter Rühren 1,4 ml (10 mMol) Triäthylamin zu und tropft dann die Lösung von 0,82 g (3,64 mMol) 4-Äthoxycarbonyl-phenylessigsäurechlorid in 2,4 ml Methylenchlorid zu, wobei die Reaktionstemperatur von 22°C auf 38°C ansteigt. Man rührt 6 Stunden bei Raumtemperatur und schüttelt nacheinander aus:
zwiemanl mit je 10 ml Wasser,
einmal mit 10 ml 2 N-Salzsäure und
einmal mit 10 ml Wasser.

Man trocknet die organische Phase über natriumsulfat, filtriert sie und dampft im Vakuum ein. Den Eindampfrückstand reinigt man durch Säulenchromatographie an Kieselgel (Toluol/Aceton = 6/1).

Ausbeute: 0,53 g (38,2% der Theorie),
Schmelzpunkt: 120—122°C

| | | | |
|---|---|---|---|
| Ber.: | C 73,90 | H 8,11 | N 6,63 |
| Gef.: | 73,96 | 7,98 | 6,61 |

$[\alpha]_D^{20} = +9,0°$ (C = 1, Methanol)

Beispiel 31

(+)-4-[(1-(2-Piperidino-phenyl)-1-butyl)-aminocarbonylmethyl]-benzoesäure

Man rührt 2,09 g (4,73 mMol) (+)-4-[(1-(2-Piperidino-phenyl)-1-butyl)-aminocarbonylmethyl]-benzosäure-äthylester [$[\alpha]_D^{20} = +9,2°$ (c = 1, Methanol)] in 20 ml Äthanol zusammen mit 7,0 ml 1 N-Natronlauge 2,5 Stunden im Bad von 65°C. Man kühlt ab und gibt 7,0 ml 1 N-Salzsäure zu. Die sich langsam ausscheidenden Kristalle filtriert man ab, wäscht sie mit Wasser und trocknet bei 100°C/4 Torr.

Ausbeute: 1,65 g (88,2% der Theorie),
Schmelzpunkt: 185—187°C

| | | | |
|---|---|---|---|
| Ber.: | C 73,07 | H 7,66 | N 7,10 |
| Gef.: | 72,90 | 7,80 | 7,17 |

$[\alpha]_D^{20} = +7,9°$ (c = 1, Methanol)

Analog Beispiel 31 wurde folgende Verbindung erhalten:

(a) (−)-4-[(1-(2-Piperidino-phenyl)-1-butyl)-aminocarbonylmethyl]-benzoesäure
Ausbeute: 80% der Theorie,
Schmelzpunkt: 187—190°C

| | | | |
|---|---|---|---|
| Ber.: | C 73,07 | H 7,66 | N 7,10 |
| Gef.: | 72,98 | 7,44 | 7,22 |

$[\alpha]_D^{20} = -7,9°$ (c = 1, Methanol)

Beispiel 32

4-[(1-(2-Piperidino-phenyl)-1-butyl)-aminocarbonylmethyl]benzonitril

Hergestellt aus 1-(2-Piperidino-phenyl)-1-butylamin und 4-Cyano-phenylessigsäure analog Beispiel 18
Ausbeute: 57,3% der Theorie,
Schmelzpunkt: 147—148°C

| | | | |
|---|---|---|---|
| Ber.: | C 76,76 | H 7,78 | N 11,19 |
| Gef.: | 76,46 | 7,81 | 11,10 |

Analog Beispiel 32 wurde folgenden Verbindung erhalten:

(a) 4-[(1-(2-Piperidino-phenyl)-1-butyl)-aminocarbonylmethyl]-toluol
Hergestellt mit 4-Tolyl-essigsäure.
Ausbeute: 60,4% der Theorie,
Schmelzpunkt: 150—153°C

| | | | |
|---|---|---|---|
| Ber.: | C 79,08 | H 8,85 | N 7,68 |
| Gef.: | 78,97 | 8,58 | 7,77 |

Beispiel 33

4-[(1-(2-Piperidino-phenyl)-1-butyl)-aminocarbonylmethyl]-benzoesäure-äthylester

Hergestellt aus 4-[(1-(2-Piperidino-phenyl)-1-butyl)-aminocarbonylmethyl]-benzonitril mit äthanolischer Salzsäure analog Beispiel 14.

Ausbeute: 58% der Theorie,
Schmelzpunkt: 127—128°C

| | | | |
|---|---|---|---|
| Ber.: | C 73,90 | H 8,11 | N 6,63 |
| Gef.: | 74,07 | 8,23 | 6,87 |

Beispiel 34

4-[(1-(2-Piperidino-phenyl)-1-butyl)-aminocarbonylmethyl]-benzoesäure-äthylester

Hergestellt analog Beispiel 10 aus 1-(2-Piperidino-phenyl)-1-butanol und 4-Cyanomethyl-benzosäure-äthylester mit konzentrierter Schwefelsäure in o-Dichlorbenzol bei Raumtemperatur.

Ausbeute: 21% der Theorie),
Schmelzpunkt: 126—128°C

| | | | |
|---|---|---|---|
| Ber.: | C 73,90 | H 8,11 | N 6,63 |
| Gef.: | 74,12 | 8,20 | 6,45 |

Analog Beispiel 34 wurde folgende Verbindung erhalten:

(a) 4-[(1-(2-Piperidino-phenyl)-1-butyl)-aminocarbonylmethyl]-benzoesäure

Hergestellt aus 1-(2-Piperidino-phenyl)-1-butanol und 4-Cyanomethyl-benzoesäure. Extraktion bei pH 5,5.

Ausbeute: 29% der Theorie,
Schmelzpunkt: 215—217°C

| | | | |
|---|---|---|---|
| Ber.: | C 73,07 | H 7,66 | N 7,10 |
| Gef.: | 72,82 | 7,69 | 6,95 |

Beispiel 35

4-[(1-(4-Amino-2-piperidino-phenyl)-1-butyl)-aminocarbonylmethyl]-benzoesäure × 0,5 $H_2O$

Man hydriert 0,60 g (1,365 mMol) 4-[(1-(4-Nitro-2-piperidino-phenyl)-1-butyl)-aminocarbonylmethyl]-benzoesäure in 10 ml Dimethylformamid an 0,1 g Palladium/Kohle (10% ig) 3 Stunden bei 25°C und 1 bar Wasserstoff. Man filtriert vom Katalysator über Kieselgur ab und dampft im Vakuum ein. Den Eindampfrückstand kristallisiert man aus Äther.

Ausbeute: 0,41 g (73,2% der Theorie),
Schmelzpunkt: 118—120°C

| | | | |
|---|---|---|---|
| Ber.(× 0,5 $H_2O$): | C 68,87 | H 7,71 | N 10,04 |
| Gef.: | 68,62 | 7,64 | 10,08 |

Analog Beispiel 35 wurden folgende Verbindungen erhalten:

(a) 4-[(1-(4-Amino-2-piperidino-phenyl)-1-butyl)-aminocarbonylmethyl]-benzoesäure-äthylester

Ausbeute: 81,7% der Theorie,
Schmelzpunkt: 145—146°C (Äther/Petroläther)

| | | | |
|---|---|---|---|
| Ber.: | C 71,37 | H 8,06 | N 9,60 |
| Gef.: | 71,50 | 8,08 | 9,68 |

(b) 4-[(1-(5-Amino-2-piperidino-phenyl)-1-butyl)-aminocarbonylmethyl]-benzoesäure

Ausbeute: 64% der Theorie,
Schmelzpunkt: 227—230°C

| | | | |
|---|---|---|---|
| Ber.: | C 70,39 | H 7,63 | N 10,26 |
| Gef.: | 70,54 | 7,54 | 10,36 |

(c) 4-[(1-(5-Amino-2-piperidino-phenyl)-1-butyl)-aminocarbonylmethyl]-benzoesäure-äthylester

Ausbeute: 84,3% der Theorie,
Schmelzpunkt: 162—165°C

| | | | |
|---|---|---|---|
| Ber.: | C 71,37 | H 8,06 | N 9.60 |
| Gef.: | 71,58 | 7,83 | 9,65 |

Beispiel 36

4-[(1-(5-Chlor-2-piperidino-phenyl)-1-butyl)-aminocarbonylmethyl]-benzoesäure-äthylester

Man stellt aus 2,0 g (4,57 mMol) 4-[(1-(5-Amino-2-piperidino-phenyl)-1-butyl)-aminocarbonylmethyl]-benzoesäure-äthylester in 4,8 ml halbkonzentrierter Salzsäure und 0,315 g (4,57 mMol) Natriumnitrit in 1,66 ml Wasser eine 0°C kalte Diazoniumsalz-Lösung her. Diese tropft man bei 0—5°C in ein gerührtes Gemisch von 0,59 g (5,94 mMol) Kupfer(I)chlorid und 2,4 ml konz. Salzsäure und erwärmt anschließend im bad von 50°C. nach Beendigung der Gasentwicklung (ca. 15 Minuten) kühlt man ab, trägt das Reaktionsgemisch in

Eis/konz. Ammoniak ein und extrahiert viermal mit je 100 ml Äthylacetat. Die vereinigten organischen Extrakte Schüttelt man mit Wasser aus, trocknet und filtriert sie und dampft sie im Vakuum ein. Den Eindampfrückstand reinigt man durch Säulenchromatographie an Kieselgel (Toluol/Äthylacetat = 10/1).

Ausbeute: 0,80 g (40% der Theorie),
Schmelzpunkt: 137—140°C (Äther)

| | | | | |
|---|---|---|---|---|
| Ber.: | C 68,32 | H 7,27 | Cl 7,75 | N 6,13 |
| Gef.: | 68,42 | 7,09 | 8,06 | 6,05 |

Analog Beispiel 36 worden folgende Verbindungen erhalten:

(a) 4-[(1-(4-Chlor-2-piperidino-phenyl)-1-butyl)-aminocarbonylmethyl]-benzoesäure-äthylester
Ausbeute: 21,9% der Theorie,
Schmelzpunkt: 123—125°C

| | | | | |
|---|---|---|---|---|
| Ber.: | C 68,32 | H 7,27 | Cl 7,75 | N 6,13 |
| Gef.: | 68,70 | 7,18 | 7,77 | 6,08 |

(b) 4-[(1-(5-Brom-2-piperidino-phenyl)-1-butyl)-aminocarbonylmethyl]-benzoesäure-äthylester
Ausbeute: 53,8% der Theorie,
Schmelzpunkt: 140—142°C

| | | | | |
|---|---|---|---|---|
| Ber.: | C 62,27 | H 6,63 | Br 15,93 | N 5,58 |
| Gef.: | 62,39 | 6,78 | 15,85 | 5,59 |

(c) 4-[(1-(4-Fluor-2-piperidino-phenyl)-1-butyl)-aminocarbonylmethyl]-benzoesäure-äthylester
Ausbeute: 21,6% der Theorie,
Schmelzpunkt: 110—112°C

| | | | |
|---|---|---|---|
| Ber.: | C 70,88 | H 7,55 | N 6,36 |
| Gef.: | 71,01 | 7,53 | 6,21 |

Daneben isoliert man noch 40% 4-[(1-(4-Hydroxy-2-piperidino-phenyl)-1-butyl)-aminocarbonylmethyl]-benzoesäure-äthylester (fester Schaum).

(d) 4-[(1-(5-Fluor-2-piperidino-phenyl)-1-butyl)-aminocarbonylmethyl]-benzoesäure-äthylester
Ausbeute: 2% der Theorie,
Schmelzpunkt: 127—129°C
Ber.: m/e = 440
Gef.: m/e = 440

(e) 4-[(1-(4-Fluor-2-piperidino-phenyl)-äthyl)-aminocarbonylmethyl]-benzoesäure
Ausbeute: 16,9% der Theorie,
Schmelzpunkt: 172—175°C

| | | | |
|---|---|---|---|
| Ber.: | C 68,73 | H 6,55 | N 7,29 |
| Gef.: | 68,78 | 6,62 | 7,31 |

## Beispiel 37

### 4-[(1-(2-Piperidino-phenyl)-1-butyl)-aminocarbonylmethyl]-benzoesäure

Man hydriert 1,0 g (2,33 mMol 4-[(1-(5-Chlor-2-piperidino-phenyl)-1-butyl)-aminocarbonylmethyl]-benzoesäure in 40 ml absolutem Äthanol an 0,5 g Palladium/Kohle (10%ig) bei 50°C und 5 bar Wasserstoff. Nach 2 Stunden filtriert man vom Katalysator über Kieselgur ab und dampft im Vakuum ein. Den Eindampfrückstand verteilt man bei pH 6 zwiswchen Wasser und Äthylacetat. Den organischen Extrakt wäscht man mit Wasser, trocknet und filtriert ihn und dampft im Vakuum ein.

Ausbeute: 0,61 g (66% der Theorie),
Schmelzpunkt: 213—215°C

| | | | |
|---|---|---|---|
| Ber.: | C 73,07 | H 7,66 | N 7,10 |
| Gef.: | 73,18 | 7,42 | 7,27 |

Zu der gleichen Verbindung gelangt man auch aus den entsprechenden 4-Chlor-, 3-Chlor- oder 6-Chlorsubstituierten Ausgangsprodukten.

## Beispiel 38

### 4-[(1-(4-Methoxy-2-piperidino-phenyl)-1-butyl)-aminocarbonylmethyl]-benzoesäure-äthylester

Zu 548 mg (11, 4 mMol) Natriumhydrid (50 %ig in Öl) in 10 ml absolutem Dimethylformamid tropft man unter Rühren bei Raumtemperatur die Lösung von 5,0 g (11,4 mMol) 4-[(1-(4-Hydroxy-2-piperidino-phenyl)-1-butyl)-aminocarbonylmethyl]-benzoesäure-äthylester in 45 ml absolutem Dimethylformamid. Man läßt 15 Minuten nachrühren und tropft dann die Lösung von 0,71 ml (11,4 mMol) Methyljodid in 8 ml absolutem Dimethylformamid langsam zu. Man rührt noch 2,5 Stunden bei Raumtemperatur, dampft im Vakuum ein und verteilt zwischen Wasser und Äther. die Ätherephase trocknet und filtriert man und dampft

sie im Vakuum ein. Den eindampfrückstand reinigt man durch Säulenchromatographie an Kieselgel (Toluol/Aceton = 20/1).

Ausbeute: 1,8 g (34,9% der Theorie),
Schmelzpunkt: 115—117°C

| | | | |
|---|---|---|---|
| Ber.: | C 71,65 | H 8,02 | N 6,19 |
| Gef.: | 71,47 | 7,86 | 6,19 |

Analog Beispiel 38 wurde folgende Verbindung erhalten:
(a) 4-[(1-(5-Methoxy-2-piperidino-phenyl)-1-butyl)-aminocarbonylmethyl]-benzoesäure-äthylester
Ausbeute: 68,4% der Theorie,
Schmelzpunkt: 142—145°C

| | | | |
|---|---|---|---|
| Ber.: | C 71,65 | H 8,02 | N 6,19 |
| Gef.: | 71,87 | 7,06 | 6,38 |

## Beispiel 39

4-[(1-(2-Piperidino-phenyl)-1-butyl)-aminocarbonylmethyl]-benzoesäure-(2,3-dihydroxy-propyl)ester

Man erhitzt die Lösung von 2,0 g (5,07 mMol) 4-[(1-(2-Piperidino-phenyl)-1-butyl)-aminocarbonylmethyl]-benzoesäure und 0,85 g (5,27 mMol) N,N'-Carbonyldiimidazol in 20 ml absolutem Tetrahydrofuran 1 Stunde auf Rückfluß, fügt 3,7 ml (50,7 mMol) Glycerin zu und erhitzt weitere 15 Stunden auf Rückfluß. Man dampft im Vakuum ein, verteilt zwischen Wasser und Äthylacetat, trocknet und filtriert die organische Lösung und dampft sie im Vakuum ein. Den Eindampfrückstand reinigt man durch Säulenchromatographie an Kieselgel (Toluol/Aceton = 1/1).

Ausbeute: 1,1 g (46,2% der Theorie),
Schmelzpunkt: 120—122°C

| | | | |
|---|---|---|---|
| Ber.: | C 69,21 | H 7,74 | N 5,98 |
| Gef.: | 69,23 | 7,78 | 5,93 |

Analog Beispiel 39 wurden folgende Verbindungen erhalten:
(a) 4-[(1-(2-Piperidino-phenyl)-1-butyl)-aminocarbonylmethyl]-benzoesäure-(2-hydroxy-äthyl)ester
Ausbeute: 80% der Theorie,
Schmelzpunkt: 125—127°C

| | | | |
|---|---|---|---|
| Ber.: | C 71,21 | H 7,81 | N 6,39 |
| Gef.: | 71,35 | 7,54 | 6,33 |

(b) 4-[(1-(2-Piperidino-phenyl)-1-butyl)-aminocarbonylmethyl]-benzoesäure-(2-methoxy-äthyl)ester
Ausbeute: 55,9% der Theorie,
Schmelzpunkt: 120—123°C

| | | | |
|---|---|---|---|
| Ber.: | C 71,65 | H 8,02 | N 6,19 |
| Gef.: | 72,03 | 8,03 | 6,24 |

## Beispiel 40

4-[(1-(2-Piperidino-phenyl)-1-butyl)-aminocarbonylmethyl]-benzoesäure-(2-nicotinoyloxy-äthyl)ester

Zu einer gerührten Lösung von 2,0 g (4,56 mMol) 4-[(1-(2-Piperidino-phenyl)-1-butyl)-aminocarbonylmethyl]-benzoesäure-(2-hydroxy-äthyl)ester in 40 ml Methylenchlorid und 0,7 ml (4,81 mMol) Triäthylamin tropft man rasch die Lösung von 0,7 g (4,68 mMol) Nicotinsäurechlorid in 20 ml Methylenchlorid. Man läßt 2,5 Stunden bei 20°C rühren, schüttelt mit Wasser aus, trocknet und filtriert die organische Phase und dampft sie im Vakuum ein. Den Eindampfrückstand reinigt man durch Säulenchromatographie an Kieselgel (Toluol/Aceton = 5/1).

Ausbeute: 1,1 g (44% der Theorie),
Schmelzpunkt: 132—135°C

| | | | |
|---|---|---|---|
| Ber.: | C 70,70 | H 6,86 | N 7,73 |
| Gef.: | 70,82 | 6,82 | 7,91 |

## Beispiel 41

4-[(1-(2-Piperidino-phenyl)-1-butyl)-aminocarbonylmethyl]-benzylalkohol

Zu einer gerührten Suspension von 0,68 g (17,95 mMol) Lithiumaluminiumhydrid in 25 ml absolutem Tetrahydrofuran tropft man bei einer Innentemperatur von 0°C die Lösung von 5,0 g (11,83 mMol) 4-[(1-(2-Piperidino-phenyl)-1-butyl)-aminocarbonylmethyl]-benzoesäure-äthylester in 75 ml absolutem Tetrahydrofuran. Man läßt 20 Stunden bei Raumtemperatur rühren, kühlt auf 0°C ab und tropft langsam soviel 4 N-Natronlauge zu, bis ein filtrierbarer Niederschlag entstanden ist. Man filtriert ab und kocht den Niederschlag mehrmals mit Äther aus. Die vereinigten organischen Lösungen dampft man in Vakuum ein. Den Eindampfrückstand verteilt man zwischen Wasser und Äther. Die Ätherphase trocknet und filtriert man

und dampft sie im Vakuum ein. Den Eindampfrückstand reinigt man durch Säulenchromatographie an Kieselgel (Toluol/Aceton = 5/1).

Ausbeute: 1,0 g (22% der Theorie),
Schmelzpunkt: 152—154°C

| | | | |
|---|---|---|---|
| Ber.: | C 75,75 | H 8,48 | N 7,36 |
| Gef.: | 75,90 | 8,45 | 7,28 |

Beispiel 42

4-[(1-(2-Piperidino-phenyl)-1-butyl)-aminocarbonylmethyl]-benzaldehyd

Man erhitzt 6,6 g (62 mMol) Natriumcarbonat zusammen mit 62 ml Äthylenglykol im Bad von 170°C und gibt unter raschem Rühren innerhalb 1 Minute 6,2 g (11 mMol) $N^1$-[4-[(1-(2-Piperidono-phenyl)-1-butyl)-aminocarbonylmethyl]-benzoyl]-$N^2$-tosyl-hydrazin vom Schmelzpunkt 195°C (Zers.) zu, wobei man eine heftige Gasentwicklung beobachtet. Anschließend erhitzt man noch 2,5 Minuten bei 170°C und gießt dann sofort aus Eis. Man extrahiert mit Äther, trocknet, filtriert und dampft die Ätherlösung im Vakuum ein. Den Eindampfrückstand reinigt man durch Säulenchromatographie an Kieselgel (Chloroform/Aceton = 20/1).

Ausbeute: 2,2 g (52,9% der Theorie),
Schmelzpunkt: 142—145°C

| | | | |
|---|---|---|---|
| Ber.: | C 76,16 | H 7,99 | N 7,40 |
| Gef.: | 76,26 | 7,96 | 7,37 |

Beispiel 43

4-[(1-(2-Piperidino-phenyl)-1-butyl)-aminocarbonylmethyl]-zimtsäure-äthylester

Zu 0,60 g (12,5 mMol) Natriumhydrid (50% ig in Öl,) in 15 ml absolutem Dimethylformamid tropft man bei Raumtemperatur die Lösung von 2,80 g (12,5 mMol) Diäthylphosphono-essigsäureäthylester in 10 ml absolutem Dimethylformamid. Man rührt 15 Minuten (bis zum Ende der Gasentwicklung) und tropft dann die Lösung von 2,4 g (6,34 mMol) 4-[(1-(2-Piperidino-phenyl)-1-butyl)-aminocarbonylmethyl]-benzaldehyd in 10 ml absolutem Dimethylformamid zu. Man läßt 2 Stunden bei Raumtemperatur rühren, dampft im Vakuum ein und verteilt zwischen Wasser und Äther. Die Ätherphase trocknet und filtriert man und dampft sie im Vakuum ein. Den Eindampfrückstand reinigt man durch Säulenchromatographie an Kieselgel (Toluol/Aceton = 10/1).

Ausbeute: 0,85 g (29,9% der Theorie),
Schmelzpunkt: 135—137°C (Äther/Petroläther)

| | | | |
|---|---|---|---|
| Ber.: | C 74,97 | H 8,09 | N 6,24 |
| Gef.: | 74,91 | 7,89 | 6,29 |

Beispiel 44

4-[(1-(2-Piperidino-phenyl)-1-butyl)-aminocarbonylmethyl]-zimtsäure

Hergestellt durch alkalische Verseifung von 4-[(1-(2-Piperidino-phenyl)-1-butyl)-aminocarbonylmethyl]-zimtsäure-äthylester analog Beispiel 25.

Ausbeute: 64% der Theorie,
Schmelzpunkt: 180—183°C

| | | | |
|---|---|---|---|
| Ber.: | C 74,26 | H 7,67 | N 6,66 |
| Gef.: | 74,03 | 7,47 | 6,80 |

Beispiel 45

3-[4-[(1-(2-Piperidino-phenyl)-1-butyl)-aminocarbonylmethyl]-propionsäure-äthylester

Man hydriert 0,60 g (1,34 mMol) 4-[(1-(2-Piperidino-phenyl)-1-butyl)-aminocarbonylmethyl]-zimtsäure-äthylester in 10 m Äthanol an 0,20 g Palladium/Kohle (10%) bei Raumtemperatur und 5 bar Wasserstoff. Man filtriert und dampft im Vakuum ein.

Ausbeute: 0,53 g (88% der Theorie),
Schmelzpunkt: 98—99°C (Petroläther)

| | | | |
|---|---|---|---|
| Ber.: | C 74,63 | H 8,50 | N 6,22 |
| Gef.: | 74,64 | 8,58 | 6,23 |

Analog Beispiel 45 wurde folgende Verbindung erhalten:

(a) 3-[4-[(1-(2-Piperidino-phenyl)-1-butyl)-aminocarbonylmethyl]-phenyl]-propionsäure

Ausbeute: 63% der Theorie,
Schmelzpunkt: 131—133°C

| | | | |
|---|---|---|---|
| Ber.: | C 73,90 | H 8,11 | N 6,63 |
| Gef.: | 73,96 | 8,30 | 6,56 |

Beispiel 46

3-[4-[(1-(2-Piperidino-phenyl)-1-butyl)-aminocarbonylmethyl]-phenyl]-propionsäure

Hergestellt durch alkalische Verseifung von 3-[4-[(1-(2-Piperidino-phenyl)-1-butyl)-aminocarbonylmethyl]-phenyl]-propionsäure-äthylester analog Beispiel 25.

Ausbeute: 50% der Theorie,
Schmelzpunkt: 131—133°C

| | | | |
|---|---|---|---|
| Ber.: | C 73,90 | H 8,11 | N 6,63 |
| Gef.: | 73,82 | 8,07 | 6,41 |

Beispiel 47

4-[(α-Aminocarbonyl-2-piperidino-benzyl)-aminocarbonylmethyl]-benzoesäure-äthylester

Zur gerührten Lösung von 2,0 g (4,7 mMol) 4-[(α-Carboxy-2-piperidino-benzyl)-aminocarbonylmethyl]-benzoesäure-äthylester × 0,167 $H_2O$; (Schmelzpunkt 156—159°C) in 20 ml wasserfreiem Tetrahydrofuran gibt man bei 20°C 0,90 g (5,5 mMol) N,N'-Carbonyldiimidazol und erhitzt dann eine halbe Stunde im Bad von 80°C. Anschließend kühlt man auf 60°C ab und leitet bei dieser Temperatur eine halbe Stunde lang einen kräftigen Strom trockenen Ammoniaks ein. Man dampft dann im Vakuum ein, verteilt zwischen Wasser und Chloroform, schüttelt die vereinigten Chloroform-Extrakte mit wenig Wasser aus, trocknet, filtriert und dampft sie im Vakuum ein. Den Eindampfrückstand reinigt man durch Säulenchromatographie an Kieselgel (Chloroform/Methanol = 5/1).

Ausbeute: 1,0 g (50,2% der Theorie),
Schmelzpunkt: 160—162°C (Aceton)

| | | | |
|---|---|---|---|
| Ber.: | C 68,07 | H 6,90 | N 9,92 |
| Gef.: | 68,40 | 6,92 | 9,84 |

Beispiel 48

4-[(α-Cyano-2-piperidino-benzyl)-aminocarbonylmethyl]benzoesäure-äthylester

Zu 520 mg (1,22 mMol) 4-[(α-Aminocarbonyl-2-piperidino-benzyl)-aminocarbonylmethyl]benzoesäure-äthylester om 0,22 ml Pyridin gibt man in 2 Portionen 234 mg (1,22 mMol) 4-Toluolsulfochlorid und erwärmt auf 50°C. Nach 2 Stunden und nach einer weiteren Stunde gibt man nochmals je die gleichen Mengen Pyridin und 4-Toluolsulfochlorid zu und erwärmt eine weitere Stunde bei 50°C. Nach zweitägigem Stehen bei 20°C gibt man 2-N-Ammoniak zu und extrahiert mit Chloroform. Die Chloroform-Lösung schüttelt man zweimal mit Wasser aus. Nach Trocknen und Filtrieren dampft man sie im Vakuum ein. Den Eindampfrückstand reinigt man durch Säulenchromatographie an Kieselgel (Chloroform/Methanol = 10/1).

Ausbeute: 325 mg (65,7% der Theorie),
Schmelzpunkt: 114—117°C (Äther/Petroläther)

| | | | |
|---|---|---|---|
| Ber.: | C 71,09 | H 6,71 | N 10,36 |
| Gef.: | 70,79 | 6,56 | 10,10 |

Beispiel 49

4-[(α-Cyano-2-piperidino-benzyl)-aminocarbonylmethyl]benzoesäure

Man rührt 1,5 g (3,7 mMol) 4-[(α-Cyano-2-piperidino-benzyl)-aminocarbonylmethyl]benzoesäure-äthylester in 15 ml Dioxan zusammen mit 3,7 ml 1 N-Natronlauge 45 Minuten im Bad von 60°C und weitere 45 Minuten im Bad von 80°C. Nach Abkühlen mit Eis versetzt man mit 3,7 ml N-Salzsäure, dampft dad Dioxan im Vakuum ab und verteilt zwischen Wasser und Chloroform. Die organische Lösung schüttelt man mit wenig Wasser aus, trocknet und filtriert sie und dampft sie im Vakuum ein. Den Eindampfrückstand reinigt man durch Säulenchromatographie an Kieselgel (Chloroform/Äthanol = 5/1).

Ausbeute: 0,50 g (35,7% der Theorie),
Schmelzpunkt: 176—180°C (Zers.)

| | | | |
|---|---|---|---|
| Ber.: | C 70,01 | H 6,14 | N 11,13 |
| Gef.: | 70,01 | 6,19 | 11,05 |

Beispiel 50

4-[(1-(2-Piperidino-phenyl)-1-butyl)-aminocarbonylmethyl]-benzoesäure × $H_2SO_4$

Zur Lösung von 1,0 g (2,53 mMol) 4-[(1-(2-Piperidino-phenyl)-1-butyl)-aminocarbonylmethyl]-benzoesäure in 50 ml Äthanol gibt man 5 ml (2,50 mMol) 1N Schwefelsäure, dampft im Vakuum zur Trockne ein und verreibt mit Aceton.

Ausbeute: 0,80 g (65% der Theorie),
Schmelzpunkt: 192—197°C (Zers.).

| | | | | |
|---|---|---|---|---|
| Ber.: | C 58,53 | H 6,55 | N 5,69 | S 6,49 |
| Gef.: | 58,05 | 6,54 | 5,49 | 6,35 |

Analog Beispiel 50 wurde folgendes Additionssalz erhalten:

(a) 4-[(1-(2-Piperidino-phenyl)-1-butyl)-aminocarbonylmethyl]-benzoesäure × 0,5 $H_2SO_4$ × 1,5 $H_2O$
Hergestellt mit der halben Menge der Schwefelsäure wie in Beispiel 50.
Ausbeute: 59,3% der Theorie,
Schmelzpunkt: 180—185°C Zers. bei 207—210°C.

| | | | | |
|---|---|---|---|---|
| Ber.: | C 61,26 | H 7,28 | N 5,95 | S 3,40 |
| Gef.: | 61,28 | 6,99 | 6,10 | 3,23 |

Beispiel A

Tabletten mit 5 mg 4-[(1-(2-Piperidino-phenyl)-1-butyl)-aminocarbonylmethyl]-benzoesäure

Zusammensetzung:
1 Tablette enthält:

| | | |
|---|---|---|
| Wirksubstanz | (1) | 5,0 mg |
| Maisstärke | (2) | 62,0 mg |
| Milchzucker | (3) | 48,0 mg |
| Polyvinylpyrrolidon | (4) | 4,0 mg |
| Magnesiumstearat | (5) | 1,0 mg |
| | | 120,0 mg |

Herstellungsverfahren:

1, 2, 3 und 4 werden gemischt und mit Wasser befeuchtet. Die feuchte mischung wird durch ein Sieb mit 1,5 mm Maschenweite gedrückt und be ca. 45°C getrocknet. Das trockene Granulat wird durch ein Sieb mit 1,0 mm Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg

Beispiel B

Dragées mit 2,5 mg 4-[(1-(2-Piperidino-phenyl)-1-butyl)-aminocarbonylmethyl]-benzoesäure

1 Dragéekern enthält:

| | | |
|---|---|---|
| Wirksubstanz | (1) | 2,5 mg |
| Kartoffelstärke | (2) | 44,0 mg |
| Milchzucker | (3) | 30,0 mg |
| Polyvinylpyrrolidon | (4) | 3,0 mg |
| Magnesiumstearat | (5) | 0,5 mg |
| | | 80,0 mg |

Herstellungsverfahren:

1, 2, 3 und 4 werden gemischt und mit Wasser befeuchtet. Die feuchte Masse drückt man durch ein Sieb mit 1 mm Maschenweite, trocknet bei ca. 45°C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 120 mg

Beispiel C

Tabletten mit 10 mg 4-[(1-(2-Piperidino-phenyl)-1-butyl)-aminocarbonylmethyl]-benzoesäure

Zusammensetzung:
1 Tablette enthält:

| | |
|---|---|
| Wirksubstanz | 10,0 mg |
| Milchzucker pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren:

Die Mischung aus der Wirksubstanz, Milchzucker und Maisstärke wird mit einer 20 %igen Lösung von Polyvinylpyrrolidon in Wasser befeuchtet. Die feuchte Masse wird durch ein Sieb mit 1,5 mm Maschenweite granuliert und bei 45°C getrocknet. Das getrocknete Granulat wird durch ein Sieb mit 1 mm Maschenweite gerieben und mit Magnesiumstearat homogen vermischt.

Tablettengewicht: 120 mg
Stempel: 7 mm Durchmesser mit Teilkerbe

Beispiel D

Dragées mit 5 mg 4-[(1-(2-Piperidino-phenyl)-1-butyl)-aminocarbonylmethyl]-benzoesäure

| 1 Dragéekern enthält: | |
|---|---|
| Wirksubstanz | 5,0 mg |
| Calciumphosphat sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren:

Die Mischung aus der Wirksubstanz, Calciumphosphat und Maisstärke wird mit einer 15%igen Lösung von Polyvinylpyrrolidon in Wasser befeuchtet. Die feuchte Masse wird durch ein Sieb mit 1 mm Maschenweite Geschlagen, bei 45°C getrocknet und anschließend durch dasselbe Sieb gerieben. Nach dem Vermischen mit der angegebenen Menge Magnesiumstearat werden daraus Dragéekerne gepreßt.

Kerngewicht: 130 mg
Stempel: 7 mm Durchmesser

Auf die so hergestellten Dragéekerne wird auf bekannte Art eine Schicht aus Zucker und Talkum aufgetragen. Die fertigen Dragées werden mit Wachs poliert.

Grageegewicht: 180 mg

**Patentansprüche**

1. Phenylessigsäure-Derivate der allgemeinen Formel

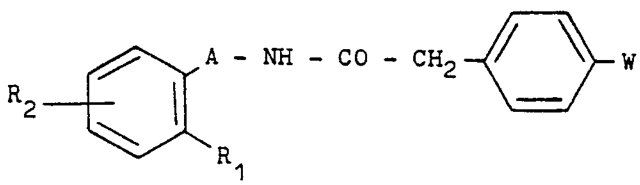
, (I)

in der

A ein Gruppe der Formeln

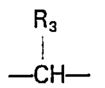 oder 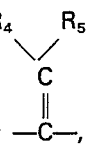, wobei

$R_3$ eine durch eine Alkoxygruppe mit 1 bis 3 Kohlenstoffatomen oder durch eine Phenylgruppe substituierte Alkylgruppe mit 1 bis 3 Kohlenstoffatomen, eine n-Propylgruppe, eine Alkylgruppe mit 4 bis 6 Kohlenstoffatomen, eine Alkenylgruppe mit 3 bis 5 Kohlenstoffatomen, eine Cyano- oder Alkyleniminocarbonylgruppe mit 4 bis 6 Kohlenstoffatomen im Alkylenteil, eine gegebenenfalls durch Alkyl- oder Phenylalkylgruppen mit jeweils 1 bis 3 Kohlenstoffatomen im Alkylteil mono- oder disubstituierte Aminocarbonylgruppe, eine durch Halogenatome, durch Alkyl-, Hydroxy-, Alkoxy-, Phenylalkoxy-, Alkylsulfenyl-, alkylsulfinyl- und/oder Alkylsulfonylgruppen mono- oder disubstituierte Arylgruppe mit 6 oder 10 Kohlenstoffatomen, wobei die Substituenten gleich oder verschieden sein können und der Alkylteil jeweils 1 bis 3 Kohlenstoffatome enthalten kann, eine Naphthylgruppe oder eine 1 oder 2 Stickstoffatome enthaltende Heteroarylgruppe mit 4, 5, 8 oder 9 Kohlenstoffatomen,

oder auch eine Methylgruppe,

wenn $R_1$ eine Piperidinogruppe sowie $R_2$ in 4-Stellung ein Fluoratom und W eine Carboxy- oder Alkoxycarbonylgruppe, in der der Alkylteil 1 bis 3 Kohlenstoffatome enthalten kann, darstellen,

oder auch eine Phenylgruppe,

wenn $R_1$ eine in 2- oder 3-Stellung durch eine Methylgruppe substituierte Piperidinogruppe, $R_2$ ein Wasserstoffatom und W ein Carboxy- oder Alkoxycarbonylgruppe, in der der Alkylteil 1 bis 3 Kohlenstoffatome enthalten kann, oder wenn $R_1$ eine Piperidinogruppe, $R_2$ in 3-, 4- oder 6-Stellung ein Chloratom oder in 4- oder 6-Stellung eine Methylgruppe und W eine Carboxy- oder Alkoxycarbonylgruppe, in der der Alkylteil 1 bis 3 Kohlenstoffatome enthalten kann, oder

wenn $R_1$ eine Piperidinogruppe, $R_2$ ein Wasserstoffatom und W eine Formylgruppe, eine 2-Carboxyäthenyl- oder 2-Alkoxycarbonyläthenylgruppe, in der der Alkylteil 1 bis 3 Kohlenstoffatome enthalten kann, darstellen,

$R_4$ und $R_5$ zusammen mit dem dazwischenliegenden Kohlenstoffatom eine Alkylidengruppe mit 3 bis 9 Kohlenstoffatomen oder eine Phenylalkylidengruppe mit 1 bis 3 Kohlenstoffatomen im Alkylidenteil,

$R_1$ eine gegebenenfalls durch Alkylgruppen mit 1 bis 3 Kohlenstoffatomen mono- oder disubstituierte unverzweigte Alkyleniminogruppe mit 4 bis 8 Kohlenstoffatomen,

$R_2$ ein Wasserstoff-, Fluor-, Chlor- oder Bromatom, eine Hydroxy-, Trifluoromethyl-, Nitro-, Amino-, Piperidino-, Alkyl-, Alkoxy, Alkylsulfenyl-, Alkylsulfinyl-, Alkylsulfonyl-, Phenylalkoxy-, Alkanoyloxy-, Alkanoylamino-, Alkylamino- oder Dialkylaminogruppe, wobei der Alkylteil jeweils 1 bis 3 Kohlenstoffatome enthalten kann, und

W eine Carboxygruppe oder eine Alkoxycarbonylgruppe mit insgesamt 2 bis 5 Kohlenstoffatomen, in welcher der Alkylteil durch eine Phenylgruppe und ab dem β-Kohlenstoffatom durch eine oder zwei Hydroxygruppen, durch eine Alkoxy-, Alkanoyloxy-, Dialkylamino-, Alkylenimino- oder Pyridincarbonyloxygruppe substituiert sein kann, wobei jeder Alkylteil jeweils 1 bis 3 Kohlenstoffatome und die Alkyleniminogruppe 4 bis 6 Kohlenstoffatome enthalten kann, eine Alkenyloxycarbonylgruppe mit insgesamt 4 bis 6 Kohlenstoffatomen, eine Alkylgruppe, mit 1 bis 3 Kohlenstoffatomen, eine Hydroxymethyl-, Formyl-, Cyano-, Aminocarbonyl-, Carboxy-methyl-, 2-Carboxy-äthyl-, 2-Carboxy-äthenyl-, 2,2-Bis-(carboxy)-äthyl-, Alkoxycarbonyl-methyl-, 2-Alkoxycarbonyl-äthyl-, 2-Alkoxycarbonyl-äthenyl- oder 2,2-Bis-(alkoxycarbonyl)-äthylgruppe, wobei die Alkoxygruppe jeweils 1 bis 3 Kohlenstoffatome enthalten kann, bedeuten,

sowie deren optisch aktive Antipoden und deren Salz mit anorganischen oder organischen Säuren oder Basen.

2. Phenylessigsäure-Derivate der allgemeinen Formel I gemäß Anspruch 1, in der

$R_3$ eine durch eine Alkoxygruppe mit 1 bis 3 Kohlenstoffatomen oder durch eine Phenylgruppe substituierte Alkylgruppe mit 1 bis 3 Kohlenstoffatomen, eine n-Propylgruppe, eine Alkylgruppe mit 4 bis 6 Kohlenstoffatomen, eine Alkenylgruppe mit 3 bis 5 Kohlenstoffatomen, eine Cyano- oder Aminocarbonylgruppe, eine durch ein Halogenatom, durch eine Alkyl-, Hydroxy-, Alkoxy-, phenylalkoxy-, alkylsulfenyl-, Alkylsulfinyl- oder Alkylsulfonylgruppe substituierte Phenylgruppe, wobei der Alkylteil jeweils 1 bis 3 Kohlenstoffatome enthalten kann, eine Naphthyl-, Pyridyl-, Chinolyl- oder Isochinolylgruppe,

$R_4$ und $R_5$, zusammen mit dem dazwischen liegenden Kohlenstoffatom eine Alkylidengruppe mit 3 bis 5 Kohlenstoffatomen oder eine Phenylalkylidengruppe mit 1 bis 3 Kohlenstoffatomen im Alkylidenteil,

$R_1$ eine unverzweigte Alkyleniminogruppe mit 4 bis 8 Kohlenstoffatomen oder eine durch Alkylgruppen mit 1 bis 3 Kohlenstoffatomen mono- oder disubstituierte Piperidinogruppe,

$R_2$ ein Wasserstoff-, Fluor-, Chlor- oder Bromatom, eine Nitro-, Alkyl- oder Alkoxygruppe mit jewiels 1 bis 3 Kohlenstoffatomen,

W eine Carboxy-, Hydroxymethyl-, Carboxymethyl-, 2-Carboxyäthyl-, 2-Hydroxy-äthoxycarbonyl-, 2-Methoxy-äthoxycarbonyl-, 2-Nicotinoyloxy-äthoxycarbonyl-, 2,3-Dihydroxy-n-propoxycarbonyl- oder Alkoxycarbonylgruppe mit insgesamt 2 bis 5 Kohlenstoffatomen, eine Formyl-, 2-Carboxy-äthenyl-, 2,2-Bis(carboxy)-äthyl-, 2-Alkoxycarbonyl-äthenyl-, 2-Alkoxycarbonyl-äthyl-, Alkoxycarbonylmethyl oder 2,2-Bis(alkoxycarbonyl)-äthylgruppe, wobei die Alkoxygruppe jeweils 1 bis 3 Kohlenstoffatome enthalten kann, bedeuten,

sowie deren optisch aktive Antipoden und deren Salze mit anorganischen oder organischen Säuren oder Basen.

3. Phenylessigsäure-Derivate der allgemeinen Formel I gemäß Anspruch 1, in der

$R_3$ eine n-Propylgruppe, eine Alkylgruppe mit 4 oder 5 Kohlenstoffatomen, eine durch eine Methylgruppe, durch ein Fluor- oder Chloratom substituierte Phenylgruppe oder eine Pyridylgruppe,

$R_4$ und $R_5$ zusammen mit dem dazwischenliegenden Kohlenstoffatom eine Alkylidengruppe mit 3 bis 5 Kohlenstoffatomen oder eine Phenylalkylidengruppe mit 1 bis 3 Kohlenstoffatomen im Alkylidenteil darstellen,

$R_1$ eine gegebenenfalls durch eine oder zwei Methylgruppen substituierte Piperidinogruppe,

$R_2$ ein Wasserstoff-, Fluor- oder Chloratom, eine Methyl- oder Methoxygruuppe und

W eine Carboxygruppe oder eine Alkoxycarbonylgruppe mit insgesamt 2 bis 4 Kohlenstoffatomen bedeuten, sowie deren optisch aktive Antipoden und deren Salze mit anorganischen oder organischen Säuren oder Basen.

4. Phenylessigsäure-Derivate der allgemeinen Formel I, gemäß Anspruch 1, in der
$R_1$, $R_2$ und W wie im Anspruch 3 definiert sind und
$R_3$ eine n-Propylgruppe oder eine Alkylgruppe mit 4 oder 5 Kohlenstoffatomen und
$R_4$ und $R_5$ zusammen mit dem dazwischenliegenden Kohlenstoffatom eine Alkylidengruppe mit 3 bis 5 Kohlenstoffatomen oder eine Phenylalkylidengruppe mit 1 bis 3 Kohlenstoffatomen im Alkylidenteil bedeuten, sowie deren optisch aktive Antipoden und deren Salze mit anorganischen oder organischen Säuren oder Basen.

5. 4-[(1-(2-Piperidino-phenyl)-1-butyl)-aminocarbonylmethyl]-benzoesäure, deren Alkylester mit 1 bis 3 Kohlenstoffatomen, deren optisch aktive Antipoden und deren Salze.

6. 4-[(1-(2-Piperidino-phenyl)-1-pentyl)-aminocarbonylmethyl]-benzoesäure, deren Alkylester mit 1 bis 3 Kohlenstoffatomen, deren optisch aktive antipoden und deren Salze.

7. Physioligisch verträgliche Salze der Verbindungen gemäß den Ansprüchen 1 bis 6 mit anorganischen oder organischen Säuren und auch Basen, sofern die erfindungsgemäßen Verbindungen eine Carboxygruppe enthalten.

8. Arzneimittel, enthaltend eine Verbindung gemäß den Ansprüchen 1 bis 6 oder ein physiologisch verträgliches Salz hiervon gemäß Anspruch 7 neben einem oder mehreren inerten Trägerstoffen und/oder Verdünnungsmitteln.

9.Verfahren zur Herstellung eines Arzneimittels gemäß Anspruch 8, dadurch gekennzeichnet, daß auf nichtchemischem Wege eine Verbindung gemäß den Ansprüchen 1 bis 7 in einen oder mehrere inerte Trägerstoffe und/oder Verdünnungsmittel eingearbeitet wird.

10. Verwendung der Verbindungen gemäß den Ansprüchen 1 bis 7 zur Herstellung von Arzneimitteln mit blutzuckersenkenden Eigenschaften.

11. Verfahren zur Herstellung der Phenylessigsäure-Derivate gemäß den Ansprüchen 1 bis 7, dadurch gekennzeichnet, daß

a) ein Amin der allgemeinen Formel

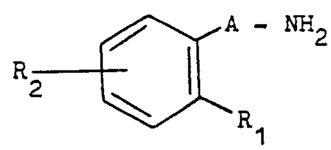 , (II)

in der
A, $R_1$ und $R_2$ wie in den Ansprüchen 1 bis 6 definiert sind, bwz., wenn a eine der eingangs erwähnten Vinylidengruppen darstellt, dessen Tautomeres, dessen Lithium- oder Magnesiumhalogenid-Komplex mit einer Carbonsäure der allgemeinen formel

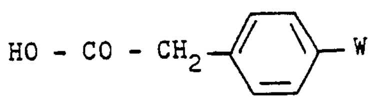 , (III)

in der
W' die für W in den Ansprüchen 1 bis 6 erwähnten Bedeutungen besitzt oder eine durch einen Schutzrest geschützte Carboxygruppe darstellt, oder mit deren gegebenenfalls im Reaktionsgemisch hergestellten reaktionsfähigen Derivaten, umgesetzt und erforderlichenfalls der verwendete Schutzrest abgespalten wird, oder

b) zur Herstellung einer Verbindung der allgemeinen Formel I, in der W eine Carboxy-, Carboxymethyl-, 2-Carboxy-äthyloder 2-Carboxy-äthenylgruppe darstellt, eine Verbindung der allgemeinen Formel

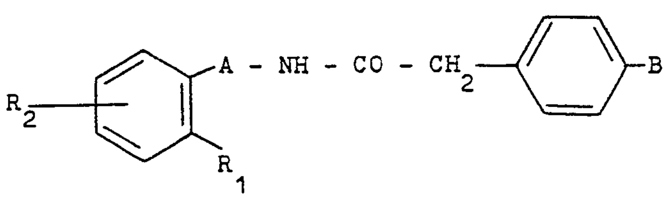 , (IV)

in der $R_1$, $R_2$ und A wie in den Ansprüchen 1 bis 6 definiert sind und
B eine in eine Carboxy-, Carboxymethyl-, 2-Carboxy-äthyl- oder 2-Carboxy-äthenylgruppe überführbare Gruppe darstellt, in die entsprechende Carboxyverbindung mittels Hydrolyse, Thermolyse oder Hydrogenolyse übergeführt wird, oder

c) zur Herstellung einer Verbindung der allgemeinen Formel I, in der A eine Gruppe der Formel

$R_3'$ darstellt, wobei
|
—CH—

$R_3'$ mit Ausnahme der Alkenylgruppe und der Cyanogruppe die für $R_3$ in den Ansprüchen 1 bis 6 erwähnten Bedeutungen besitzt, eine Verbindung der allgemeinen Formel

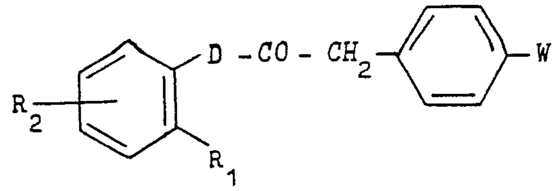 , (V)

in der $R_1$, $R_2$ und W wie in den Ansprüchen 1 b is 6 definiert sind und
D eine Gruppe der Formeln

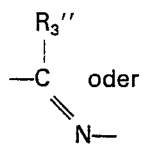 oder 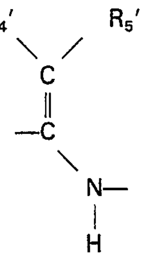

bedeutet, wobei $R_3''$ mit Ausnahme der Cyanogruppe die für $R_3$ eingangs erwähnten Bedeutungen besitzt und $R_4'$ und $R_5'$ zusammen mit dem dazwischen liegenden Kohlenstoffatom eine Propylidengruppe, eine Alkylidengruppe mit 4 bis 6 Kohlenstoffatomen oder eine Phenylalkylidengruppe mit 1 bis 3 Kohlenstoffatomen im Alkylidenteil bedeuten, mit Wasserstoff in Gegenwart eines Hydrierungskatalysators reduziert wird, oder

d) zur Herstellung von Verbindungen der allgemeinen Formel I, in der $R_3$ mit Ausnahme der Cyanogruppe die für $R_3$ in den Ansprüchen 1 bis 6 erwähnten Bedeutungen besitzt, eine Verbindung der allgemeinen Formel

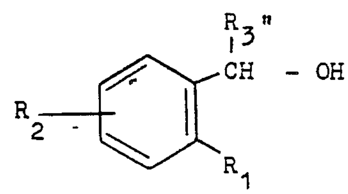 , (VI)

in der
$R_3''$ die für $R_3$ oben erwähnten Bedeutungen besitzt sowie $R_1$ und $R_2$ wie in den Ansprüchen 1 bis 6 definiert sind, mit einer Verbindung der allgemeinen Formel

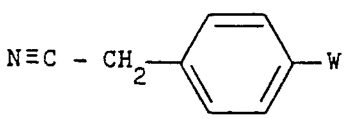 , (VII)

in der W wie in den Ansprüchen 1 bis 6 definiert ist, umgesetzt wird, oder

e) zur Herstellung von Verbindungen der Allgemeinen Formel I, in der $R_2$ ein Wasserstoffatom und A eine Gruppe der Formel

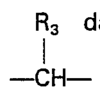 darstellen, wobei

$R_3$ wie in den Ansprüchen 1 bis 6 definiert ist, eine Verbindung der allgemeinen Formel

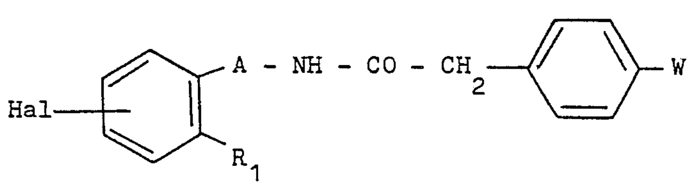 , (VIII)

in der
$R_1$, A und W wie in den Ansprüchen 1 bis 6 definiert sind und
Hal ein Fluor-, Chlor-, Brom- oder Jodatom darstellt, enthalogeniert wird, oder
f) zur Herstellung von Verbindungen der allgemeinen Formel I, in der A eine Gruppe der Formel

$R_3$
|
—CH— darstellen, wobei

$R_3$ eine Alkyleniminocarbonylgruppe mit 4 bis 6 Kohlenstoffatomen im Alkylenring oder eine gegebenenfalls durch Alkyl- oder Phenylalkylgruppen mit jeweils 1 bis 3 Kohlenstoffatomen im Alkylteil mono- oder disubstituierte Aminocarbonylgruppe darstellt, eine Verbindung der allgemeinen Formel

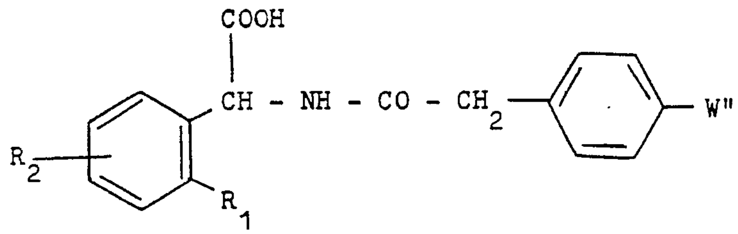
, (IX)

in der $R_1$ und $R_2$ wie in den Ansprüchen 1 bis 6 definiert sind und
W'' mit Ausnahme der Carboxy-, Carboxy-methyl-, 2-Carboxyäthyl-, 2-Carboxy-äthenyl- oder 2,2-Bis-(carboxy)-äthylgruppe die für W in den Ansprüchen 1 bis 6 erwähnten Bedeutungen besitzt, mit einem Amin der allgemeinen Formel

$$H—R_6 \quad , \quad (X)$$

in der
$R_6$ eine Alkyleniminogruppe mit 4 bis 6 Kohlenstoffatomen oder eine gegebenenfalls durch Alkyl- oder Phenylalkylgruppen mit jeweils 1 bis 3 Kohlenstoffatomen im Alkylteil mono- oder disubstituierte Aminogruppe darstellt, umgesetzt wird, oder
g) zur Herstellung von Verbindungen der allgemeinen Formel I, in der W die Carboxygruppe darstellt, eine Verbindung der allgemeinen Formel

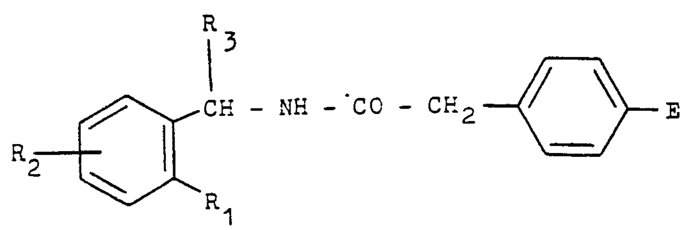
, (XI)

in der
$R_1$, $R_2$ und $R_3$ wie in den Ansprüchen 1 bis 6 definiert sind und
E eine durch Oxidation in eine Carboxygruppe überführbare Gruppe darstellt, oxidiert wird, oder
h) zur Herstellung von Verbindungen der allgemeinen Formel I, in der W eine Alkoxycarbonylgruppe mit insgesamt 2 bis 5 Kohlenstoffatomen darstellt, in welcher der Alkylteil ab dem β-Kohlenstoffatom durch eine oder zwei Hydroxygruppen oder durch eine Alkoxygruppe mit 1 bis 3 Kohlenstoffatomen substituiert sein kann, eine Carbonsäure der allgemeinen Formel

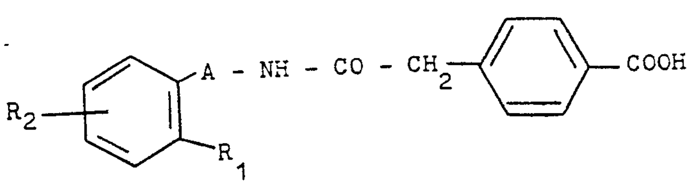
, (XII)

in der
$R_1$, $R_2$ und a wie in den Ansprüchen 1 bis 6 definiert sind, oder deren gegebenenfalls im Reaktionsgemisch hergestellte Derivate mit einem Alkohol der allgemeinen Formel

$$HO—R_7 \quad , \quad (XIII)$$

in der
$R_7$ eine Alkylgruppe mit 1 bis 4 Kohlenstoffatomen darstellt, welche ab dem β-Kohlenstoffatom durch eine oder zwei Hydroxygruppen oder eine Alkoxygruppe mit 1 bis 3 Kohlenstoffatomen substituiert sein kann, verestert wird, oder

i) zur Herstellung einer Verbindung der allgemeinen Formel I, in der W eine Alkoxycarbonyl-, alkoxycarbonyl-methyl-, 2-Alkoxycarbonyl-äthyl- oder 2-Alkoxycarbonyl-äthenylgruppe und A eine Gruppe der Formel

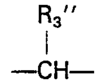 darstellen, wobei

$R_3''$ mit Ausnahme der Cyanogruppe die für in den Ansprüchen 1 bis 6 erwähnten Bedeutungen besitzt, eine Verbindung der allgemeinen Formel

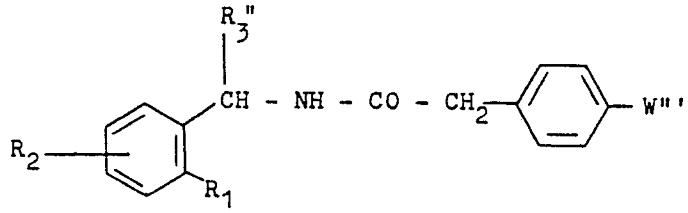 , (XIV)

in der $R_1$ und $R_2$ wie in den Ansprüchen 1 bis 6 definiert sind, $R_3''$ mit Ausnahme der Cyangruppe die für $R_3$ in den Ansprüchen 1 bis 6 erwähnten Bedeutungen besitzt und W''' eine Cyano-, Cyano-methyl-, 2-Cyanoäthyl- oder 2-Cyanoäthenylgruppe darstellt, mittels Alkoholyse in einen entsprechenden Ester übergeführt wird,

und gewünschtenfalls anschließend eine erfindungsgemäß erhaltene Verbindung der allgemeinen Formel I, in der W eine Carboxy- oder Alkoxycarbonylgruppe darstellt, mittels Reduktion in eine entsprechende Verbindung der allgemeinen Formel I, in der W eine Formyl- oder Hydroxymethylgruppe darstellt, übergeführt wird, und/oder

eine erfindungsgemäß erhaltene Verbindung der allgemeinen Formel I, in der W die Carboxygruppe darstellt, mittels Überführung in ein Sulfonsäurehydrazid und anschließende Disproportionierung in eine entsprechende Verbindung der allgemeinen Formel I, in der W die Formylgruppe darstellt, übergeführt wird, und/oder

eine erfindungsgemäß erhaltene Verbindung der allgemeinen Formel I, in der W eine Formylgruppe darstellt, mittels Kondensation und gegebenenfalls anschließender Hydrolyse und/-oder Decarboxylierung in eine entsprechende Verbindung der allgemeinen Formel I, in der W eine 2-Alkoxycarbonyläthenyl- oder eine 2-Carboxy-äthenylgruppe darstellt, übergeführt wird, und/oder

eine erfindungsgemäß erhaltene Verbindung der allgemeinen Formel I, in der W eine 2-Carboxy-äthenyl- oder 2-Alkoxycarbonyl-äthenylgruppe darstellt, mittels katalytischer Hydrierung in eine entsprechende Verbindung der allgemeinen Formel I, in der W eine 2-Carboxy-äthyl- oder 2-Alkoxycarbonyläthylgruppe darstellt, übergeführt wird, und/oder

eine erfindungsgemäß erhaltene Verbindung der allgemeinen Formel I, in der W eine Alkoxycarbonylgruppe darstellt, welche ab dem β-Kohlenstoffatom durch eine Hydroxygruppe substituiert ist, mittels Acylierung durch eine Pyridincarbonsäure in eine entsprechende (Pyridincarbonyloxyalkoxy)carbonyl-Verbindung der allgemeinen Formel I übergeführt wird, und/oder

eine erfindungsgemäß erhaltene Verbindung der allgemeinen Formel I, in der W eine Hydroxymethylgruppe darstellt, durch Überführung in eine entsprechende Halogenmethylverbindung und nachfolgende Umsetzung mit einem Malonsäurediester in eine entsprechende Verbindung der allgemeinen Formel I, in der W eine durch zwei Alkoxycarbonylgruppen substituierte Äthylgruppe darstellt, übergeführt wird, und/oder

eine erfindungsgemäß erhaltene Verbindung der allgemeinen Formel I, in der W eine durch zwei Alkoxycarbonylgruppen substituierte Äthylgruppe darstellt, mittels Hydrolyse in eine entsprechende Verbindung der allgemeinen Formel I, in der W eine durch zwei Carboxygruppen substituierte Äthylgruppe darstellt, übergeführt wird, und/oder

eine erfindungsgemäß erhaltene Verbindung der allgemeinen Formel I, in der W eine durch zwei Alkoxycarbonylgruppen substituierte Äthylgruppe darstellt, mittels Hydrolyse und Decarboxylierung in eine entsprechende Verbindung der allgemeinen Formel I, in der W eine 2-Carboxy-äthylgruppe darstellt, übergeführt wird, und/oder

eine erfindungsgemäß erhaltene Verbindung der allgemeinen Formel I, in der $R_2$ eine Nitrogruppe darstellt, mittels Reduktion in eine entsprechende Verbindung der allgemeinen Formel I, in der $R_2$ eine Aminogruppe darstellt, übergeführt wird, und/oder

eine erfindungsgemäß erhaltene Verbindung der allgemeinen Formel I, in der $R_2$ eine Aminogruppe darstellt, über ein entsprechende Diazoniumsalz in eine entsprechende Verbindung der allgemeinen Formel I,

in der $R_2$ eine Wasserstoff-, Fluor-, Chlor- oder Bromatom, eine Hydroxy-, Alkoxy- oder Alkylsulfenylgruppe darstellt, übergefühet wird, und/oder
eine erfindungsgemäß erhaltene Verbindung der allgemeinen Formel I, in der $R_2$ einen benzyloxyrest und/oder $R_3$ einen durch einen Benzyloxyrest substituierten Arylrest darstellt, mittels Entbenzylierung in eine entsprechende Verbindung der allgemeinen Formel I, in der $R_2$ die Hydroxygruppe und/oder $R_3$ einen durch eine Hydroxygruppe substituierten Arylrest darstellt, übergeführt wird, und/oder
eine erfindungsgemäß erhaltene Verbindung der allgemeinen Formel I, in der $R_3$ eine Aminocarbvonylgruppe darstellt, mittels Dehydratisierung in eine entsprechende Verbindung der allgemeinen Formel I, in der $R_3$ eine Cyanogruppe darstellt, übergeführt wird, und/oder
eine erfindungsgemäß erhaltene Verbindung der allgemeinen Formel I, sofern sie ein chirales Zentrum enthält, mittels Chromatographie an chiralen Phasen in ihre Enantiomeren aufgetrennt wird, und/oder
eine erfindungsgemäß erhaltene Verbindung der allgemeinen Formel I in ihre Salze, insbesondere in ihre physiologisch verträglichen Salz mit anorganischen oder organischen Säuren oder Basen übergeführt wird.

**Revendications**

1. Dérivés de l'acide phénylacétique de formule générale

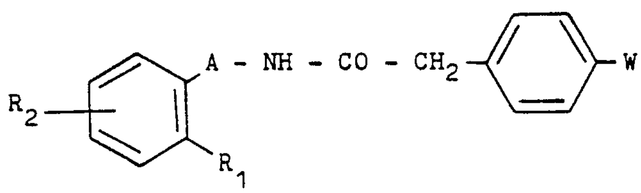 , (I)

dans laquelle
A représente un groupe de formule

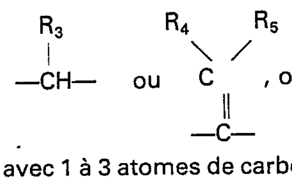 , où

$R_3$ représente un groupe alcoyle avec 1 à 3 atomes de carbone, substitué par un groupe alcoxy avec 1 à 3 atomes de carbone ou par un groupe phényle, ou représente un groupe n-propyle, un groupe alcoyle avec 4 à 6 atomes de carbone, un groupe alcényle avec 3 à 5 atomes de carbone, un groupe cyano ou alcoylène-iminocarbonyle avec 4 à 6 atomes de carbone dans la partie alcoylène, un groupe aminocarbonyle éventuellement mono- ou di-substitué par des groupes alcoyle ou phénylalcoyle avec chacun 1 à 3 atomes de carbone dans la partie alcoyle, ou représente un groupe aryle avec 6 ou 10 atomes de carbone, mono- ou di-substitué par des atomes d'halogène, des groupes alcoyle, hydroxy, alcoxy, phénylalcoxy, alcoylsulfényle, alcoylsulfinyle et/ou alcoylsulfonyle, les substituants pouvant être identiques ou différents et la partie alcoyle pouvant contenir dans chaque cas 1 à 3 atomes de carbone, ou représente un groupe naphtyle ou un groupe hétéroaryle avec 4, 5, 8 ou 9 atomes de carbone, contenant 1 ou 2 atomes d'azote,
ou également un groupe méthyle,
lorsque $R_1$ représente un groupe pipéridino ainsi que $R_2$ en position 4 un atome de fluor et W un groupe carboxy- ou alcoxycarbonyle dans lequel la partie alcoyle peut contenir 1 à 3 atomes de carbone,
ou également un groupe phényle,
lorsque $R_1$ représente un groupe pipéridino substitué en position 2 ou 3 par un groupe méthyle, $R_2$ représente un atome d'hydrogène et W représente un groupe carboxy ou alcoxycarbonyle, dans lequel la partie alcoyle peut contenir 1 à 3 atomes de carbone, ou
lorsque $R_1$ représente un groupe pipéridino, $R_2$ en position 3, 4 ou 6 représente un atome de chlore ou en position 4 ou 6 représente un groupe méthyle et W représente un groupe carboxy ou alcoxycarbonyle dans lequel la partie alcoyle peut contenir 1 à 3 atomes de carbone, ou lorsque $R_1$ représente un groupe pipéridino, $R_2$ représente un atome d'hydrogène et W représente un groupe formyle, un groupe 2-carboxyéthényle ou 2-alcoxycarbonyléthényle, dans lequel la partie alcoyle peut contenir 1 à 3 atomes de carbone,
$R_4$ et $R_5$, ensemble avec l'atome de carbone intermédiaire, représentent un groupe alcoylidène avec 3 à 9 atomes de carbone ou un groupe phénylalcoylidène avec 1 à 3 atomes de carbone dans la partie alcoylidène,
$R_1$ représente un groupe alcoylènimino avec 4 à 8 atomes de carbone, non ramifié, éventuellement mono- ou di-substitué par des groupes alcoyle avec 1 à 3 atomes de carbone,
$R_2$ représente un atome d'hydrogène, de fluor, de chlore ou de brome, un groupe hydroxy, trifluorométhyle, nitro, amino, pipéridino, alcoyle, alcoxy, alcoylsulfényle, alcoylsulfinyle, alcoylsulfonyle,

phénylalcoxy, alcanoyloxy, alcanoylamino, alcoylamino ou dialcoylamino, la partie alcoyle pouvant dans chaque cas contenir 1 à 3 atomes de carbone, et

W représente un groupe carboxy ou un groupe alcoxycarbonyle avec en tout 2 à 5 atomes de carbone, dans lequel la partie alcoyle peut être substituée par un groupe phényle et à partir de l'atome de carbone β par un ou deux groupes hydroxy, par un groupe alcoxy, alcanoyloxy, dialcoylamino, alcoylènimino ou pyridinecarbonyloxy, chaque partie alcoyle pouvant dans chaque cas contenir 1 à 3 atomes de carbone et le groupe alcoylènimino 4 à 6 atomes de carbone, un groupe alcényloxycarbonyle avec en tout 4 à 6 atomes de carbone, un groupe alcoyle avec 1 à 3 atomes de carbone, un groupe hydroxyméthyle, formyle, cyano, aminocarbonyl, carboxyméthyle, 2-carboxyéthyle, 2-carboxyéthènyle, 2,2-bis-(carboxy)-éthyle, alcoxycarbonyl-méthyle, 2-alcoxycarbonyl-éthyle, 2-alcoxycarbonyléthènyle, ou 2,2-bis-(alcoxycarbonyl)-éthyle, le groupe alcoxy pouvant dans chaque cas contenir 1 à 3 atomes de carbone,

ainsi que leurs antipodes optiquement actifs et leurs sels avec des acides ou des bases minéraux ou organiques.

2. Dérivés de l'acide phénylacétique de formule générale I selon la revendication 1, dans laquelle $R_3$ représente un groupe alcoyle avec 1 à 3 atomes de carbone, substitué par un groupe alcoxy avec 1 à 3 atomes de carbone ou par un groupe phényle, ou représente un groupe n-propyle, un groupe alcoyle avec 4 à 6 atomes de carbone, un groupe alcényle avec 3 à 5 atomes de carbone, un groupe cyano ou aminocarbonyle, un groupe phényle substitué par un atome d'halogène, par un groupe alcoyle, hydroxy, alcoxy, phénylalcoxy, alcoylsulfényle, alcoylsulfinyle ou alcoylsulfonyle, la partie alcoyle pouvant contenir à chaque fois 1 à 3 atomes de carbone, un groupe naphtyle, pyridyle, quinoléyle, ou isoquinoléyle,

$R_4$ et $R_5$, ensemble avec l'atome de carbone intermédiaire, représentent un groupe alcoylidène avec 3 à 5 atomes de carbone ou un groupe phénylalcoylidène avec 1 à 3 atomes de carbone dans la partie alcoylidène,

$R_1$ représente un groupe alcoylènimino non ramifié avec 4 à 8 atomes de carbone ou un groupe pipéridino mono- ou di-substitué par des groupes alcoyle avec 1 à 3 atomes de carbone,

$R_2$ représente un atome d'hydrogène, de fluor, de chlore ou de brome, un groupe nitro, alcoyle, ou alcoxy avec dans chaque cas 1 à 3 atomes de carbone,

W représente un groupe carboxy, hydroxyméthyle, carboxyméthyle, 2-carboxyéthyle, 2-hydroxy-éthoxycarbonyle, 2-méthoxy-éthoxycarbonyle, 2-nicotinoyloxy-éthoxycarbonyle, 2,3-dihydroxy-n-propoxycarbonyle ou alcoxycarbonyle avec en tout 2 à 5 atomes de carbone, un groupe formyle, 2-carboxyéthènyle, 2,2-bis-(carboxy)-éthyle, 2-alcoxycarbonyl-éthènyle, 2-alcoxycarbonyléthyle, alcoxy-carbonylméthyle ou 2,2-bis-(alcoxycarbonyl)-éthyle, le groupe alcoxy pouvant à chaque fois contenir 1 à 3 atomes de carbone,

ainsi que leurs antipodes optiquement actifs et leurs sels avec des acides ou des bases minéraux ou organiques.

3. Dérivés de l'acide phénylacétique de formule générale I selon la revendication 1, dans laquelle,

$R_3$ représente un groupe n-propyle, un groupe alcoyle avec 4 ou 5 atomes de carbone, un groupe phényle substitué par un groupe méthyle, par un atome de fluor ou de chlore, ou représente un groupe pyridyle,

$R_4$ et $R_5$, ensemble avec l'atome de carbone intermédiaire, représentent un groupe alcoylidène avec 3 à 5 atomes de carbone ou un groupe phénylalcoylidène avec 1 à 3 atomes de carbone dans la partie alcoylidène,

$R_1$ représente un groupe pipéridino éventuellement substitué par un ou deux groupes méthyle,

$R_2$ représente un atome d'hydrogène, de fluor ou de chlore, un groupe méthyle ou méthoxy, et

W représente un groupe carboxy ou un groupe alcoxycarbonyle avec en tout 2 à 4 atomes de carbone,

ainsi que leurs antipodes optiquement actifs et leurs sels avec des acides ou des bases minéraux ou organiques.

4. Dérivés de l'acide phénylacétique de formule générale I selon la revendication 1, dans laquelle $R_1$, $R_2$ et W sont définis comme dans la revendication 3 et

$R_3$ représente un groupe n-propyle ou un groupe alcoyle avec 4 ou 5 atomes de carbone et

$R_4$ et $R_5$, ensemble avec l'atome de carbone intermédiaire, représentent un groupe alcoylidène avec 3 à 5 atomes de carbone, ou un groupe phénylalcoylidène avec 1 à 3 atomes de carbone dans la partie alcoylidène,

ainsi que leurs antipodes optiquement actifs et leurs sels avec des acides ou des bases minéraux ou organiques.

5. L'acide 4-[(1-(2-pipéridinophényl)-1-butyl)-aminocarbonylméthyl]-benzoïque, ses esters d'alcoyles avec 1 à 3 atomes de carbone, ses antipodes optiquement actifs et ses sels.

6. L'acide 4-[(1-(2-pipéridinophényl)-1-pentyl)-aminocarbonylméthyl]-benzoïque, ses esters d'alcoyles avec 1 à 3 atomes de carbone, ses antipodes optiquement actifs et ses sels.

7. Sels physiologiquement supportables des composés selon les revendications 1 à 6 avec des acides minéraux ou organiques et également des bases, dans la mesure où les composés selon l'invention contiennent un groupe carboxy.

8. Médicament contenant un composé selon les revendications 1 à 6 ou un sel physiologiquement supportable de celui-ci selon la revendication 7, conjointement à un ou plusieurs excipients et/ou diluants inertes.

9. Procédé pour la préparation d'un médicament selon la revendication 8, caractérisé en ce qu'on introduit, par voie non chimique, un composé selon les revendications 1 à 7, dans un ou plusieurs excipients et/ou diluants inertes.

10. Utilisation des composés selon les revendications 1 à 7 pour la préparation de médicaments présentant des propriétés hypoglycémiantes.

11. Procédé pour la préparation des dérivés de l'acide phénylacétique selon les revendications 1 à 7, caractérisé en ce que

a) on fait réagir un amine de formule générale

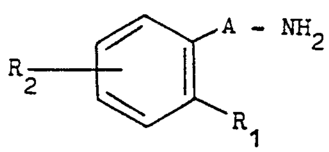

, (II)

dans laquelle

A, $R_1$ et $R_2$ sont définis comme dans les revendications 1 à 6, ou lorsque A représente l'un des groupes vinylidène mentionnés au début, ses tautomères, son complexe d'halogénure de lithium ou de magnésium,

avec un acide carboxylique de formule générale

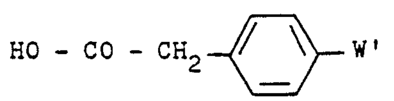

, (III)

dans laquelle

W' possède les significations mentionnées pour W dans les revendications 1 à 6, ou représente un groupe carboxy protégé par un radical protecteur, ou avec ses dérivés réactifs éventuellement préparés dans le mélange réactionnel et, si cela est nécessaire, on clive le radical protecteur utilisé, ou

b) pour la préparation d'un composé de formule générale I, dans laquelle W représente un groupe carboxy, carboxyméthyle, 2-carboxyéthyle ou 2-carboxyéthènyle, on transforme un composé de formule générale

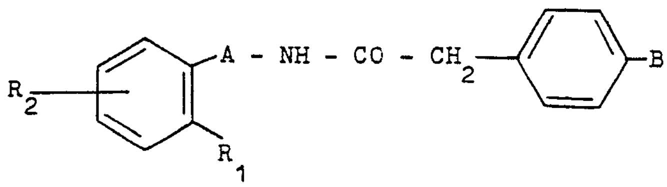

, (IV)

dans laquelle

$R_1$, $R_2$ et A sont définis comme dans les revendications 1 à 6 et

B représente un groupe transformable en un groupe carboxy, carboxyméthyle, 2-carboxyéthyle ou 2-carboxyéthènyle,

en le composé carboxy correspondant au moyen d'une hydrolyse, d'une thermolyse ou d'une hydrogénolyse, ou

c) pour la préparation d'un composé de formule générale I dans laquelle A représente un groupe de formule

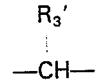

, où

$R_3'$ possède les significations mentionnées dans les revendications 1 à 6 pour $R_3$, à l'exception du groupe alcényle et du groupe cyano,

on réduit un composé de formule générale

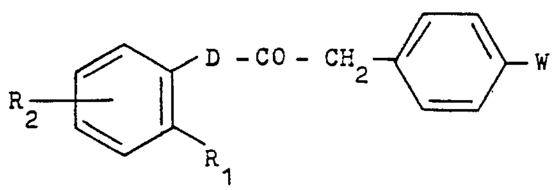

, (V)

dans laquelle

$R_1$, $R_2$ et W sont définis comme dans les revendications 1 à 6 et

D représente un groupe de formule

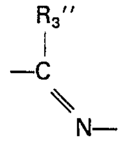

ou

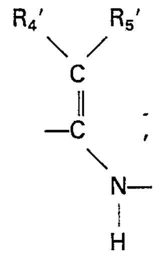

,

$R_3''$ possédant les significations mentionnées au début pour $R_3$, à l'exception du groupe cyano et $R_4'$ et $R_5'$, ensemble avec l'atome de carbone intermédiaire, représentant un groupe propylidène, un groupe alcoylidène avec 4 à 6 atomes de carbone ou un groupe phénylalcoylidène avec 1 à 3 atomes de carbone dans la partie alcoylidène,

au moyen d'hydrogène en présence d'un catalyseur d'hydrogénation, ou

d) pour la préparation de composés de formule générale I dans laquelle $R_3$ possède les significations mentionnées dans les revendications 1 à 6 pour $R_3$, à l'exception du groupe cyano, on fait réagir un composé de formule générale

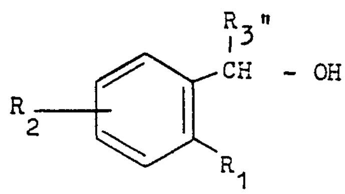

, (VI)

dans laquelle

$R_3''$ possède les significations mentionnées plus haut pour $R_3$ de même que $R_1$ et $R_2$ sont définis comme dans les revendications 1 à 6, avec un composé de formule générale

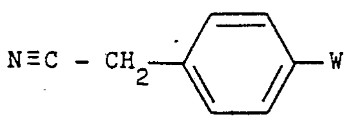

, (VII)

dans laquelle

W est défini comme dans les revendications 1 à 6, ou

e) pour la préparation de composés de formule générale I dans laquelle $R_2$ représente un atome d'hydrogène et A un groupe de formule

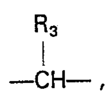

, où

$R_3$ est défini comme dans les revendications 1 à 6, on déshalogène un composé de formule générale

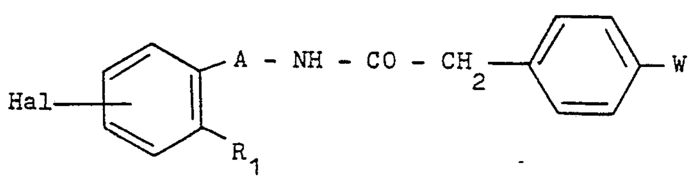

, (VIII)

dans laquelle

$R_1$, A et W sont définis comme dans les revendications 1 à 6 et

Hal représente un atome de fluor, de chlore, de brome ou d'iode, ou

f) pour la préparation de composés de formule générale I dans laquelle A représente un groupe de formule

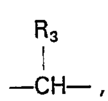

, où

$R_3$ représente un groupe alcoylèniminocarbonyle avec 4 à 6 atomes de carbone dans le cycle alcoylène

ou un groupe aminocarbonyle éventuellement mono- ou di-substitué par des groupes alcoyle ou phénylalcoyle avec chacun 1 à 3 atomes de carbone dans la partie alcoyle,

on fait réagir un composé de formule générale

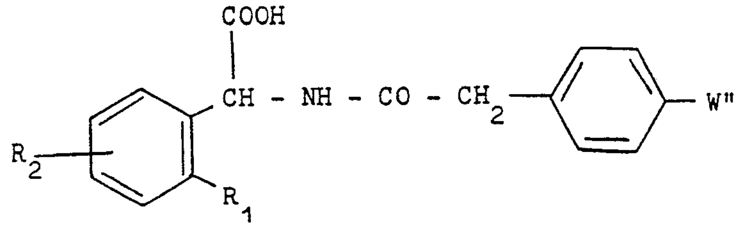 , (IX)

dans laquelle

$R_1$ et $R_2$ sont définis comme dans les revendications 1 à 6 et

W'' possède les significations données pour W dans les revendications 1 à 6, à l'exception du groupe carboxy, carboxy-méthyle, 2-carboxyéthyle, 2-carboxyéthènyle ou 2,2-bis-(carboxy)-éthyle,

avec une amine de formule générale

$$H—R_6 , \qquad (X)$$

dans laquelle

$R_6$ représente un groupe alcoylènimino avec 4 à 6 atomes de carbone ou un groupe amino éventuellement mono- ou di-substitué par des groupes alcoyle ou phénylalcoyle avec chacun 1 à 3 atomes de carbone dans la partie alcoyle, ou

g) pour la préparation de composés de formule générale I dans laquelle W représente le groupe carboxy,

on oxyde un composé de formule générale

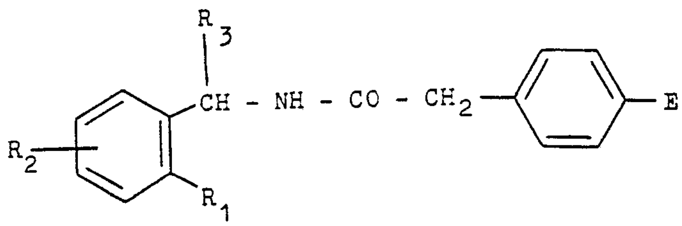 , (XI)

dans laquelle

$R_1$, $R_2$ et $R_3$ sont définis comme dans les revendications 1 à 6 et

E représente un groupe transformable par oxydation en un groupe carboxy, ou

h) pour la préparation de composés de formule générale I dans laquelle W représente un groupe alcoxycarbonyle avec en tout 2 à 5 atomes de carbone, dans lequel la partie alcoyle peut être substituée, à partir de l'atome de carbone β, par un ou deux groupes hydroxy ou par un groupe alcoxy avec 1 à 3 atomes de carbone,

on estérifie un acide carboxylique de formule générale

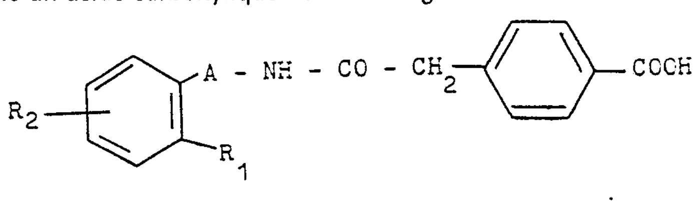 , (XII)

dans laquelle

$R_1$, $R_2$ et A sont définis comme dans les revendiations 1 à 6, ou ses dérivés éventuellement préparés dans le mélange réactionnel,

au moyen d'un alcool de formule générale

$$HO—R_7 , \qquad (XIII)$$

dans laquelle

$R_7$ représente un groupe alcoyle avec 1 à 4 atomes de carbone, lequel, à partir de l'atome de carbone β, peut être substitué par un ou deux groupes hydroxy ou par un groupe alcoxy avec 1 à 3 atomes de carbone, ou

i) pour la préparation d'un composé de formule générale I dans laquelle W représente un groupe alcoxycarbonyle, alcoxycarbonyle-méthyle, 2-alcoxycarbonyl-éthyle ou 2-alcoxycarbonyl-éthènyle et A représente un groupe de formule

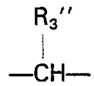 , où

$R_3''$ possède les significations, mentionnées pour $R_3$ dans les revendications 1 à 6, à l'exception du groupe cyano,

on transforme au moyen d'une alcoolyse, en un ester correspondant, un composé de formule générale

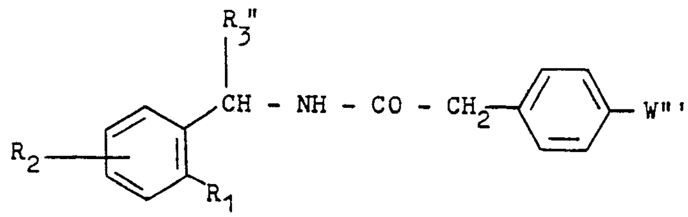 , (XIV)

dans laquelle

$R_1$ et $R_2$ sont définis comme dans les revendications 1 à 6,

$R_3''$ possède les significations mentionnées pour $R_3$ dans les revendications 1 à 6, à l'exception du groupe cyano, et

W''' représente un groupe cyano, cyano-méthyle, 2-cyanoéthyle ou 2-cyanoéthènyle,

et éventuellement ensuite on transforme un composé de formule générale I obtenu selon l'invention, dans lequel W représente un groupe carboxy ou alcoxycarbonyle, au moyen d'une réduction en un composé correspondant de formule générale I dans laquelle W représente un groupe formyle ou hydroxyméthyle, et/ou

on transforme un composé obtenu selon l'invention, de formule générale I dans laquelle W représente le groupe carboxy, au moyen d'une transformation en un hydrazide d'acide sulfonique avec dismutation subséquente, en un composé correspondant de formule générale I dans laquelle W représente le groupe formyle, et/ou

on transforme un composé selon l'invention, de formule générale I dans laquelle W représente un groupe formyle, au moyen d'une condensation et éventuellement d'une hydrolyse et/ou d'une décarboxylation subséquentes en un composé correspondant de formule générale I dans laquelle W représente un groupe 2-alcoxycarbonyléthènyle ou un groupe 2-carboxyéthènyle, et/ou

on transforme un composé obtenu selon l'invention, de formule générale I dans laquelle W représente un groupe 2-carboxyéthènyle ou 2-alcoxycarbonyléthènyle, au moyen d'une hydrogénation catalytique, en un composé correspondant de formule générale I dans laquelle W représente un groupe 2-carboxyéthyle ou 2-alcoxycarbonyléthyle, et/ou

on transforme un composé obtenu selon l'invention, de formule générale I dans laquelle W représente un groupe alcoxycarbonyle, lequel à partir de l'atome de carbone β est substitué par un groupe hydroxy, au moyen d'une acylation par un acide pyridinecarboxylique, en un composé (pyridinecarbonyloxyalcoxy)-carbonylé correspondant de formule générale I, et/ou

on transforme un composé obtenu selon l'invention, de formule générale I dans laquelle W représente un groupe hydroxyméthyle, par transformation en un composé halogénométhylé correspondant et réaction subséquente avec un diester malonique, en un composé correspondant de formule générale I dans laquelle W représente un groupe éthyle substitué par deux groupes alcoxycarbonyle, et/ou

on transforme un composé obtenu selon l'invention, de formule générale I dans laquelle W représente un groupe éthyle substitué par deux groupes alcoxycarbonyle, au moyen d'une hydrolyse dans un composé correspondant de formule générale I, dans laquelle W représente un groupe éthyle substitué par deux groupes carboxy, et/ou

on transforme un composé obtenu selon l'invention, de formule générale I dans laquelle W représente un groupe éthyle substitué par deux groupes alcoxycarbonyle, au moyen d'une hydrolyse et d'une décarboxylation, en un composé correspondant de formule générale I dans laquelle W représente un groupe 2-carboxy-éthyle, et/ou

on transforme un composé obtenu selon l'invention, de formule générale I dans laquelle $R_2$ représente un groupe nitro, au moyen d'une réduction, en un composś correspondant de formule générale I dans laquelle $R_2$ représente un groupe amino, et/ou

on transforme un composé obtenu selon l'invention, de formule générale I dans laquelle $R_2$ représente un groupe amino, par l'intermédiaire d'un sel de diazonium correspondant, en un composé correspondant de formule générale I dans laquelle $R_2$ représente un atome d'hydrogène, de fluor, de chlore ou de brome, un groupe hydroxy, alcoxy ou alcoylsulfényle, et/ou

on transforme un composé obtenu selon l'invention, de formule générale I dans laquelle $R_2$ représente un radical benzyloxy et/ou $R_3$ représente un radical aryle substitué par un radical benzyloxy, au moyen d'une débenzylation en un composé correspondant de formule générale I dans laquelle $R_2$ représente le groupe hydroxy et/ou $R_3$ représente un radical aryle substitué par un groupe hydroxy, et/ou

on transforme un composé obtenu selon l'invention, de formule générale I dans laquelle $R_3$ représente

un groupe aminocarbonyle, au moyen d'une déshydratation en un composé correspondant de formule générale I dans laquelle $R_3$ représente un groupe cyano, et/ou

on sépare un composé obtenu selon l'invention, de formule générale I, en ses énantiomères au moyen d'une chromatographie sur des phases chirales, dans la mesure où il contient un centre chirale, et/ou

on transforme un còmposé obtenu selon l'invention, de formule générale I en ses sels, en particulier en ses sels physiologiquement supportables avec des acides ou des bases minéraux ou organiques.

**Claims**

1. Phenylacetic acid derivatives of general formula

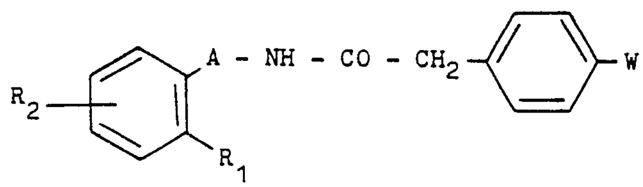 , (I)

wherein

A represents a group of formula

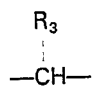 or 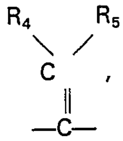 ,

wherein

$R_3$ represents an alkyl group with 1 to 3 carbon atoms substituted by an alkoxy group with 1 to 3 carbon atoms or by a phenyl group; and n-propyl group; an alkyl group with 4 to 6 carbon atoms, an alkenyl group with 3 to 5 carbon atoms, a cyano or alkyleneiminocarbonyl group with 4 to 6 carbon atoms in the alkylene moiety, an aminocarbonyl group optionally mono- or disubstituted by alkyl or phenylalkyl groups each having 1 to 3 carbon atoms in the alkyl moiety, an aryl group with 6 to 10 carbon atoms mono- or disubstituted by halogen atoms, or by alkyl, hydroxy, alkoxy, phenylalkoxy, alkylsulphenyl, alkylsulphinyl and/or alkylsulphonyl groups, whilst the substituents may be the same or different and the alkyl moiety may contain 1 to 3 carbon atoms in each case, or a heteroaryl group with 4, 5, 8 or 9 carbon atoms containing 1 or 2 nitrogen atoms,

or a methyl group,

if $R_1$ represents a piperidino group and $R_2$ in the 4 position represents a fluorine atom and W represents a carboxy or alkoxycarbonyl group in which the alkyl moiety may contain 1 to 3 carbon atoms,

or a phenyl group,

if $R_1$ represents a piperidino group substituted by a methyl group in the 2 or 3 position, $R_2$ represents a hydrogen atom and W represents a carboxy or alkoxycarbonyl group in which the alkyl moiety may contain 1 to 3 carbon atoms, or

if $R_1$ represents a piperidino group, $R_2$ in the 3, 4 or 6 position represents a chlorine atom or in the 4 or 6 position represents a methyl group and W represents a carboxy or alkoxycarbonyl group in which the alkyl moiety may contain 1 to 3 carbon atoms, or

if $R_1$ represents a piperidino group, $R_2$ represents a hydrogen atom and W represents a formyl group, a 2-carbonylethenyl or 2-alkoxycarbonylethenyl group in which the alkyl moiety may contain 1 to 3 carbon atoms,

$R_4$ and $R_5$ together with the carbon atom between them represent an alkylidene group with 3 to 9 carbon atoms or a phenylalkylidene group with 1 to 3 carbon atoms in the alkylidene moiety,

$R_1$ represents an unbranched alkyleneimino group with 4 to 8 carbon atoms optionally mono- or disubstituted by alkyl groups with 1 to 3 carbon atoms,

$R_2$ represents a hydrogen, fluorine, chlorine or bromine atom, or a hydroxy, trifluoromethyl, nitro, amino, piperidino, alkyl, alkoxy, alkulsulphenyl, alkylsulphinyl, alkylsulphonyl, phenylalkoxy, alkanoyloxy, alkanoylamino, alkylamino or dialkylamino group wherein the alkyl component may contain 1 to 3 carbon atoms in each case,

W represents a carboxy group or an alkoxycarbonyl group with a tótal of 2 to 5 carbon atoms wherein the alkyl component may be substituted by a phenyl group and, from the β-carbon atom, by 1 or 2 hydroxy groups or by an alkoxy, alkanoyloxy, dialkylamino, alkyleneimino or pyridinecarbonyloxy group, whilst each alkyl component may contain 1 to 3 carbon atoms and the alkyleneimino group may contain 4 to 6 carbon atoms, an alkenyloxycarbonyl group with a total of 4 to 6 carbon atoms, an alkyl group with 1 to 3 carbon atoms, a hydroxymethyl, formyl, cyano, aminocarbonyl, carboxymethyl, 2-carboxyethyl, 2-carboxy-ethenyl, 2,2-bis-(carboxy)-ethyl, alkoxycarbonylmethyl, 2-alkoxycarbonyl-ethyl, 2-alkoxycarbonylethenyl or 2,2-bis-(alkoxycarbonyl)-ethyl group, whilst the alkoxy group may contain from 1 to 3 carbon atoms in each case,

and the optically active enantiomers thereof and the salts thereof with inorganic acids or bases.

2. Phenylacetic acid derivatives of general formula I as claimed in claim 1, wherein

$R_3$ represents an alkyl group with 1 to 3 carbon atoms substituted by an alkoxy group with 1 to 3 carbon atoms or by a phenyl group, an n-propyl group, an alkyl group with 4 to 6 carbon atoms, an alkenyl group with 3 to 5 carbon atoms, a cyano or aminocarbonyl group, a phenyl group substituted by a halogen atom, or by an alkyl, hydroxy, alkoxy, phenylalkoxy, alkylsulphenyl, alkylsulphinyl or alkylsulphonyl group, whilst the alkyl component may contain from 1 to 3 carbon atoms, or a naphthyl, pyridyl, quinolyl or isoquinolyl group,

$R_4$ and $R_5$ together with the carbon atom between them represent an alkylidene group with 3 to 5 carbon atoms or a phenylalkylidene group with 1 to 3 carbon atoms in the alkylidene moiety,

$R_1$ represents an unbranched alkyleneimino group with 4 to 8 carbon atoms or a piperidino group mono- or disubstituted by alkyl groups each having 1 to 3 carbon atoms,

$R_2$ represents a hydrogen, fluorine, chlorine or bromine atom or a nitro, alkyl or alkoxy group each having 1 to 3 carbon atoms,

W represents a carboxy, hydroxymethyl, carboxymethyl, 2-carboxy-ethyl, 2-hydroxy-ethoxycarbonyl, 2-methoxyethoxycarbonyl, 2-nicotinoyloxy-ethoxycarbonyl, 2,3-dihdyroxy-n-propoxycarbonyl or alkoxycarbonyl group with a total of 2 to 5 carbon atoms, a formyl, 2-carboxy-ethenyl, 2,2-bis(carboxy)-ethyl, 2-alkoxycarbonyl-ethenyl, 2-alkoxycarbonyl-ethyl, alkoxycarbonylmethyl or 2,2-bis(alkoxycarbonyl)-ethyl group, whilst the alkoxy group may contain from 1 to 3 carbon atoms in each case, and

the optically active enantiomers thereof and the salts thereof with inorganic or organic acids or bases.

3. Phenylacetic acid derivatives of general formula I as claimed in claim 1, wherein

$R_3$ represents an n-propyl group, an alkyl group with 4 or 5 carbonyl atoms, a phenyl group substituted by a methyl group or by a fluorine or chlorine atom, or a pyridyl group,

$R_4$ and $R_5$ together with the carbon atom between them represent an alkylidene group with 3 to 5 carbon atoms or a phenylalkylidene group with 1 to 3 carbon atoms in the alkylidene part,

$R_1$ represents a piperidino group optionally substituted by one or two methyl groups,

$R_2$ represents a hydrogen, fluorine or chlorine atom or a methyl or methoxy group, and

W represents a carboxy group or an alkoxycarbonyl group with a total of 2 to 4 carbon atoms, the optically active enantiomers thereof and the salts thereof with inorganic or organic acids or bases.

4. Phenylacetic acid derivatives of general formula I as claimed in claim 1,

wherein $R_1$, $R_2$ and W are defined as in claim 3 and

$R_3$ represents an n-propyl group or an alkyl group with 4 or 5 carbon atoms and

$R_4$ and $R_5$ together with the carbon atom between them represent an alkylidene group with 3 to 5 carbon atoms or a phenylalkylidene group with 1 to 3 carbon atoms in the alkylidene part, the optically active enantiomers and the salts thereof with inorganic or organic acids or bases.

5. 4-[(1-(2-Piperidino-phenyl)-1-butyl)-aminocarbonylmethyl)-benzoic acid, the alkyl esters thereof with 1 to 3 carbon atoms, the optically active enantiomers and the salts thereof.

6. 4-[(1-(2-Piperidino-phenyl)-1-pentyl)-aminocarbonylmethyl]-benzoic acid, the alkyl esters thereof with 1 to 3 carbon atoms, the optically active enantiomers and the salts thereof.

7. Physiologically acceptable salts of the compounds as claimed in claims 1 to 6 with inorganic or organic acids and also bases if the compounds according to the invention contain a carboxy group.

8. Pharmaceutical compositions containing a compound as claimed in claims 1 to 6 or a physiologically acceptable salt thereof as claimed in claim 7 together with one or more inert carriers and/or diluents.

9. Process for preparing a pharmaceutical composition as claimed in claim 8, characterised in that a compound as claimed in claims 1 to 7 is incorporated, by a non-chemical method, in one or more inert carriers and/or diluents.

10. Use of compounds as claimed in claims 1 to 7 for the preparation of pharmaceutical compositions with a hypoglycaemic effect.

11. Process for the preparation of the phenylacetic acid derivatives as claimed in claims 1 to 7, characterised in that

a) an amine of general formula

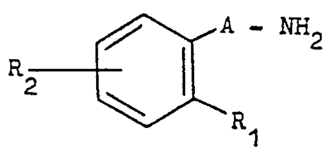

, (II)

wherein

A, $R_1$ and $R_2$ are defined as in claims 1 to 6 or, if A represents one of the vinylidene groups mentioned hereinbefore, the tautomers thereof or a lithium or magnesium halide complex thereof, is reacted with a carboxylic acid of general formula

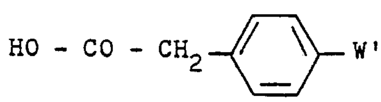

, (III)

wherein

W' has the meanings given for W in claims 1 to 6 or represents a carboxy group protected by a protecting group, or with the reactive derivatives thereof optionally formed in the reaction mixture and, if necessary, any protecting group used is cleaved, or

b) in order to prepare a compound of general formula I wherein W represents a carboxy, carboxymethyl, 2-carboxyethyl or 2-carboxyethenyl group: a group of general formula

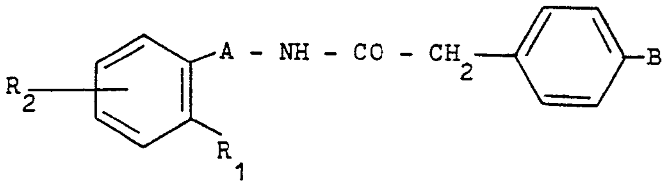 , (IV)

wherein

$R_1$, $R_2$ and A are defined as in claims 1 to 6 and

B represents a group which can be converted into a carboxy, carboxymethyl, 2-carboxyethyl or 2-carboxyethenyl group, is converted by hydrolysis, thermolysis or hydrogenolysis into the corresponding carboxy compound or

c) in order to prepare a compound of general formula I

wherein A represents a group of formula

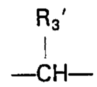

wherein $R_3'$ has the meanings given in claims 1 to 6 for $R_3$, with the exception of the alkenyl group and of the cyano group,

a compound of general formula

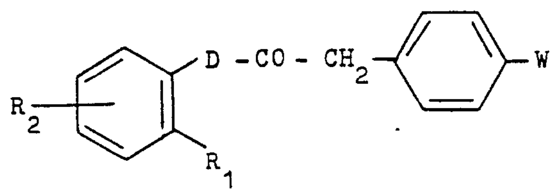 , (V)

wherein

$R_1$, $R_2$ and W are defined as in claims 1 to 6 and

D represents a group of formula

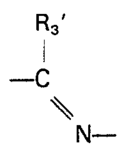 or 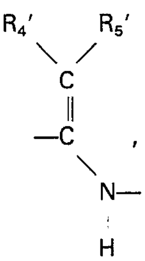 ,

wherein

$R_3''$ has the meanings given hereinbefore for $R_3$, with the exception of the cyano group and $R_4'$ and $R_5'$ together with the carbon atom between them represent a propylidene group, an alkylidene group with 4 to 6 carbon atoms or a phenylalkylidene group with 1 to 3 carbon atoms in the alkylidene moiety,

is reduced with hydrogen in the presence of a hydrogenation catalyst, or

d) in order to prepare compounds of general formula I wherein $R_3'$ has the meanings given in claims 1 to 6 for $R_3$, with the exception of the cyano group:

a compound of general formula

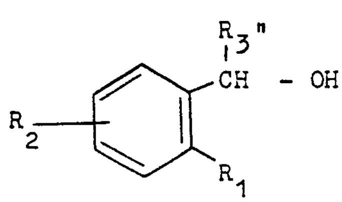 , (VI)

wherein

$R_3$ has the meanings given above for $R_3$ and $R_1$ and $R_2$ are defined as in claims 1 to 6, is reacted with a compound of general formula

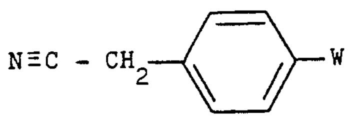 , (VII)

wherein

W is defined as in claims 1 to 6, or

e) in order to prepare compounds of general formula I wherein $R_2$ represents a hydrogen atom and A represents a group of formula wherein

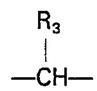

$R_3$ is defined as in claims 1 to 6, a compound of general formula

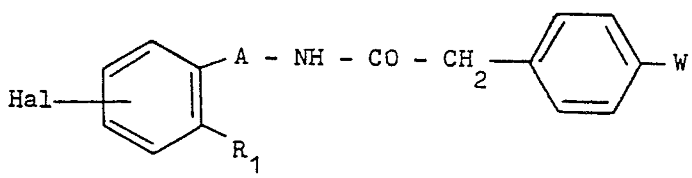 , (VIII)

wherein

$R_1$, A and W are defined as in claims 1 to 6 and

Hal represents a fluorine, chlorine, bromine or iodine atom, is dehalogenated, or

f) in order to prepare compounds of general formula I, wherein A represents a group of formula

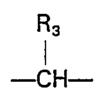

wherein

$R_3$ represents an alkyleneiminocarbonyl group with 4 to 6 carbon atoms in the alkylene ring or an aminocarbonyl group optionally mono- or disubstituted by alkyl or phenylalkyl groups each having 1 to 3 carbon atoms in the alkyl moiety:

a compound of general formula

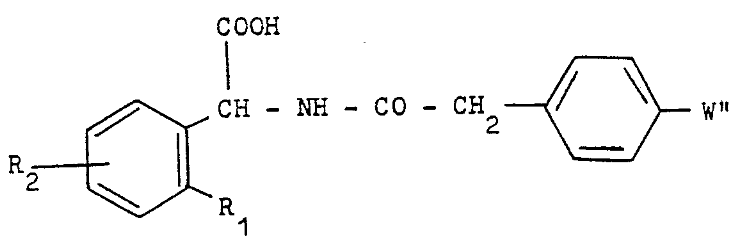 , (IX)

wherein

$R_1$ and $R_2$ are defined as in claims 1 to 6 and

W'' has the meanings given for W in claims 1 to 6, with the exception of the carboxy, carboxymethyl, 2-carboxyethyl, 2-carboxyethenyl or 2,2-bis(carboxy)ethyl group, is reacted with an amine of general formula

$$H—R_6 \qquad (X)$$

wherein

$R_6$ represents an alkyleneimino group with 4 to 6 carbon atoms or an amino group optionally mono- or disubstituted by alkyl or phenylalkyl groups each having 1 to 3 carbon atoms in the alkyl moiety, or

g) in order to prepare compounds of general formula I wherein W represents the carboxy group:

a compound of general formula

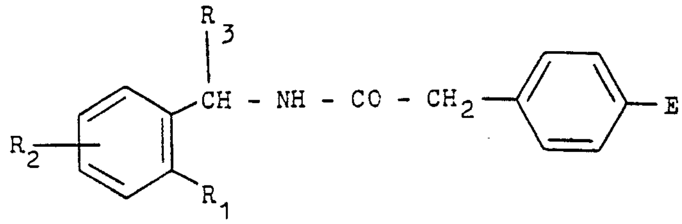

, (XI)

wherein

$R_1$, $R_2$ and $R_3$ are defined as in claims 1 to 6 and

E represents a group which can be converted into a carboxy group by oxidation, is oxidised, or

h) in order to prepare compounds of general formula I wherein W represents an alkoxycarbonyl group with a total of 2 to 5 carbon atoms wherein the alkyl component may be substituted, from the β-carbon atom, by one or two hydroxy groups or by an alkoxy group with 1 to 3 carbon atoms:

a carboxylic acid of general formula

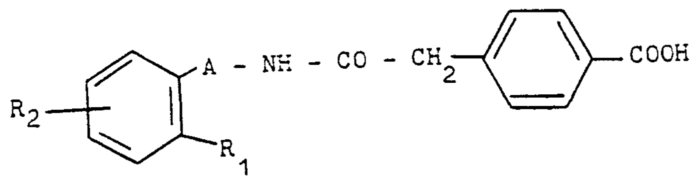

, (XII)

wherein

$R_1$, $R_2$ and A are defined as in claims 1 to 6, or the reactive derivatives thereof optionally prepared in the reaction mixture, is esterified with an alcohol of general formula

$$HO—R_7 \qquad (XIII)$$

wherein

$R_7$ represents an alkyl group with 1 to 4 carbon atoms which may be substituted, from the β-carbon atom, by one or two hydroxy groups or by an alkoxy group with 1 to 3 carbon atoms, or

i) in order to prepare a compound of general formula I wherein W represents an alkoxycarbonyl, alkoxycarbonylmethyl, 2-alkoxycarbonyl-ethyl or 2-alkoxycarbonyl-ethenyl group and

A represents a group of formula

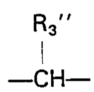

wherein

$R_3''$ has the meanings given for $R_3$ in claims 1 to 6 with the exception of the cyano group, a compound of general formula

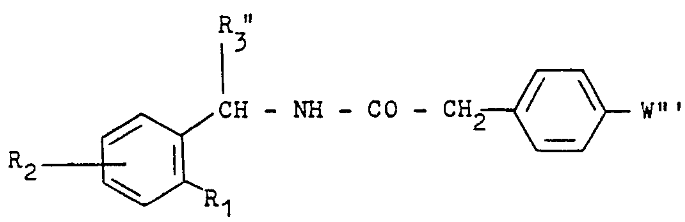

, (XIV)

wherein

$R_1$ and $R_2$ are defined as in claims 1 to 6,

$R_3''$ has the meanings given for $R_3$ in claims 1 to 6 with the exception of the cyano group

and W''' represents a cyano, cyanomethyl, 2-cyanoethyl or 2-cyanoethenyl group, is converted into a corresponding ester by alcoholysis,

and subsequently, if desired, a compound of general formula I obtained according to the invention wherein W represents a carboxy or alkoxycarbonyl group is converted by reduction into a corresponding compound of general formula I wherein W represents a formyl or hydroxymethyl group, and/or

a compound of general formula I obtained according to the invention wherein W represents the carboxy group may be converted by conversion into a sulphonic acid hydrazide and subsequent disproportionation into a corresponding compound of general formula I wherein W represents the formyl group, and/or

a compound of general formula I obtained according to the invention wherein W represents a formyl group may be converted by condensation and optional subsequent hydrolysis and/or decarboxylation into

a corresponding compound of general formula I wherein W represents a 2-alkoxycarbonyl-ethenyl or a 2-carboxy-ethenyl group, and/or

a compound of general formula I obtained according to the invention wherein W represents a 2-carboxyethenyl or 2-alkoxycarbonyl-ethenyl group may be converted by catalytic hydrogenation into a corresponding compound of general formula I wherein W represents a 2-carboxyethyl or 2-alkoxycarbonyl-ethyl group, and/or

a compound of general formula I obtained according to the invention wherein W represents an alkoxycarbonyl group which is substituted from the β-carbon atom by a hydroxy group may be converted by acylation by means of a pyridine-carboxylic acid into a corresponding (pyridine-carbonyloxyalkoxy)-carbonyl compound of general formula I, and/or

a compound of general formula I obtained according to the invention wherein W represents a hydroxymethyl group may, after being converted into a corresponding halomethyl compound, be converted by reaction with a malonic acid diester into a corresponding compound of general formula I wherein W represents an ethyl group substituted by two alkoxycarbonyl groups, and/or

a compound of general formula I obtained according to the invention wherein W represents an ethyl group substituted by two alkoxycarbonyl groups may be converted by hydrolysis into a corresponding compound of general formula I wherein W represents an ethyl group substituted by two carboxy groups, and/or

a compound of general formula I obtained according to the invention wherein W represents an ethyl group substituted by two alkoxycarbonyl groups may be converted by hydrolysis and decarboxylation into a corresponding compound of general formula I wherein W represents a 2-carboxyethyl group, and/or

a compound of general formula I obtained according to the invention wherein $R_2$ represents a nitro group may be converted by reduction into a corresponding compound of general formula I wherein $R_2$ represents an amino group, and/or

a compound of general formula I obtained according to the invention wherein $R_2$ represents an amino group may be converted, via a corresponding diazonium salt, into a corresponding compound of general formula I wherein $R_2$ represents a hydrogen, fluorine, chlorine or bromine atom or a hydroxy, alkoxy or alkylsulphenyl group, and/or

a compound of general formula I obtained according to the invention wherein $R_2$ represents a benzyloxy group and/or $R_3$ represents an aryl group substituted by a benzyloxy group may be converted by debenzylation into a corresponding compound of general formula I wherein $R_2$ represents a hydroxy group and/or $R_3$ represents an aryl group substituted by a hydroxy group, and/or

a compound of general formula I obtained according to the invention wherein $R_3$ represents an aminocarbonyl group may be converted by dehydration into a corresponding compound of general formula I wherein $R_4$ represents a cyano group, and/or

a compound of general formula I obtained according to the invention may be resolved, by chromatography on chiral phases, into the enantiomers thereof, if it contains a chiral centre, and/or

a compound of general formula I obtained according to the invention may be converted into the salts thereof, more particularly the physiololgically acceptable salts thereof with inorganic or organic acids or bases.